# EUROPEAN PATENT SPECIFICATION

(11) **EP 1 694 641 B1**
(45) Date of publication and mention of the grant of the patent: **15.04.2009**
(21) Application number: 04801246.2
(22) Date of filing: 01.12.2004
(51) Int. Cl.: C07C 405/00, C07D 309/12

(54) **PROSTAGLANDIN SYNTHESIS**
PROSTAGLANDINSYNTHESE
SYNTHESE DE LA PROSTAGLANDINE

(30) Priority: 19.12.2003 GB 0329379
(43) Date of publication of application: 30.08.2006
(73) Proprietor: JOHNSON MATTHEY PUBLIC LIMITED COMPANY, 40-42 Hatton Garden London EC1N 8EE (GB)
(72) Inventor: CLISSOLD, Derek Wyndham, Wokingham RG40 5YE (GB); CRAIG, Stuart Wilbert, Brampton, Ontario L6T 4T4 (CA); GADIKOTA, Rajendrakumar, Reddy, Arlington, MA 02474 (US); HE, Min, Groton, MA 01450 (US); JURAYJ, Jurjus Fayez, Acton, MA 01720 (US); KAZERANI, Shahrokh, Leominster, MA 01453 (US); RANNALA, Erwin, Maryborough Woods, Douglas, Cork (IE); SHARMA, Pradeep, Kumar, Westford, MA 01886 (US)
(74) Representative: Swift, Jane Elizabeth Berrisford
(86) International application number: PCT/GB2004/005028
(87) International publication number: WO 2005/058812

(56) References cited:
- US-A1- 2003 187 071
- HAZATO A ET AL: "Synthesis of thiaprostaglandin E1 derivatives" CHEMICAL AND PHARMACEUTICAL BULLETIN, PHARMACEUTICAL SOCIETY OF JAPAN. TOKYO, JP, vol. 33, no. 5, May 1985 (1985-05), pages 1815-1825, XP002257910 ISSN: 0009-2363
- DATABASE BEILSTEIN BEILSTEIN INSTITUT ZUR FÖRDERUNG DER CHEMISCHEN WISSENSCHAFT, FRANKFURT AM MAIN, DE; XP002323895 retrieved from XFIRE Database accession no. 9073863 -& ADV. SYNTH. CATAL., vol. 344, no. 1, 2002, pages 50-56, XP002323893
- DATABASE BEILSTEIN BEILSTEIN INSTITUT ZUR FÖRDERUNG DER CHEMISCHEN WISSENSCHAFT, FRANKFURT AM MAIN, DE; XP002323896 retrieved from XFIRE Database accession no. 9058777 -& BIOORG. MED. CHEM., vol. 10, no. 4, 2002, pages 975-988, XP002323894

## Description

The present invention relates to a novel process for the synthesis of prostaglandins and prostaglandin analogues. The present invention further relates to novel synthetic intermediates that can be used in the synthesis of prostaglandins and prostaglandin analogues.

PGF_{α} prostaglandins and prostaglandin analogues comprise a cyclopentyl ring carrying two hydroxyl groups in a *cis* configuration and two side chains in a *trans* configuration. The side chains may contain double bonds and a variety of substituents. They have a number of therapeutic uses, e.g. for the treatment of glaucoma and ocular hypertension or to induce and accelerate labour.

A variety of methods of synthesising PGF_{α} prostaglandins and prostaglandin analogues are known and are disclosed in, e.g. Chem. Rev. (1993, vol. 93, pages 1533-1564), WO 02/096868, WO 02/090324 and Chinese Journal of Medicinal Chemistry (1998, vol. 36, pages 213-217).

The present inventors have sought to provide an alternative method of synthesising PGF_{α} prostaglandins and prostaglandin analogues. Ideally the synthetic route will be generally applicable to a variety of prostaglandin compounds and will provide high yields.

Accordingly, the present invention provides a process for the preparation of prostaglandin compounds having the formula (I): 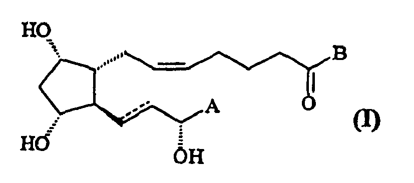 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; B is selected from OR" and NHR" wherein R" is C₁-C₆ alkyl; and - - - - - represents a double bond or a single bond.

Scheme 1 shows the synthesis of prostaglandins of formula (I) starting from an allyl cyclopentenone (X) and a substituted alkene (IX) (P is a hydroxyl protecting group): 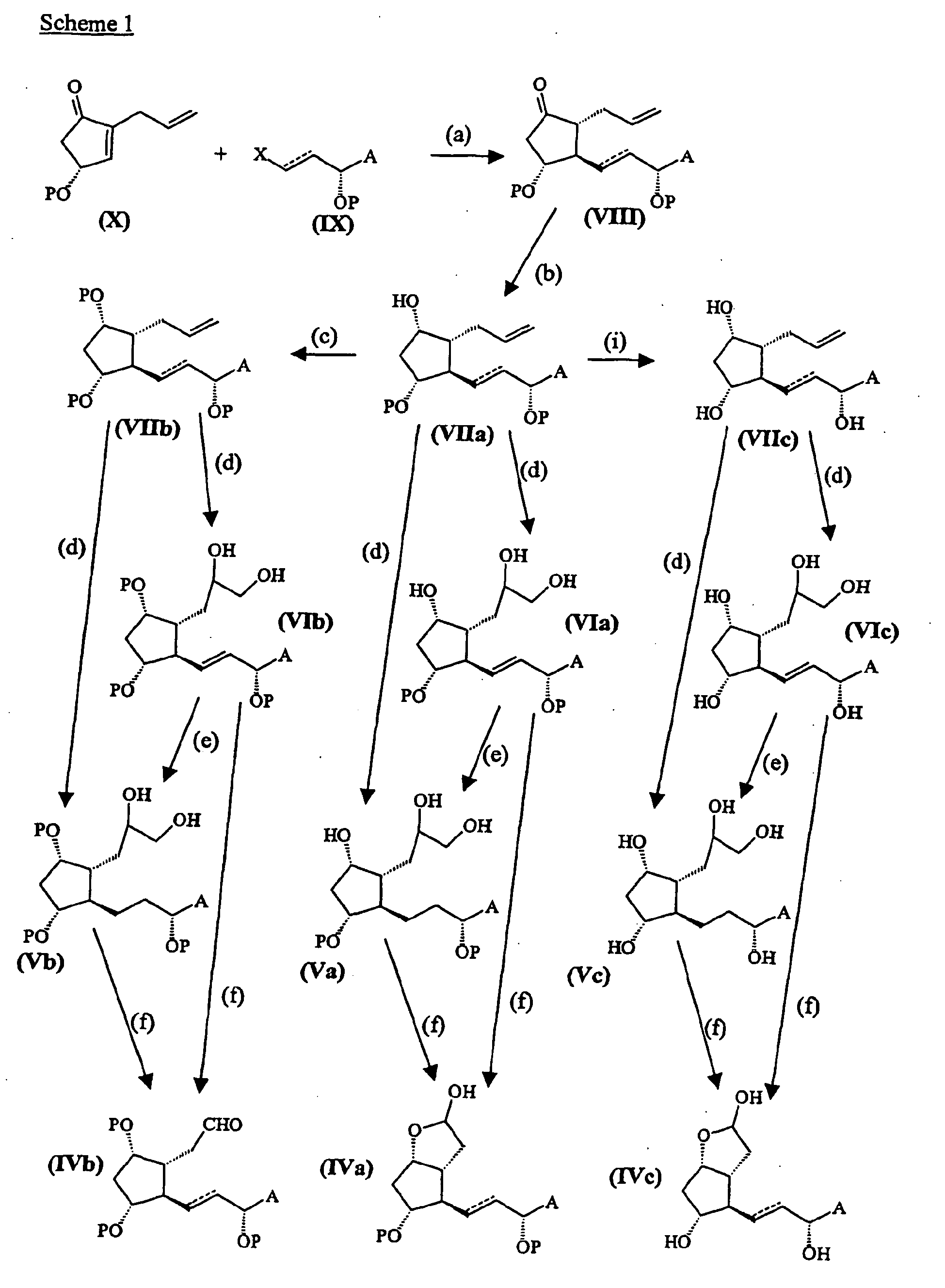 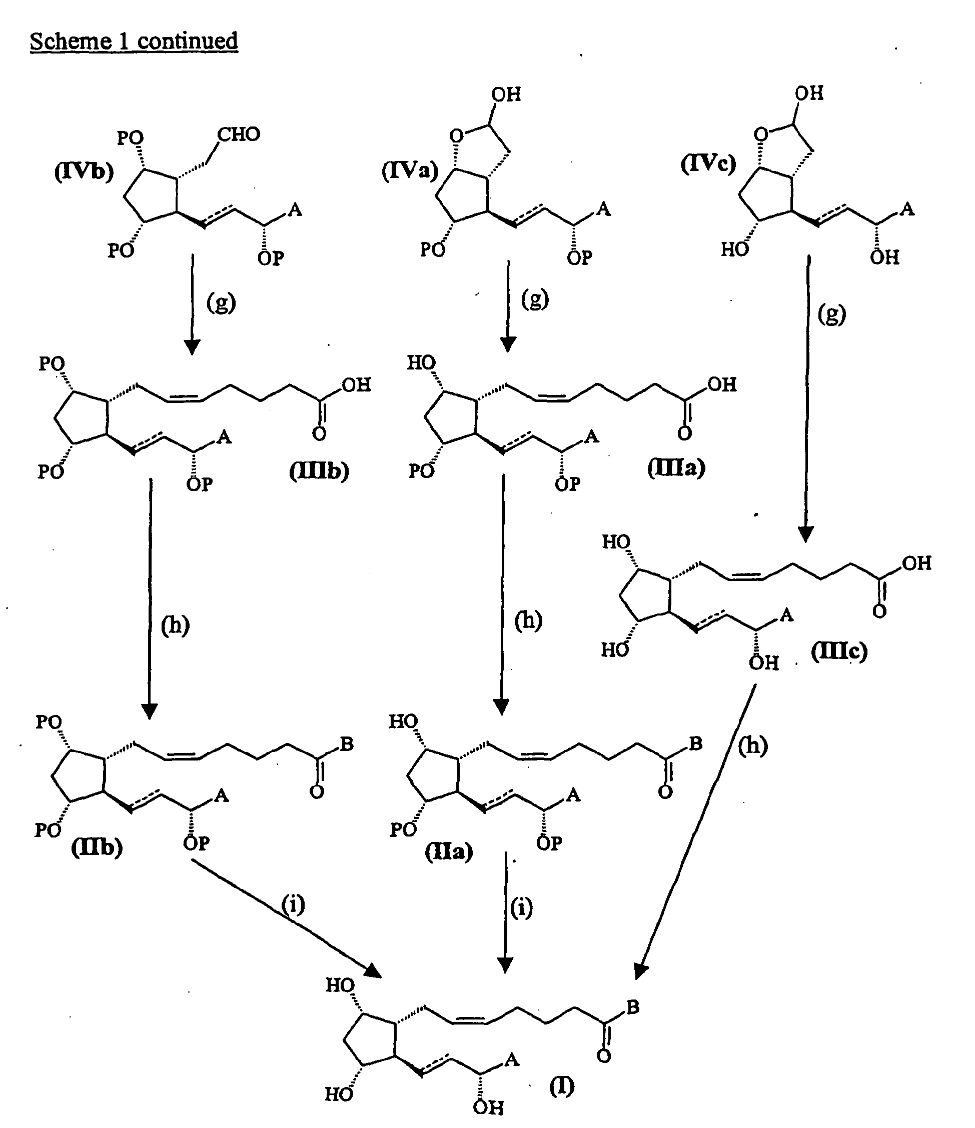
(a) 1,4 addition of a cuprate reagent formed from compound of formula (IX); (b) stereoselective reduction; (c) protection with a hydroxyl protecting group; (d) dihydroxylation; (e) reduction; (f) diol cleavage; (g) Wittig reaction: (h) esterification or amidation; (i) deprotection.

JP 59-044336 and JP 57-171965 disclose methods of synthesising prostaglandins wherein a compound of Formula (VIIa) wherein A is CR¹R²OR³, wherein R¹ is C₁₋₁₀ alkyl, R² is H or methyl and R³ is H or a hydroxy protecting group, is reacted with a sulphur compound. The methods are not suitable for forming prostaglandins such as Latanoprost, Bimatoprost and Travoprost, wherein the side chains do not contain sulphur.

In one aspect the present invention provides a process for the preparation of a compound of Formula (VIII): 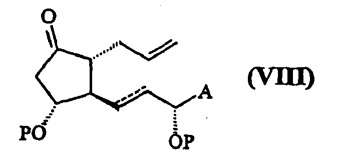 wherein A is (CH₂)₂Ph or CH₂OR'", wherein R"' is phenyl, substituted at the meta position with CF₃ ; P is a hydroxyl protecting group and - - - - - represents a double bond or a single bond;
wherein a compound of Formula (IX): 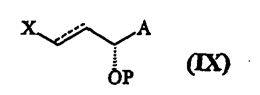 wherein A, P and - - - - - are as defined above and X is a leaving group, is reacted to form a cuprate reagent which undergoes a 1,4 addition reaction with a compound of Formula (X): 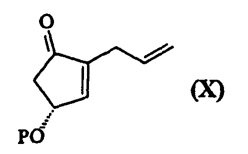 wherein P is as defined above.

Suitably, P is a tetrahydropyranyl (THP) or silyl ether protecting group. Preferably P is a THP protecting group. Suitably X is a halogen, preferably iodine.

Techniques for forming suitable cuprate reagents from compounds of Formula (IX) are well known to the person skilled in the art. For example, compounds wherein X is iodine can be reacted with a lithiated alkane, e.g. n-BuLi, in a solvent such as tetrahydrofuran (THF) or tert-butyl methyl ether (TBME) at -78°C for one hour. Subsequently, copper cyanide and methyl lithium are added to the reaction mixture, and the temperature rises to 0°C over 45 minutes. The cuprate reagent is formed in the reaction mixture. The cyclopentenone of formula (X) can be added to the reaction mixture, preferably at -78°C, and the cuprate reagent will undergo 1,4 addition to compound (X), forming compound (VIII).

Methods of synthesising compounds of formula (IX) wherein - - - - - represents a single bond are disclosed in WO 02/90324.

Cyclopentenones of formula (X) can be made from readily available starting materials such as allylmagnesium chloride and 2-furaldehyde: 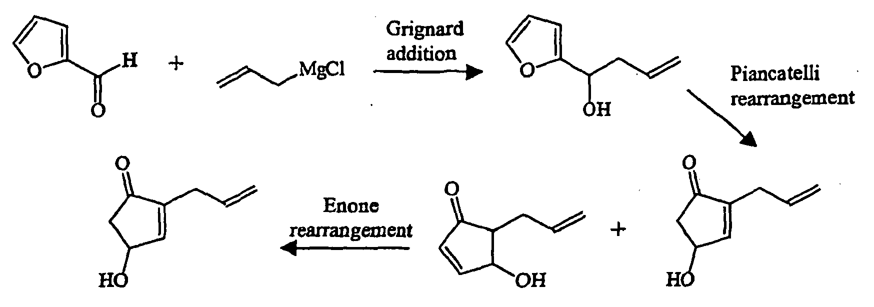 The racemic cyclopentenone can be resolved using standard techniques and the alcohol should be protected. Other methods of forming cyclopentenones of formula (X) are disclosed in EP 115 680.

In a preferred embodiment of the invention, A is (CH₂)₂Ph, - - - - - represents a double bond, P is THP and X is I:

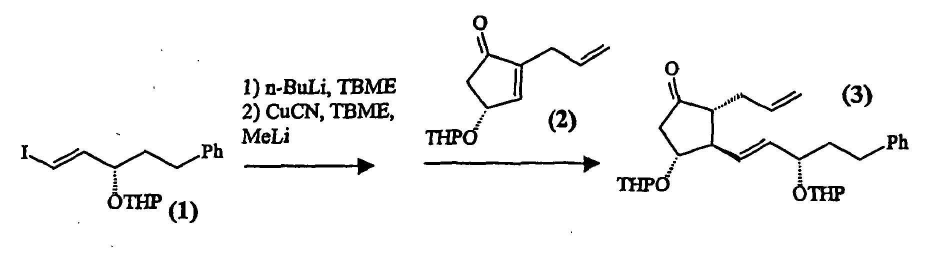

In a further aspect, the present invention provides compounds of formula (VIII): 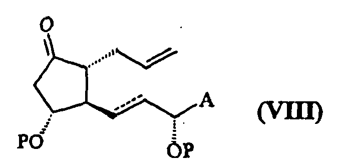 wherein A, P and - - - - - are as defined above.

In a preferred embodiment the present invention provides a compound of formula (VIII) wherein A is (CH₂)₂Ph, - - - - - represents a double bond and P is THP:

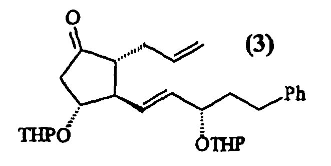

In a further aspect the invention provides a process for the preparation of a compound of formula (VIIa): 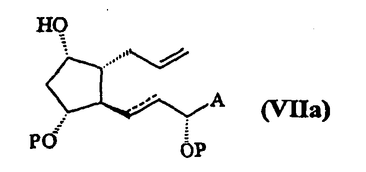 wherein A is (CH₂)₂Ph or CH₂OR"', wherein R"' is phenyl, substituted at the meta position with CF₃; P is a hydroxyl protecting group and - - - - - represents a double bond or a single bond;
wherein a compound of Formula (VIII): 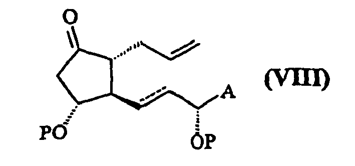 wherein A, P and - - - - - are as defined above, undergoes selective reduction.

Suitably, P is a tetrahydropyranyl (THP) or silyl ether protecting group. Preferably P is a THP protecting group.

Suitable selective reduction reagents are well known to the skilled person. Preferably the ketone is selectively reduced using L-Selectride in tetrahydrofuran (THF) solvent at -78°C.

In a preferred embodiment, A is (CH₂)₂Ph, - - - - - represents a double bond and P is THP:

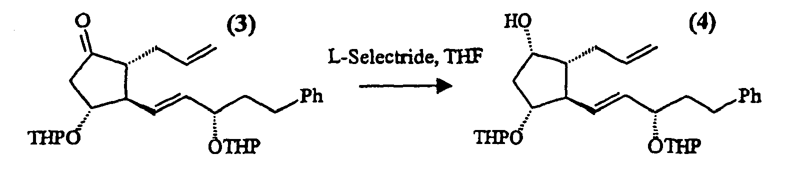

In a further aspect, the present invention provides compounds of formula (VIIa): 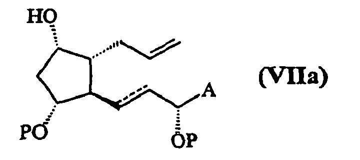 wherein A, P and - - - - - are as defined above.

In a preferred embodiment the present invention provides a compound of formula (VIIa) wherein A is (CH₂)₂Ph, - - - - - represents a double bond and P is THP:

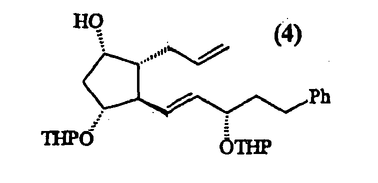

In a further aspect the invention provides a process for the preparation of a compound of formula (VIIb): 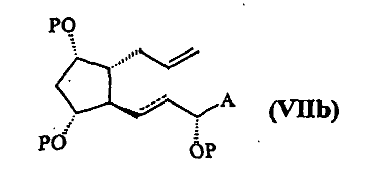 wherein A (CH₂)₂Ph or CH₂OR"', wherein R"' is phenyl, substituted at the meta position with CF₃; P is a hydroxyl protecting group and - - - - - represents a double bond or a single bond;
wherein a compound of Formula (VIIa): 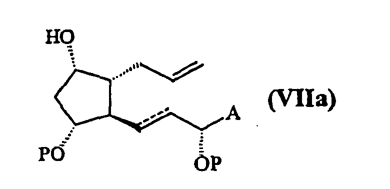 wherein A, P and - - - - - are as defined above, is protected with a hydroxyl protecting group.

Suitably, P is a tetrahydropyranyl (THP) or silyl ether protecting group. Preferably P is a THP protecting group.

Methods of protecting hydroxyl groups are well known to the skilled person. If P is THP, the hydroxyl group can be protected by reaction with 2,3-dihydro-4H-pyran (DHP) and pyridinium p-toluenesulfonate (PPTS) at room temperature in a dichloromethane (DCM) solvent.

In a preferred embodiment, A is (CH₂)₂Ph, - - - - - represents a double bond and P is THP:

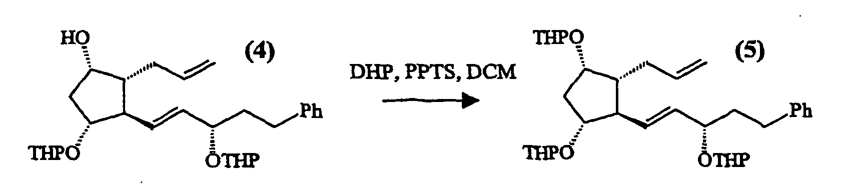

In a further aspect, the present invention provides compounds of formula (VIIb): 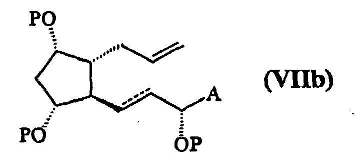 wherein A, P and - - - - - are as defined above.

In a preferred embodiment the present invention provides a compound of formula (VIIb) wherein A is (CH₂)₂Ph, - - - - - represents a double bond and P is THP:

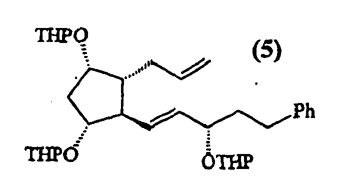

In a further aspect the invention provides a process for the preparation of a compound of formula (VIIc): 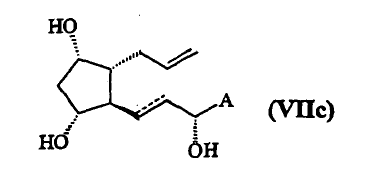 wherein A is (CH₂)₂Ph or CH₂OR"', wherein R"' is phenyl, substituted at the meta position with CF₃ and - - - - - represents a double bond or a single bond;
wherein a compound of Formula (VIIa): 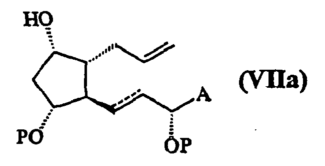 wherein A, P and - - - - - are as defined above, is deprotected.

Suitably, P is a tetrahydropyranyl (THP) or silyl ether protecting group. Preferably P is a THP protecting group.

Methods of deprotecting protected hydroxyl groups are well known to the skilled person. If P is THP, the hydroxyl groups can be removed by reaction by reaction with pyridinium p-toluenesulfonate (PPTS) in a methanol solvent.

In a preferred embodiment, A is (CH₂)₂Ph and - - - - - represents a double bond:

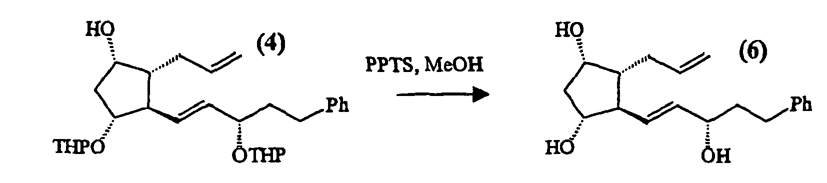

In a further aspect, the present invention provides compounds of formula (VIIc): 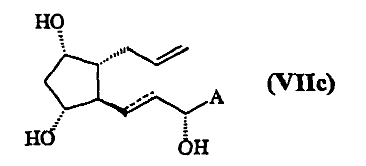 wherein A and - - - - - are as defined above.

In a preferred embodiment the present invention provides a compound of formula (VIIc) wherein A is (CH₂)₂Ph and - - - - - represents a double bond:

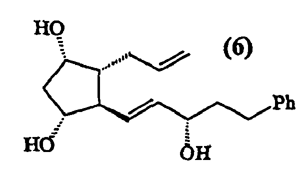

In a further aspect the invention provides a process for the preparation of a compound of formula (VIa), (VIb), (VIc), (Va), (Vb) or (Vc): 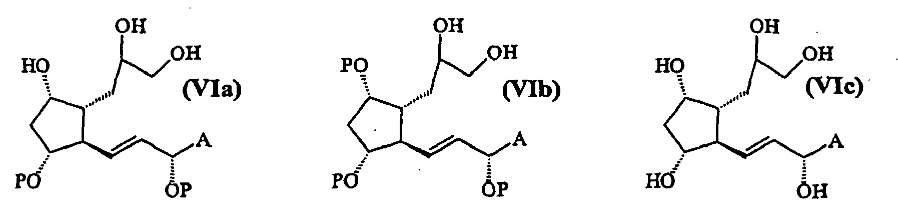 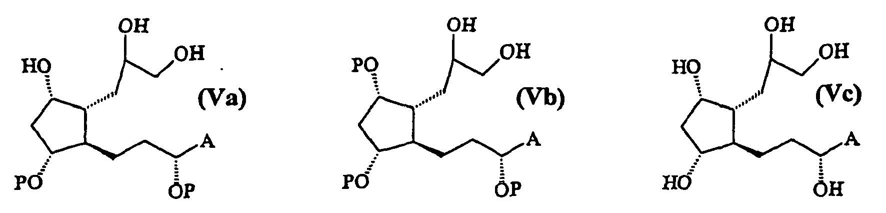 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; and P is a hydroxyl protecting group;
wherein a compound of Formula (VIIa), a compound of Formula (VIIb) or a compound of Formula (VIIc): 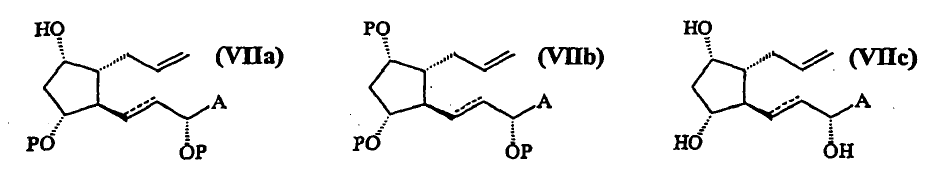 wherein A and P are as defined above and - - - - - is a double or single bond, undergoes dihydroxylation.

If there is more than one double bond in the compound of formula (VIIa), (VIIb) or (VIIc), then it is the terminal double bond that undergoes dihydroxylation.

Suitably, P is a tetrahydropyranyl (THP) or silyl ether protecting group. Preferably P is a THP protecting group.

Suitable methods of dihydroxylating double bonds are well known to the skilled person. A preferred reagent is *N*-methyl morpholine-*N*-oxide (NMO) in the presence of catalytic amounts of osmium tetroxide. The solvent is preferably a 4:1 mixture of tetrahydrofuran (THF) and water. The reaction is suitably carried out from -10 to -4°C.

In a preferred embodiment, A is (CH₂)₂Ph, P is THP, - - - - - represents a double bond, and (VIIa) is reacted to give (VIa):

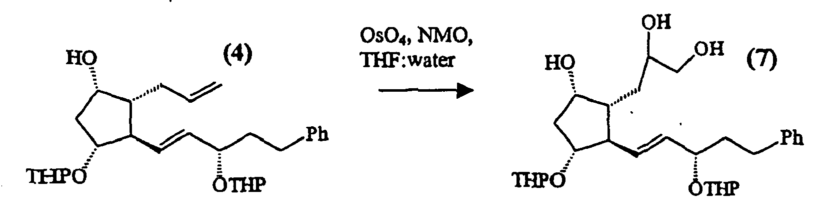

In a second preferred embodiment, A is (CH₂)₂Ph, P is THP, - - - - - represents a double bond, and (VIIb) is reacted to give (VIb):

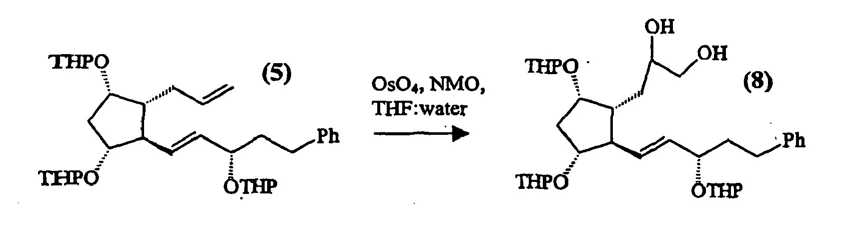

In a third preferred embodiment, A is (CH₂)₂Ph, - - - - - represents a double bond, and (VIIc) is reacted to give (VIc):

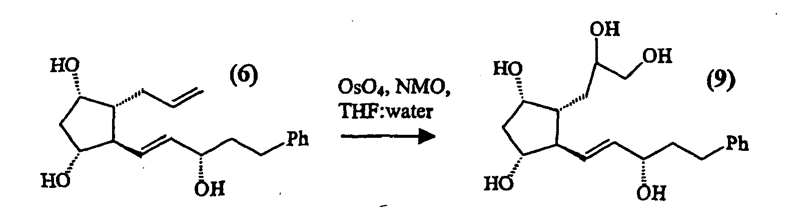

In a further aspect, the present invention provides compounds of formula (VIa), (VIb) (VIc), (Va), (Vb) and (Vc): 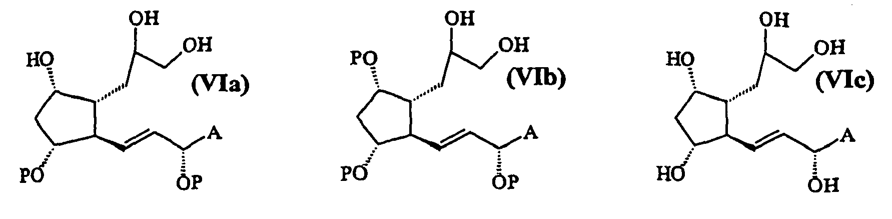 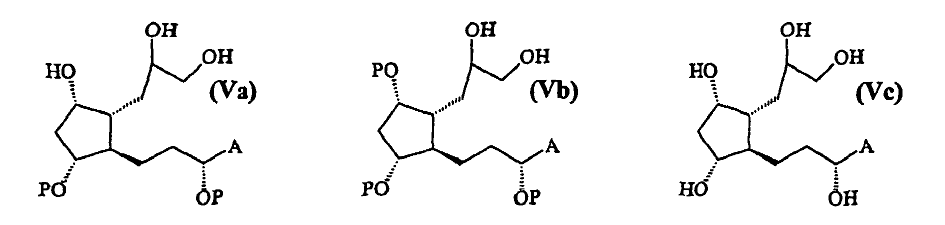 wherein A and P are as defined above.

In a preferred embodiment the present invention provides compounds of formula (VIa), (VIb), (Va) and (Vb) wherein A is (CH₂)₂Ph and P is THP, and compounds of formula (VIc) and (Vc) wherein A is (CH₂)₂Ph:

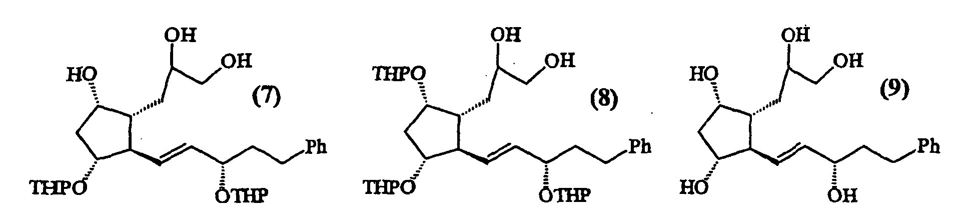

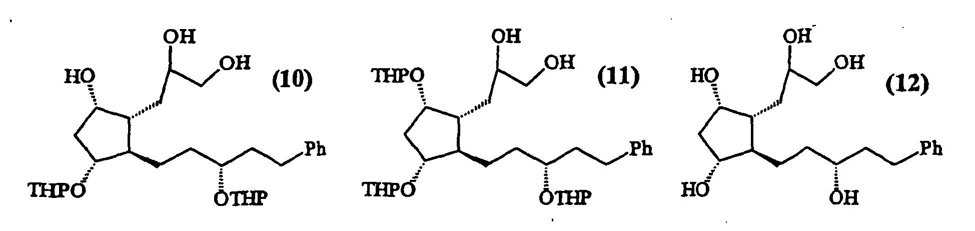

In a further aspect the invention provides a process for the preparation of a compound of formula (Va), (Vb) or (Vc): 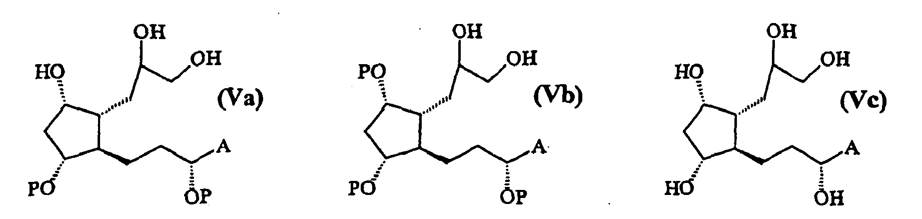 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; and P is a hydroxyl protecting group;
wherein a compound of Formula (VIa), a compound of Formula (VIb) or a compound of Formula (VIc): 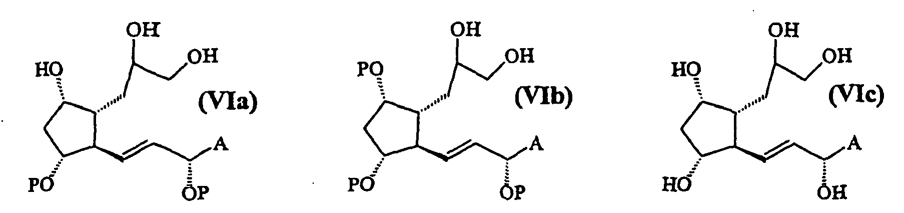 wherein A and P are as defined above, undergoes reduction of the double bond.

Suitably, P is a tetrahydropyranyl (THP) or silyl ether protecting group. Preferably P is a THP protecting group.

Suitable methods of reducing double bonds are well known to the skilled person. In a preferred method, the double bond is hydrogenated, e.g. by passing hydrogen gas at a pressure of 40psi into the reaction mixture which comprises a 5% Pd on carbon catalyst in an ethanol solvent.

In a preferred embodiment, A is (CH₂)₂Ph, P is THP and (VIa) is reacted to give (Va):

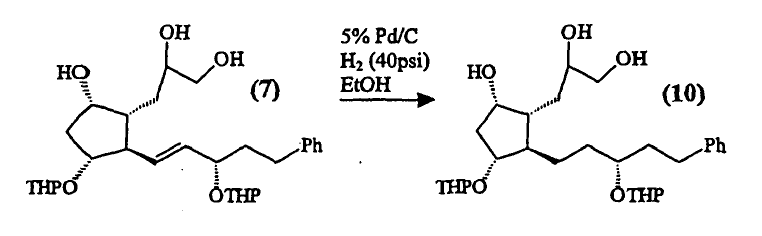

In a second preferred embodiment, A is (CH₂)₂Ph, P is THP and (VIb) is reacted to give (Vb):

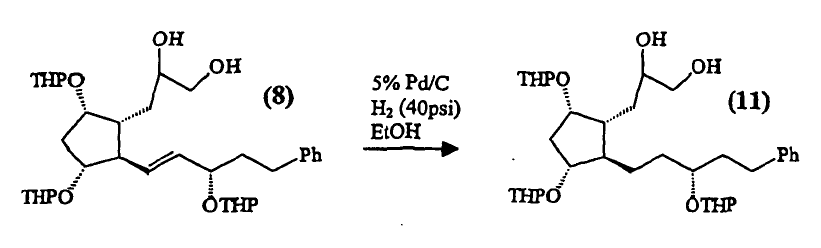

In a third preferred embodiment, A is (CH₂)₂Ph and (VIc) is reacted to give (Vc):

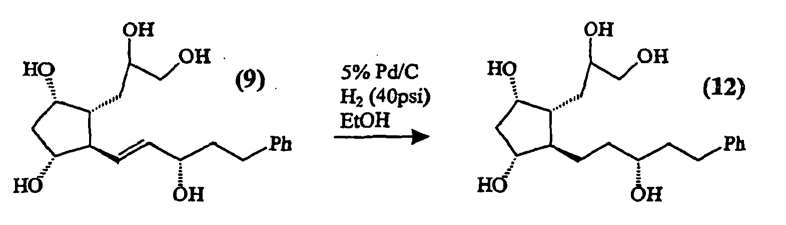

In a further aspect the invention provides a process for the preparation of a compound of formula (IVa), (IVb) or (IVc): 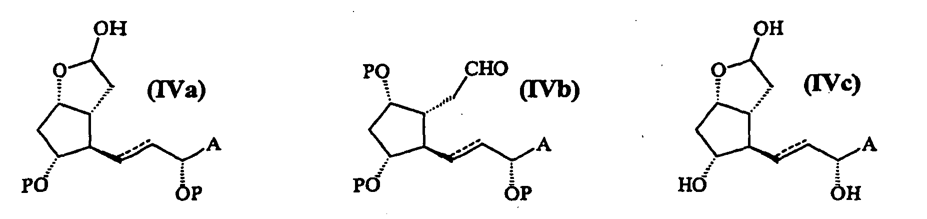 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂).OR' wherein n is from 1 to 3 and R' represents a C₆-C₁₀ aryl group which is optionally substituted with one to three substituents selected from the group consisting of C₁-C₆ alkyl, halo and CF₃; P is a hydroxyl protecting group and - - - - - represents a double bond or a single bond;
wherein a compound of Formula (VIa), (Va), (VIb), (Vb), (VIc) or (Vc): 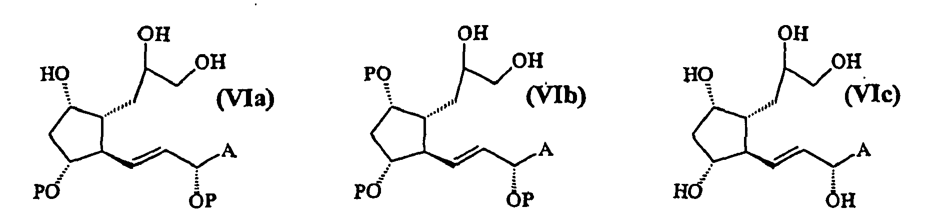 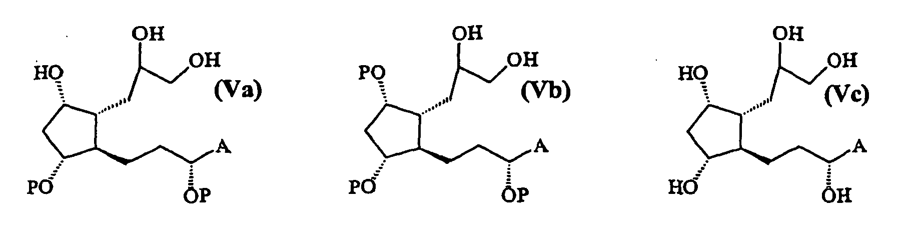 wherein A and P are as defined above, undergoes diol cleavage.

Suitably, P is a tetrahydropyranyl (THP) or silyl ether protecting group. Preferably P is a THP protecting group.

Suitable methods of diol cleavage are well known to the skilled person. A preferred method uses two equivalents of sodium periodate and silica in a 1:1 ratio. A suitable solvent is a mixture of water and dichloromethane.

In a preferred embodiment, A is (CH₂)₂Ph, P is THP, - - - - - represents a single bond, and (Va) is reacted to give (IVa):

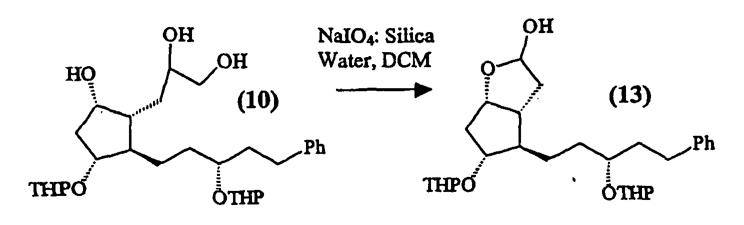

In a second preferred embodiment, A is (CH₂)₂Ph, P is THP, - - - - - represents a single bond, and (Vb) is reacted to give (IVb):

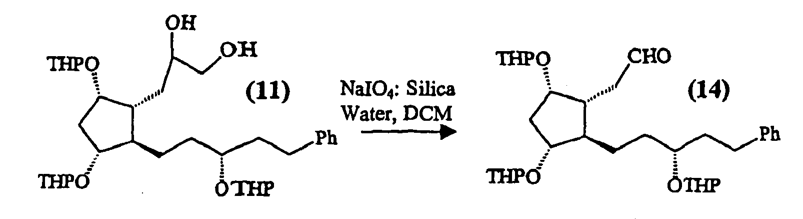

In a third preferred embodiment, A is (CH₂)₂Ph, - - - - - represents a single bond, and (Vc) is reacted to give (IVc):

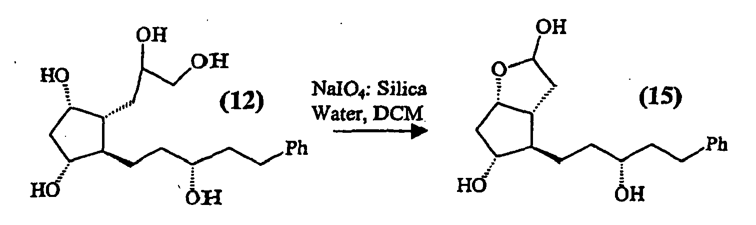

In a further aspect, the present invention provides a compound of formula (IVb) wherein A is (CH₂)₂Ph, P is THP and - - - - - is a single bond:

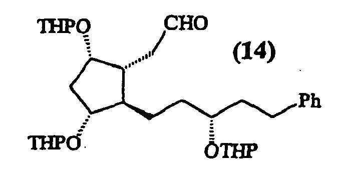

Compounds of formula (IVa), (IVb) or (IVc): 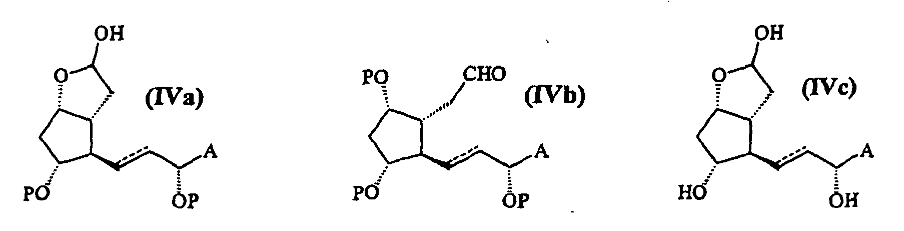 wherein A is selected from the group consisting of C₁-C₆ alkyl; C₇-C₁₆ aralkyle wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; P is a hydroxyl protecting group and - - - - - represents a double bond or a single bond; can be reacted with Wittig reagents to give compounds of formula (IIIa), (IIIb) and (IIIc): 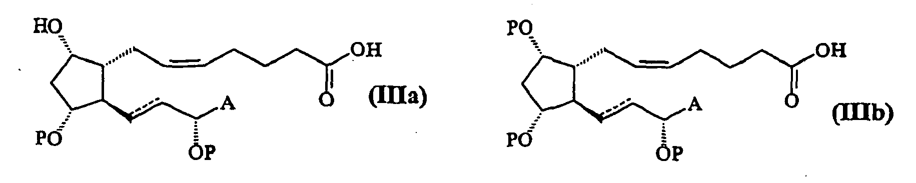 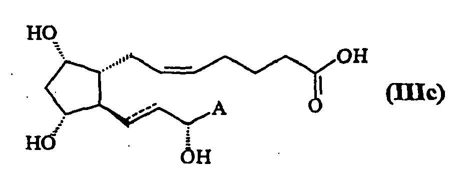 wherein A, P and - - - - - are as defined above.

Suitable Wittig reagents will be well known to the person skilled in the art. A preferred Wittig reagent is (4-Carboxybutyl)triphenylphosphonium bromide and this is reacted with the compound of formula (IVa), (IVb) or (IVc) and potassium t-butoxide in tetrahydrofuran (THF) solvent at 0°C.

For example, a compound of formula (IVa) wherein A is (CH₂)₂Ph, P is THP, - - - - - represents a single bond is reacted to give a compound of formula (IIIa):

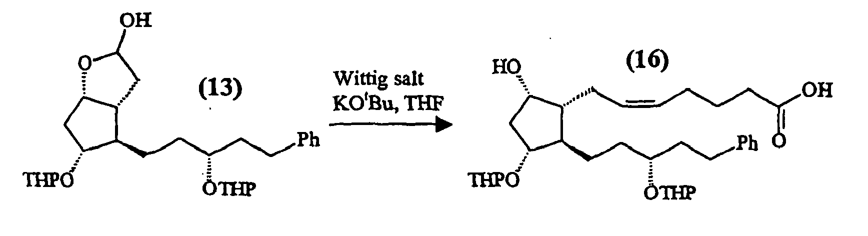

In a second example, a compound of formula (IVb) wherein A is (CH₂)₂Ph, P is THP, - - - - - represents a single bond is reacted to give a compound of formula (IIIb):

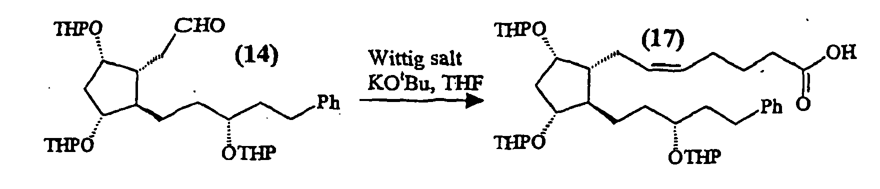

In a third example, a compound of formula (IVc) wherein A is (CH₂)₂Ph and - - - - - represents a single bond is reacted to give a compound of formula (IIIc):

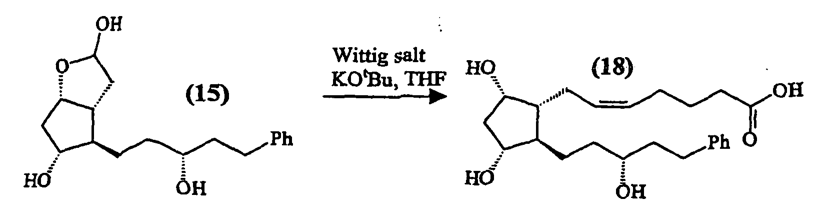

Compounds of formula (IIIa), (IIIb) or (IIIc): 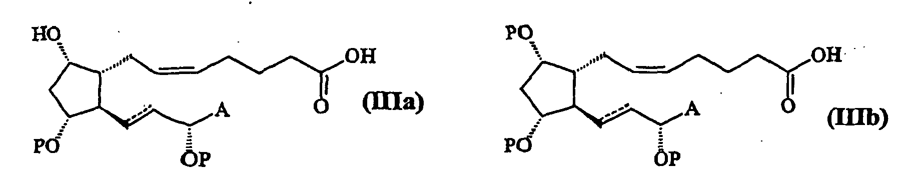 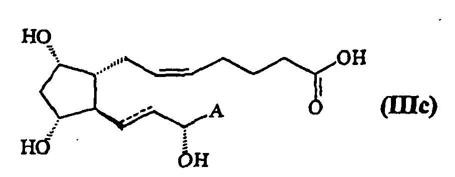 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; P is a hydroxyl protecting group and - - - - - represents a double bond or a single bond; can be esterified or amidated to give compounds of formula (IIa), (IIb) and (I): 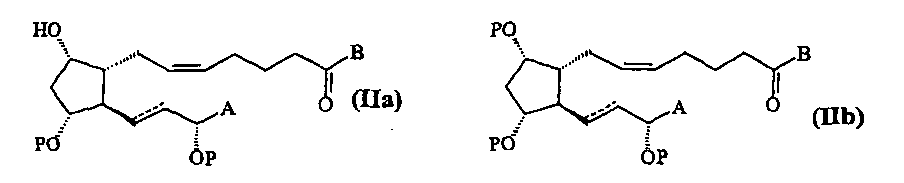 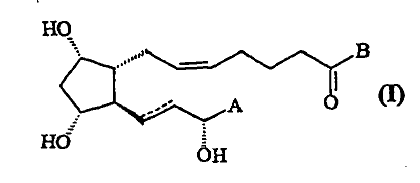 wherein A, P and - - - - - are as defined above, and B is selected from OR" and NHR" wherein R" is C₁-C₆ alkyl.

Suitable esterification/ amidation reagents will be well known to the person skilled in the art. A preferred esterification reagent is 2-iodopropane (isopropyl iodide) and the reaction mixture further comprises a base such as 1,8-diazabicyclo[5.4.0]undec-7-ene (DBU). Acetone is a suitable solvent. A preferred amidation reagent is ethylamine (EtNH₂) and the reaction mixture suitably further comprises 1-(3-Dimethylaminopropyl)-3-ethylcarbodiimide hydrochloride (EDC-HCl). Amidation can also be achieved via a multi-step process wherein the compound of formula (IIIa), (IIIb) or (IIIc) is initially esterified before exposure to an amidation reagent such as ethylamine.

For example, a compound of formula (IIIa) wherein A is (CH₂)₂Ph, P is THP and - - - - - represents a single bond, is esterified to give a compound of formula (IIa):

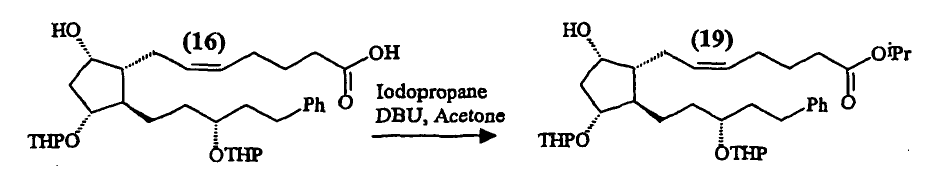

In a second example, a compound of formula (IIIa) wherein A is (CH₂)₂Ph, P is THP and - - - - - represents a double bond is amidated, to give a compound of formula (IIa):

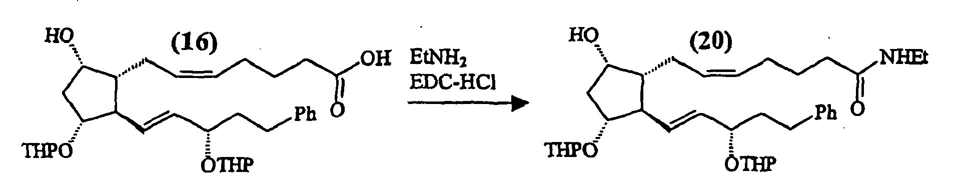

In a third example, a compound of formula (IIIc) wherein A is (CH₂)₂Ph and - - - - - represents a single bond, is esterified to give a compound of formula (I) which is commonly known as Latanoprost:

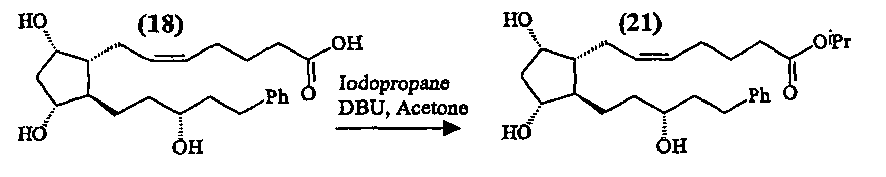

Compounds of formula (Ia) or (IIb): 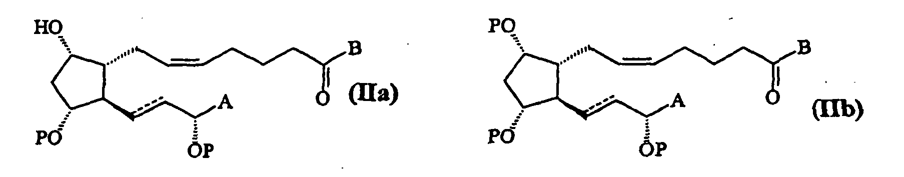 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; P is a hydroxyl protecting group, - - - - - represents a double bond or a single bond and B is selected from OR" and NHR" wherein R" is C₁-C₆ alkyl; can be deprotected to give compounds of formula (I): 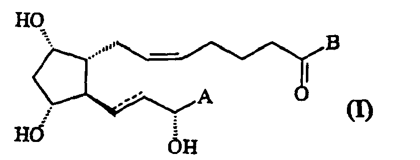 wherein A, B and - - - - - are as defined above.

Suitable deprotection reagents are well known to the person skilled in the art. A preferred reagent is pyridinium p-toluenesulfonate (PPTS) in a methanol solvent.

For example, a compound of formula (IIa) wherein A is (CH₂)₂Ph, P is THP, - - - - - represents a single bond and B is OⁱPr, can be deprotected to give a compound of formula (I) which is commonly known as Latanoprost:

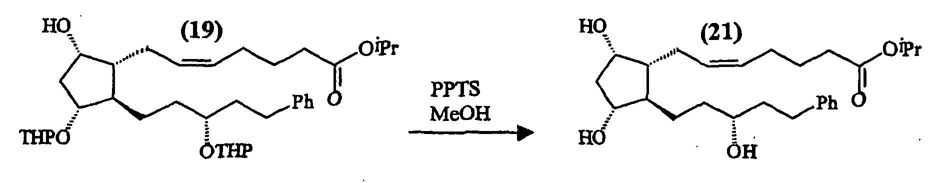

In a second example, a compound of formula (IIa) wherein A is (CH₂)₂Ph, P is THP, - - - - - represents a double bond and B is NHEt can be deprotected to give a compound of formula (I) which is commonly known as Bimatoprost:

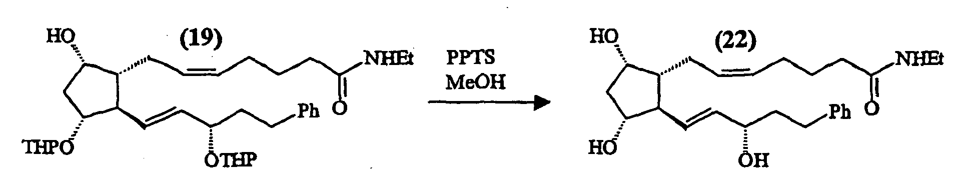

The present invention further provides methods for synthesising Latanoprost as shown in Scheme 2: 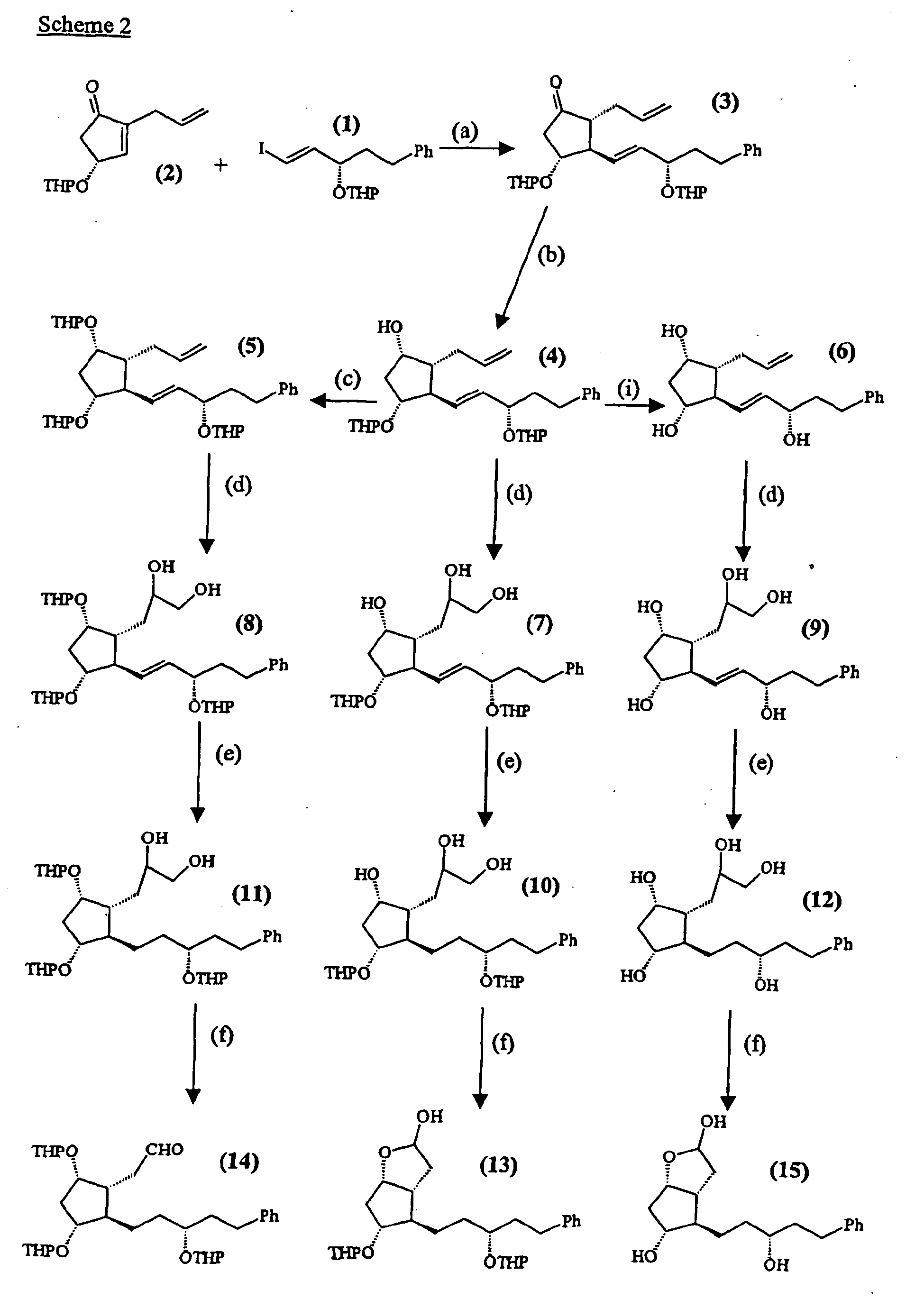 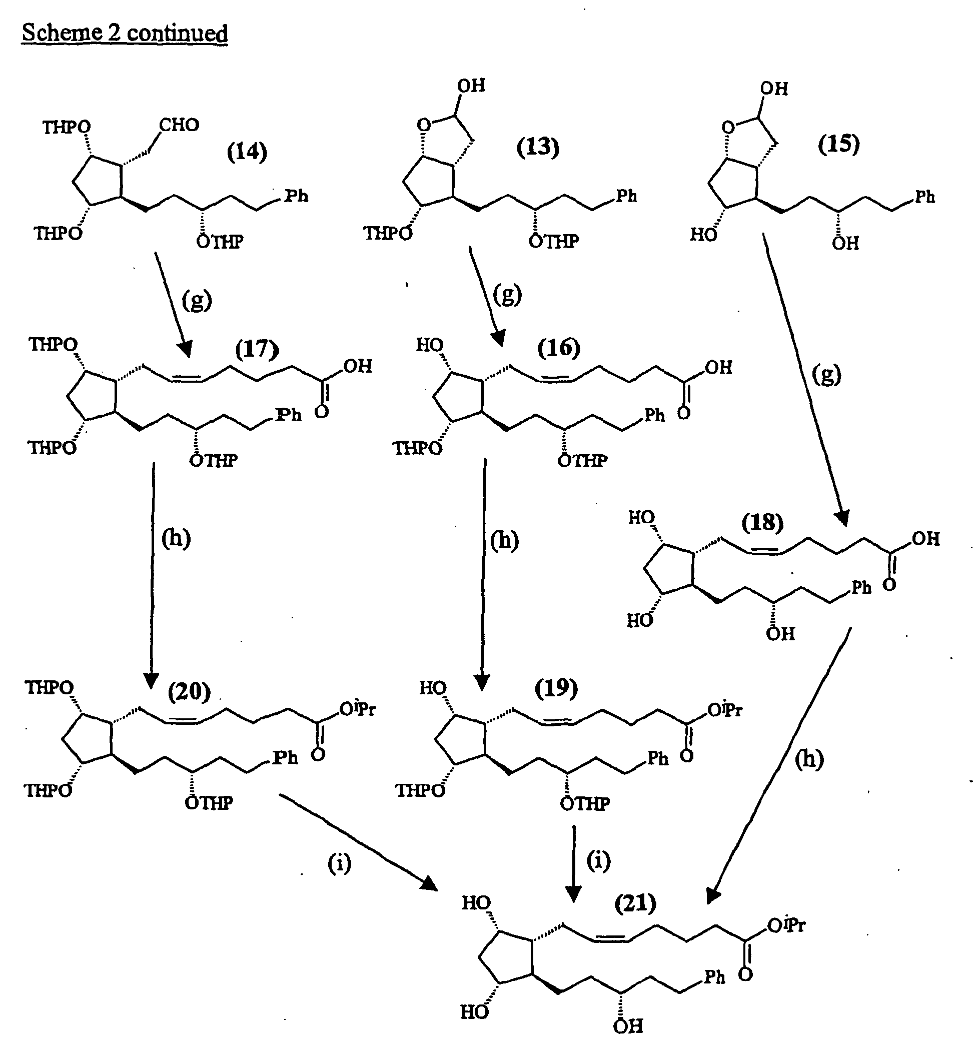 (a) 1,4 addition of a cuprate reagent formed from compound of formula (IX); (b) stereoselective reduction; (c) protection with a hydroxyl protecting group; (d) dihydroxylation; (e) reduction; (f) diol cleavage; (g) Wittig reaction: (h) esterification; (i) deprotection.

The present invention further provides methods for synthesising Bimatoprost as shown in Scheme 3: 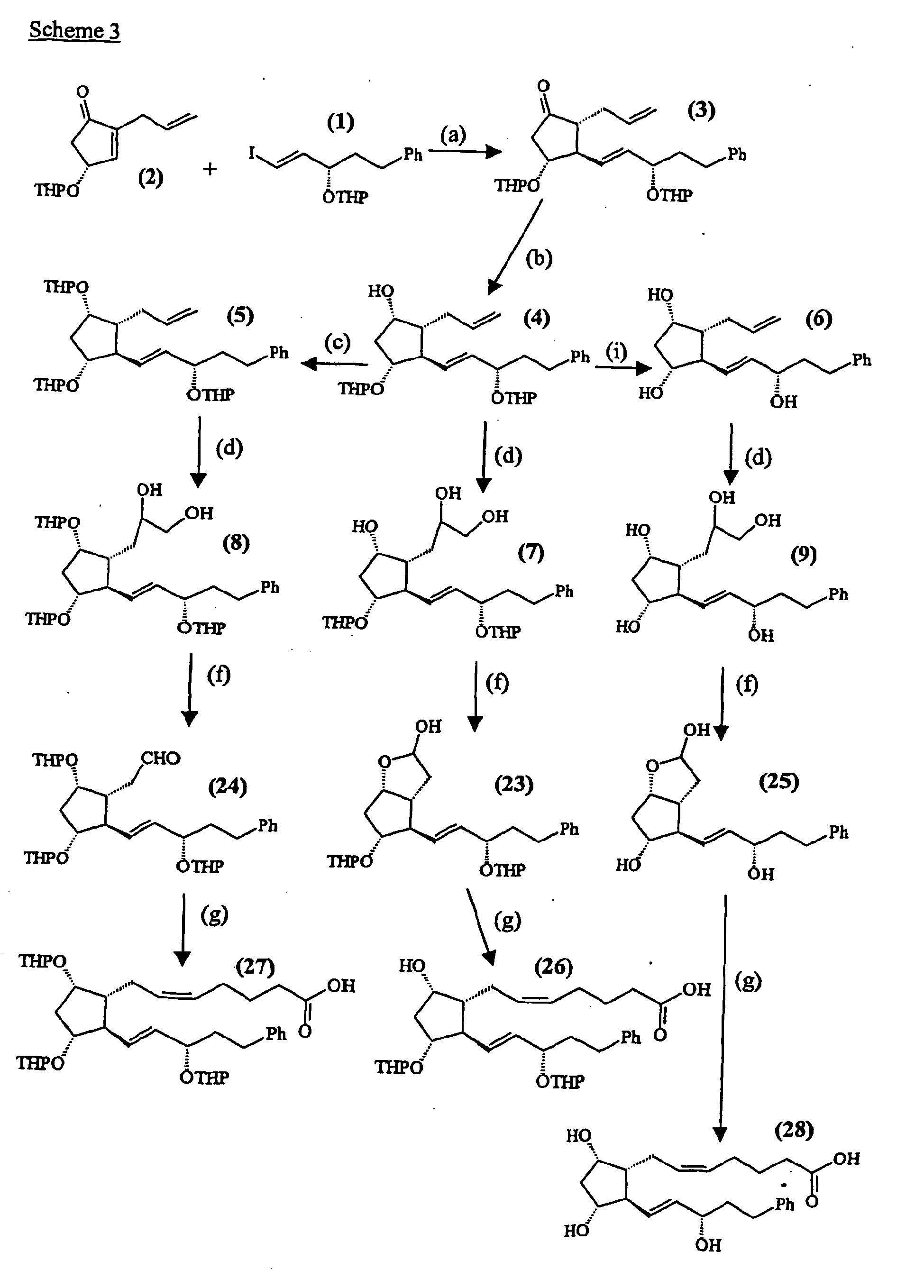 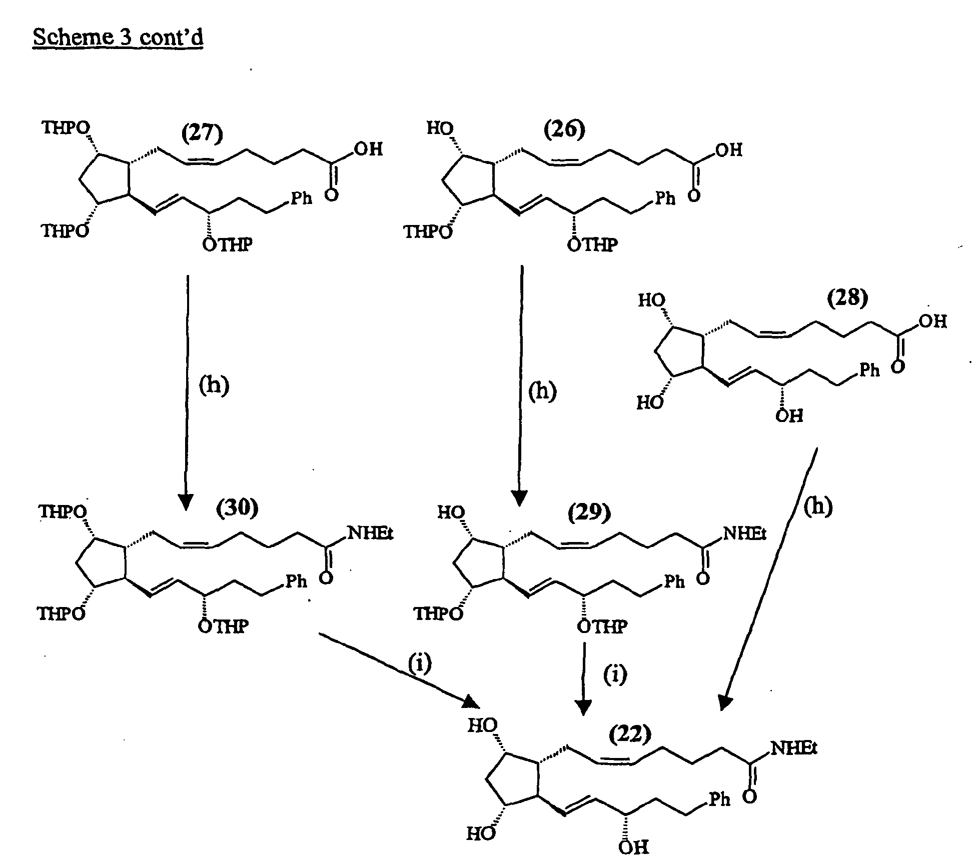 (a) 1,4 addition of a cuprate reagent formed from compound of formula (IX); (b) stereoselective reduction; (c) protection with a hydroxyl protecting group; (d) dihydroxylation; (f) diol cleavage; (g) Wittig reaction: (h) amidation; (i) deprotection.

The present invention further provides methods for synthesising Travoprost as shown in Scheme 4: 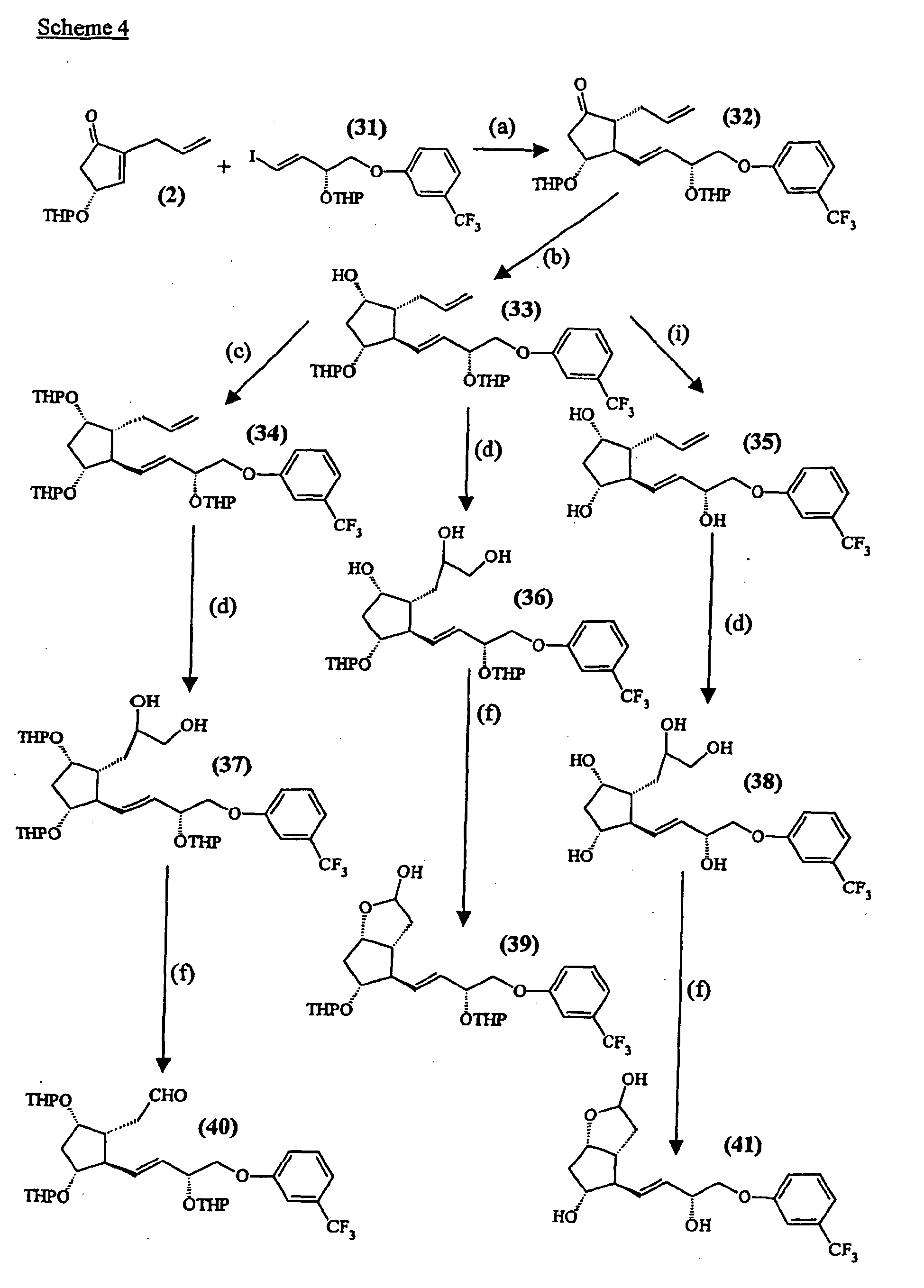 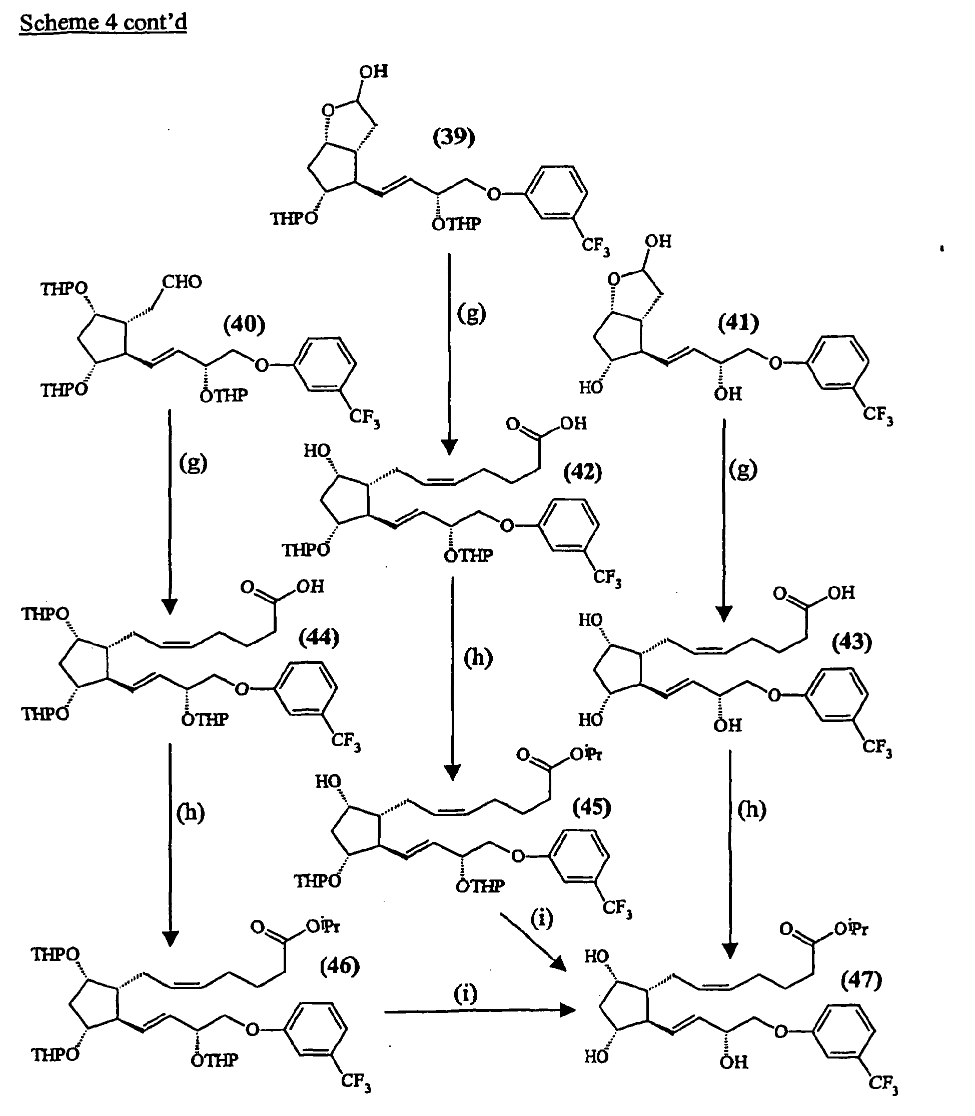 (a) 1,4 addition of a cuprate reagent formed from compound of formula (IX); (b) stereoselective reduction; (c) protection with a hydroxyl protecting group; (d) dihydroxylation; (f) diol cleavage; (g) Wittig reaction: (h) esterification; (i) deprotection.

The invention will now be described by reference to examples which are not intended to be limiting of the invention:

### Synthesis of vinyl iodide (1)

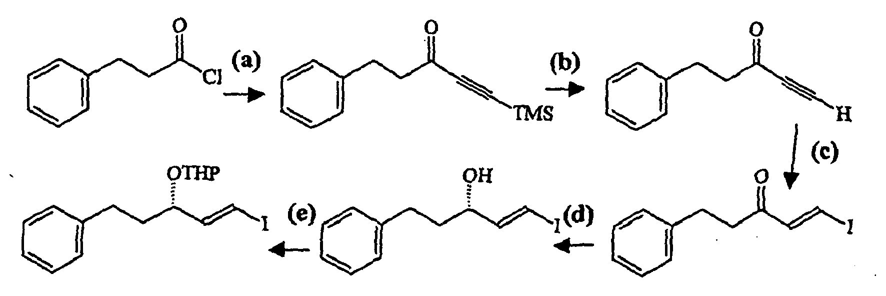

Step (a): A 51, three-necked round bottom flask equipped with a mechanical stirrer, a dropping funnel, a cooling bath and an internal temperature probe was charged with AlCl₃ (217.48g, 1.63mole, 1.1eq.) and CH₂Cl₂ (11) under N₂ atmosphere. The reaction mixture was cooled using ice/salt/methanol bath while the agitation was initiated. Once the temperature was ≤-10°C, a solution of 3-phenylpropionyl chloride (Aldrich, 250g, 1.48mole, 1eq.) and bis(trimethylsilyl)acetylene (GFS Chemicals, 278g, 1.63 mole, 1.1 eq.) in CH₂Cl₂ (11) was added slowly via addition funnel keeping the temperature ≤0°C throughout the addition. Upon completion of the addition, the cooling bath was removed and the mixture was allowed to warm to RT with agitation. After ∼0.5h, the reaction was monitored by TLC for completion. Upon completion of the reaction (ca. 20 min), the mixture was poured slowly to phosphate buffer (pH ∼7, 41) with agitation. The organic layer was separated and the aqueous layer was extracted with CH₂Cl₂ (500ml). The organic layers were combined, washed with 10% aq. NaCl (2 x 750ml), dried over anhydrous MgSO₄ (500g), filtered and the solvent was removed *in vacuo* to produce a dark oil. The oil was further dried under high vacuum at RT for ∼18 h.
Step (b): A 121, three-necked round bottom flask equipped with a mechanical stirrer and a dropping funnel was charged with the oil produced in step (a) (316g, 1.37mole, 1eq.) and methanol (31). To this was added 0.01M Na₂B₄O₇.10H₂O (11.44g in 31 of H₂O) slowly via addition funnel with agitation. The mixture was stirred at room temperature. The progress of the reaction was monitored by TLC. Upon completion of the reaction (ca. 30 min), the pH of the reaction mixture was adjusted to ∼3 (pH paper) using 1N HCl (82.6ml). The solvent was removed in vacuo and the aqueous layer was extracted with CH₂Cl₂ (3x500ml). The organic layers were combined, washed with brine solution (500ml), dried over anhydrous MgSO₄, filtered and the solvent was removed *in vacuo* to give a dark viscous oil. The oil was passed through a plug of silica gel (550g) using 2.5% EtOAc/Heptane (v/v, 5 L) as an eluent.
Step (c): A 51 three-necked round bottom flask equipped with a mechanical stirrer, a dropping funnel and an internal temperature probe was charged with the oil produced in step (b) (121.36g, 0.77mole, leq.), NaI (117.42g, 0.78 mole, 1.02 mole), *n-*Bu₄NI (28.4g, 0.077mole, 10mole%) and MTBE (1213ml). The agitation was initiated. To this was slowly added 4M H₂SO₄ (348g) via addition funnel with agitation. After ∼0.5h, additional amount of 4M H₂SO₄ (174g) was added. The completion of the reaction was monitored by TLC. Upon completion of the reaction (ca 4.5 h), the reaction mixture was transferred to a 41 separatory funnel. The organic layer was separated and the aqueous layer was extracted with MTBE (500ml). The organic layers were combined and washed with 5%aq. NaHCO₃ (2 x 750ml), saturated brine (750ml), dried over MgSO₄ (300g), filtered and the solvent was removed in vacuo to produce a brown red viscous oil. The oil was passed through a pad of silica gel (600g) using 2.5% EtOAc / Heptane (v/v, 6L) to give a viscous oil, which was further dried under high vacuum for ∼18 h at RT.
Step (d): A 11 three-necked round bottom flask equipped with a cooling bath, a magnetic stirrer, a N₂ inlet and an internal temperature probe was charged with the oil produced in step (c) (35g, 0.12mole, leq.), dry toluene (350ml) and *R*-2-methyl-CBS-oxazoborolidine (Callery Chemicals, 24.47ml, 20 mole%, 1M solution in toluene). The mixture was cooled in an acetone-dry ice bath to -78 °C (internal temperature) with stirring, and a solution of catecholborane (Callery Chemicals, 29.34g, 0.25 mole, 2eq.) in dry toluene (260ml) was added over a period of 8 to 9 hours via a syringe pump. Upon completion of the addition, the reaction flask was kept in the bath, and the reaction mixture was allowed to warm slowly to RT overnight. TLC analysis showed that the reaction was complete. The mixture was cooled to ≤10°C (salt/ice bath) and methanol (150ml) was added slowly. The mixture was allowed to warm to room temperature (∼20°C) and 4N NaOH solution (165ml) was added. The biphasic solution was allowed to stir at room temperature for ∼45 minutes and then transferred to a separatory funnel. The organic layer was separated and the aqueous layer was back washed with toluene (100ml). The organic layers were combined and washed with 4 N HCl (2 x 150ml), saturated brine solution (250ml), dried over MgSO₄ (100g), filtered and the solvent was removed *in vacuo* to give a viscous oil which solidified upon standing at RT. The crude product was purified by silica gel chromatography (250 g) using 5% EtOAc-heptane (v/v). The product was obtained as a solid (30g), which was further triturated with heptane (60 mL) at room temperature to give an off-white solid. The solid was suction filtered and dried under high vacuum at room temperature for ∼16h.
Step (e): The hydroxy group of the product of step (d) was protected using 3,4-dihydro-2*H*-pyran and standard techniques.

### Synthesis of cyclopentenone (2)

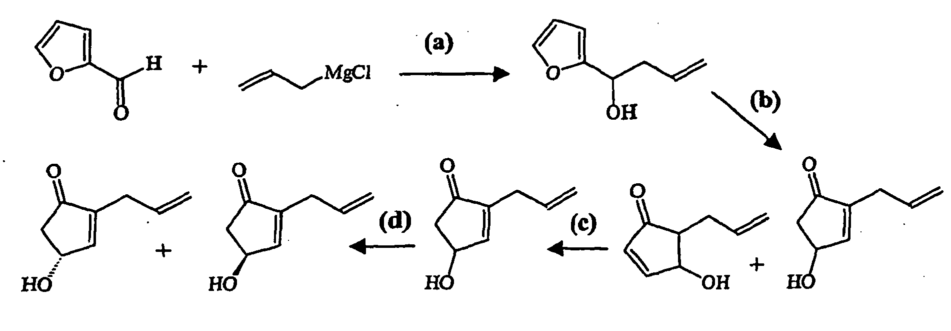

Step (a): A 221 3-necked round bottom flask equipped with an overhead mechanical stirrer, a temperature probe, and an addition funnel was purged with nitrogen and charged with a 2M solution of allylmagnesium chloride in THF (81, 16mol). The flask was cooled in an ice bath to bring the internal temperature to 5°C. A solution of 2-furaldehyde (1.2kg, 12.5mol) in anhydrous THF (21) was added to the Grignard solution slowly over 4.75h maintaining the internal temperature below 12°C. TLC analysis after 15min indicated that the reaction was complete. The reaction was quenched by the sequential addition of saturated NH₄Cl (41), water (21), and concentrated HCl (1.41). The layers were separated, and the aqueous layer was extracted with ethyl acetate (31). The combined organic layers were washed with brine (800g of NaCl dissolved in 31 of water), and concentrated on a rotary evaporator to give the addition product as a dark coloured oil (2.1kg), which was used without further purification. ¹H NMR spectrum conformed to structure.
Step (b): A 221 3-necked round bottom flask equipped with an overhead mechanical stirrer, a temperature probe, and a heating mantle was charged with a buffered solution with a pH of 4.80. A solution of the product of step (a) (500g, 2.9mol) in 1,4-dioxane (1.51) was added in one portion, and the mixture was heated to around 95°C over a 5.5h period. The reaction mixture was stirred at this temperature for ca. 60h, at which time TLC analysis indicated near complete consumption of the step (a) product. The reaction mixture was allowed to cool to 50°C, and solid NaCl (6kg) was added with stirring. The resulting solution was extracted with ethyl acetate (1 x 31 and 1 x 21), and the combined organic phases were dried over MgSO₄. The drying agent was filtered off, and the filtrate was concentrated under reduced pressure. The residual oil was co-evaporated with toluene (250ml) and dried under high vacuum to give a reddish brown viscous oil (456g).
Step (c): A 51 3-necked round bottom flask equipped with an overhead mechanical stirrer, and a nitrogen inlet was charged with the above enone mixture (456g), toluene (3.51), triethylamine (367.3g, 3.6mol), and anhydrous tribromoacetaldehyde (92.65g, 0.33mol). The resulting mixture was allowed to stir at room temperature for 21h, and diluted with water (31). The layers were separated, and the aqueous layer was extracted with ethyl acetate (3 x 0.751). The combined organic layers were washed with 25% wt/v brine solution (21) and dried over MgSO₄. The drying agent was filtered off and the filtrate was concentrated under reduced pressure to give a dark viscous oil (415g). The oil was chromatographed on silica gel (1kg) eluting with 15:85 ethyl acetate-heptane (71), 30:70 ethyl acetate-heptane (21), and 50:50 ethyl acetate-heptane (51) to give an enone as an oil (199g, 50% yield).
Step (d): The enone prepared in step (c) was resolved by Simulated Moving Bed chiral chromatography (SMB). The desired *R*-enantiomer was obtained in >99% optical purity.

To form the cyclopentenone (2), the alcohol of the *R*-enantiomer formed in step (d) was protected using 3,4-dihydro-2*H*-pyran and standard techniques.

### Example 1: 1,4 addition of a cuprate reagents formed from a compound of formula (IX)

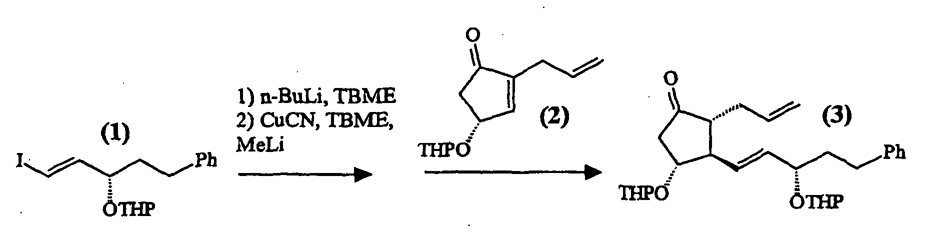

A dry 250ml 3-necked round bottom flask equipped with a magnetic stirring bar, a temperature probe, and rubber septa, was purged with nitrogen. The flask was charged with a solution of vinyl iodide (1) (8.43g, 22.65mmol) in *tert*-butyl methyl ether (TBME, 40ml) and cooled in an acetone-dry ice bath. A solution of *n*-butyllithium in hexanes (2.517M, 9.5ml, 23.8mmol) was added, and the mixture was stirred at -78°C for 2h.

A dry 11 3-necked round bottom flask equipped with a magnetic stirring bar, a temperature probe, and rubber septa, was purged with nitrogen and charged with solid cuprous cyanide (2.12g, 23.78mmol). Anhydrous TBME (60ml) was added and the flask was cooled to -78°C in an acetone-dry ice bath. A solution of methyllithium in THF-cumene (1M, 23.78ml, 23.78mmol) was slowly added to the stirring suspension. After the addition was complete, the flask was placed in an ice bath and the contents stirred for 30min giving a clear solution. The cuprate solution was cooled again to -78°C, and the vinyl lithium solution was added from the 250ml flask via cannula. The resulting yellow solution was quickly warmed to -40°C, stirred for 20min, and recooled to -78°C. A solution of cyclopentenone (2) (2.52g, 11.34mmol) in anhydrous TBME (30ml) was added by cannula, and the mixture was stirred at -78°C for 30 min. The reaction flask was removed from the cooling bath, and the reaction was quenched by the careful addition of saturated NH₄Cl (15ml). The layers were separated and the organic layer was washed with 1:9 NH₄OH-NH₄Cl (2 x 200ml). The combined aqueous washes were back extracted with TBME (100ml). The combined organic layers were washed with saturated brine (100ml), dried (Na₂SO₄), filtered and concentrated to give a pale yellow oil (22.80g). The oil was chromatographed on silica gel (200g) eluting with 1:9 ethyl acetate-heptane (11) and 1.5:8.5 ethyl acetate-heptane (31) to give the pure product (3) (4.15g, 78.6% yield). ¹H NMR conformed to structure.

### Example 2: Stereoselective Reduction

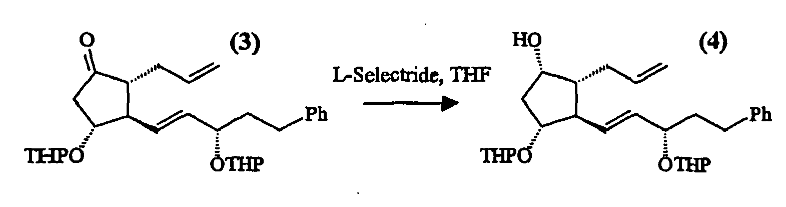

A dry 250ml 3-necked round bottom flask equipped with a magnetic stirring bar, a temperature probe, and an addition funnel, was purged with nitrogen. The flask was charged with a solution of L-Selectride in THF (1M, 16.25ml, 16.25mmol) and cooled to -78°C in an acetone-dry ice bath. A solution of (3) (3.80 g, 8.13 mmol) in anhydrous THF (60ml) was added slowly from the addition funnel over 35min, and the mixture was stirred at -78°C for 4.5h. TLC analysis indicated that the reaction was complete. The cooling bath was removed, and the mixture was quenched by the addition of 30% hydrogen peroxide (2.2ml, 19.4mmol) followed by saturated NH₄Cl (50ml). Ethyl acetate (200ml) was added and the layers were separated. The aqueous layer was extracted with ethyl acetate (200ml). The combined organic layers were washed with 10% sodium bisulfite (50ml), dried (Na₂SO₄), filtered and concentrated to give the crude product (5.05g). This material was chromatographed on silica gel (75g) eluting with 1:9 ethyl acetate-heptane and 3:7 ethyl acetate-heptane to give the pure product (4) (3.25g, 85.1% yield). ¹H NMR conformed to structure.

### Example 3a: Selective Dihydroxylation

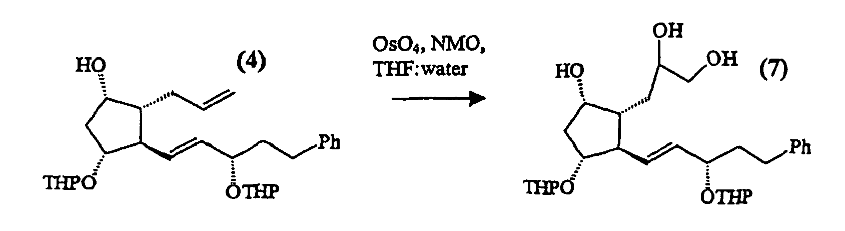

A solution of alkene (4) (2.5g, 5.3mmol) in THF (20ml) was cooled to -9°C and a solution of *N*-methylmorpholine oxide monohydrate (NMO, 1.6g, 11.7mmol) in water (4ml) was added. A solution of osmium tetroxide (60mg, 0.236mmol) in water (1.5my) was added slowly while keeping the reaction temperature below -7°C. After the addition was complete, the reaction mixture was stirred at -7 to -5°C for 4h. The reaction was quenched by the addition of solid sodium bisulfite (1.5g), the mixture stirred for 5min, and filtered through a bed of celite. The filter cake was washed with ethyl acetate, and the combined filtrate was washed with saturated aqueous sodium bicarbonate and water. The organic layer was dried (Na₂SO₄), filtered and concentrated to give the crude product as a dark brown oil. This oil was chromatographed on silica gel (50g) eluting with 1:99 methanol-dichloromethane and 5:95 methanol-dichloromethane to give the pure triol (7) as a yellow oil (2.3g, 86.8% yield). ¹H NMR conformed to structure.

### Example 3b: Selective Dihydroxylation

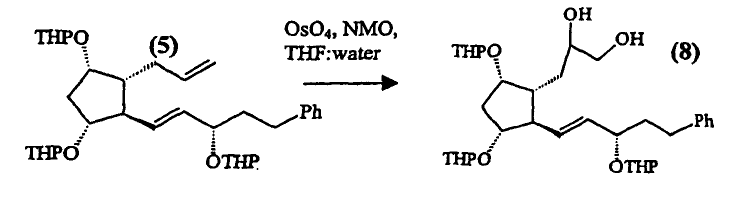

A solution of alkene (5) (11.37g, 20.17mmol) in THF (100ml) was cooled to -9°C and a solution of *N*-methylmorpholine oxide monohydrate (NMO, 6.84g, 50.42mmol) in water (20ml) was added. A solution of osmium tetroxide (256mg, 1.010mmol) in water (6.42ml) was added slowly while keeping the reaction temperature below -6.5°C. After the addition was complete, the reaction mixture was stirred at -10 to -7.8°C for 4h. The reaction was quenched by the addition of solid sodium bisulfite (6.84g), the mixture stirred for 5min, and filtered through a bed of celite. The filter cake was washed with ethyl acetate (200ml), and the combined filtrates were washed with saturated aqueous sodium bicarbonate and water. The organic layer was dried (Na₂SO₄), filtered and concentrated to give the crude product as a dark brown oil. This oil was chromatographed on silica gel (140g) eluting with 70:30 ethyl acetate-heptane, ethyl acetate, and 2:98 methanol-ethyl acetate to give the pure diol (8) (8.70g, 72.1% yield). ¹H NMR spectrum conformed to structure.

### Example 4a: Reduction

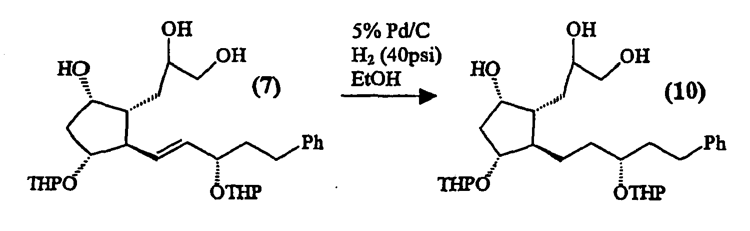

A solution of alkene (7) (2.3g, 4.6mmol) in ethanol (200ml) and 5% palladium on activated carbon (contains 50-60wt% water, 300mg) was stirred under 40psi of hydrogen for 4h. A fresh charge of catalyst (200mg) was added and the hydrogenation continued for 8h. ¹H NMR analysis indicated complete reduction of the double bond. The reaction mixture was filtered through a bed of celite and the filtrate was concentrated to give triol (10) as an oil (2.1g, 91.3% yield).

### Example 4b: Reduction

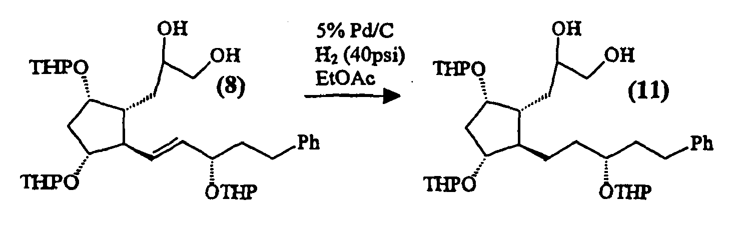

A solution of alkene (8) (9.32g, 15.59mmol) in ethyl acetate (200ml) and 5% palladium on activated carbon (contains 50-60wt% water, 1.0g) was stirred under 45psi of hydrogen for 18h. A fresh charge of catalyst (0.5g) was added and the hydrogenation continued for 2 days. A second charge of fresh catalyst (0.5g) was added and the hydrogenation continued for 1 day. ¹H NMR analysis indicated that the double bond was not completely reduced. The reaction mixture was filtered, concentrated to dryness, the residue dissolved in ethanol (200ml), and hydrogenated over 5% palladium on activated carbon (0.5 g, 40psi of hydrogen) for 24h. The reaction mixture was filtered through a bed of celite and the filtrate was concentrated to give diol (11) as a colourless oil (8.40g, 89.8% yield).

### Example 5a: Diol Cleavage

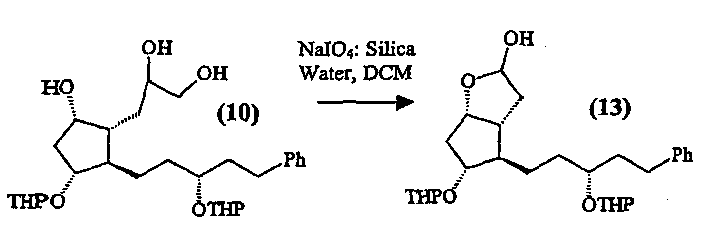

A mixture of sodium periodate (1.90g, 8.14mmol), silica gel (2.00g) and water (2ml) were stirred until a free flowing powder was obtained. This powder was added in one portion to a solution of triol (10) (2.10g, 4.14mmol) in dichloromethane (25ml) and the mixture was stirred at room temperature for 2h. The solids were removed by filtration through a short pad of Na₂SO₄ (5g), and the filtrate was concentrated to dryness. The crude product was chromatographed on silica gel (30g) eluting with 20:80 ethyl acetate-heptane to give the product (13) as an oil which solidified slowly on storage (1.65g, 83.8% yield). ¹H NMR conformed to structure.

### Example 5b: Diol Cleavage

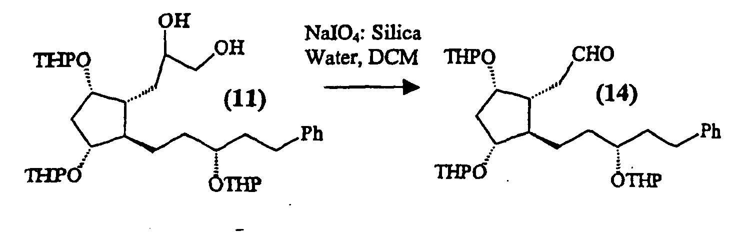

A mixture of sodium periodate (5.00g, 23.38mmol), silica gel (5.00g) and water (5ml) were stirred until a free flowing powder was obtained. This powder was added in one portion to a solution of diol (11) (7.00g, 11.69mmol) in dichloromethane (50ml) and the mixture was stirred at room temperature for 3h. The solids were removed by filtration through a short pad of Na₂SO₄ (10g), and the filtrate was concentrated to dryness. The crude product was chromatographed on silica gel (55g) eluting with 15:85 ethyl acetate-heptane to give aldehyde (2.25g, 33.9% yield). ¹H NMR spectrum conformed to structure.

### Example 5c: Diol Cleavage

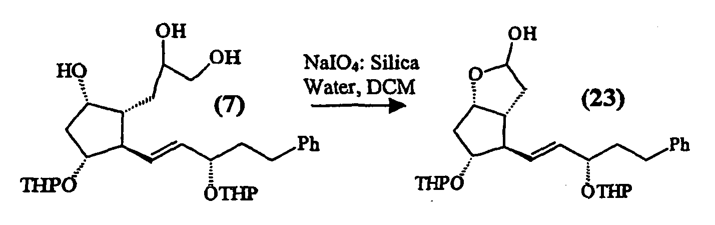

A mixture of sodium periodate (155.0g, 724.6mmol), silica gel (275g) and water (175ml) was stirred until a free flowing powder was obtained. This powder was added in portions to a solution of crude triol (7) (230g, 369mmol) in dichloromethane (1.51) and the mixture was stirred at room temperature for 4h. Solid anhydrous sodium sulfate (500g) was added and the mixture was stirred for 10min. The solids were collected by vacuum filtration, and the filtrate was passed through a plug of silica gel (750g). The plug was washed successively with dichloromethane (21) and ethyl acetate (2.51), and the combined filtrates were concentrated to dryness. The residue was dissolved in toluene (0.51) and concentrated to dryness to give crude lactol (23) (180g). ¹H NMR conformed to structure.

### Example 6a: Wittig Olefination

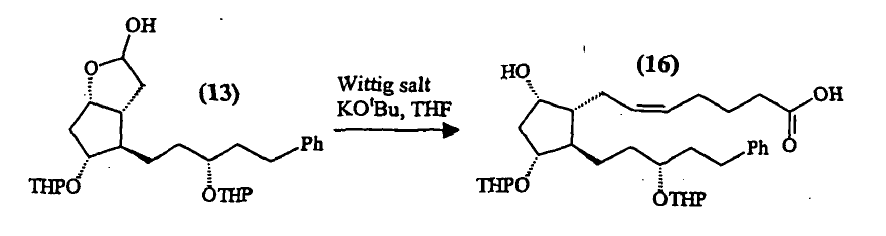

A dry 250ml 3-necked round bottom flask equipped with a magnetic stirring bar, a temperature probe, and rubber septa, was purged with nitrogen. The flask was charged with solid 4-carboxybutylphosphonium bromide (3.67g, 8.27mmol) and anhydrous THF (20ml), and cooled to -12°C in an ice-methanol bath. A solution of potassium *tert-*butoxide (1.86g, 16.54mmol) in anhydrous THF (20ml) was added by syringe keeping the internal temperature below -9°C. The resulting orange-red suspension was stirred for 1h between -9°C and -12°C. A solution of the lactol (13) (1.60g, 3.39mmol) in anhydrous THF (20ml) was added by syringe keeping the temperature below -6°C. The resulting suspension was stirred in the cold for 2h, and quenched by the addition of water (50ml). The THF was removed under reduced pressure and TBME (100ml) was added to the residue. The layers were separated, and the organic layer was washed with 6% aqueous sodium chloride (60ml). The combined aqueous layers were acidified to pH 4.0 with 5% aqueous citric acid, and extracted with ethyl acetate (2 x 100ml). The combined ethyl acetate extracts were washed with water (100ml), dried (Na₂SO₄), filtered and concentrated to a colorless syrup (1.90g) which was carried forward without further purification. ¹H NMR showed that this syrup is a mixture of acid (16) and unreacted Wittig salt.

### Example 6b: Wittig Olefination

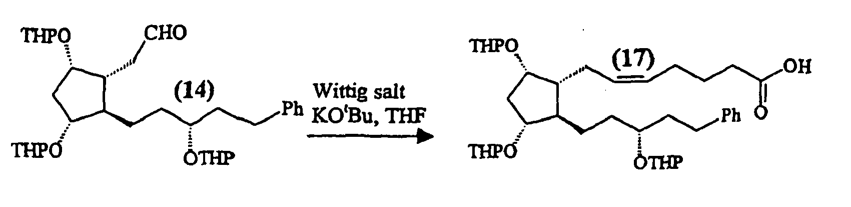

A dry 250ml 3-necked round bottom flask equipped with a magnetic stirring bar, a temperature probe, and rubber septa, was purged with nitrogen. The flask was charged with solid 4-carboxybutylphosphonium bromide (3.51g, 7.93mmol) and anhydrous THF (100ml), and cooled to -10°C in an ice-methanol bath. A solution of potassium *tert-*butoxide (1.78g, 15.85mmol) in anhydrous THF (10ml) was added by syringe. The resulting orange-red suspension was stirred for 1h at 0°C, cooled to -10°C, and a solution of aldehyde (14) (2.25g, 3.96mmol) in anhydrous THF (15ml) was added by syringe. The resulting suspension was stirred in the cold for 2h, and quenched by the addition of water (50ml). The THF was removed under reduced pressure and the mixture was washed with TBME (2 x 70ml). The combined organic layers were extracted with water (50ml). The combined aqueous layers were acidified to pH 3.0 with 5% aqueous citric acid, and extracted with ethyl acetate (2 x 100ml). The combined ethyl acetate extracts were dried (Na₂SO₄), filtered and concentrated to crude acid (17) (3.30g) which was carried forward without further purification.

### Example 6c: Wittig Olefination

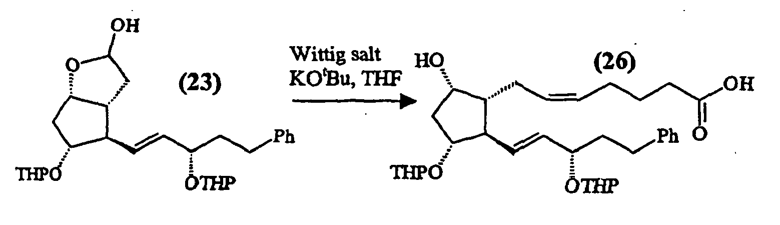

A dry 51 3-necked round bottom flask equipped with an overhead stirrer, a temperature probe, and rubber septa, was purged with nitrogen. The flask was charged with solid 4-carboxybutylphosphonium bromide (360.8g, 814.4mmol) and anhydrous THF (1.21), and cooled to -10°C in an ice-salt bath. A solution of potassium *tert*-butoxide (182.4g, 1628.5mmol) in anhydrous THF (0.51) was added by cannula keeping the internal temperature below 0°C. The resulting orange-red suspension was stirred for 2h below 0 °C. A solution of the lactol (23) (180g, 369mmol) in anhydrous THF (0.4ml) was added by cannula keeping the temperature below 0 °C. The resulting suspension was stirred in the cold for 1h, and quenched by the careful addition of water (0.61). The solvent was removed under reduced pressure and water (1.41) was added to the residue. The pH of the aqueous mixture was adjusted to between 4 and 4.5 by the addition of 40% aqueous citric acid (300ml), and extracted with ethyl acetate (1 x 1.51 and 1 x 0.61). The combined ethyl acetate extracts were washed with saturated brine (0.51), dried (Na₂SO₄), filtered, and the filtrate concentrated to dryness. The residue was triturated with acetone (1.01), filtered, and the filter cake was washed with acetone (2 x 0.61). The filtrates contained the crude acid (26), which was carried forward without further purification.

### Example 7a: Esterification

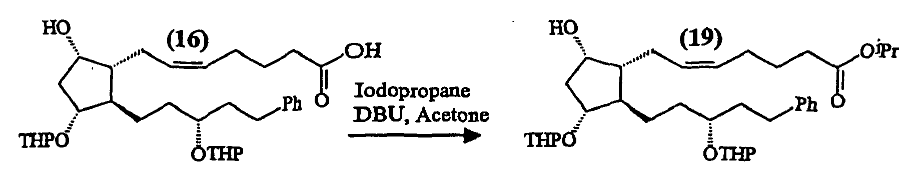

The crude olefination product from example 6a (1.84g) was dissolved in acetone (20ml), and the solution was cooled in an ice bath. The solution was treated with 1,8-diazabicyclo[5.4.0]undec-7-ene (DBU, 2.02g, 19.86mmol), and 2-iodopropane (3.38g, 13.24mmol), and allowed to stir at room temperature under an atmosphere of nitrogen for 16h. The reaction mixture was concentrated, and the residue was partitioned between ethyl acetate (100ml) and 5% aqueous citric acid (50ml). The layers were separated, the organic layer was washed with saturated aqueous sodium bicarbonate (20ml), and dried (Na₂SO₄). The drying agent was filtered off and the filtrate was concentrated to dryness. The residue was chromatographed on silica gel (30g) eluting with 15:85 ethyl acetate-heptane to give pure ester (19) (1.41 g, 69.2% yield from lactol (13)). ¹H NMR conformed to structure.

### Example 7b: Esterification

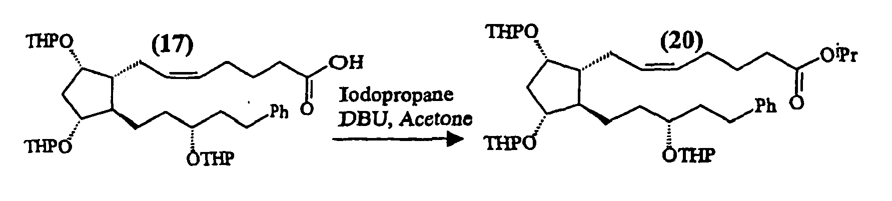

The crude olefination product from example 6b (3.30g) was dissolved in acetone (30ml), and the solution was cooled in an ice bath. The solution was treated with 1,8-diazabicyclo[5.4.0]undec-7-ene (DBU, 2.85g, 18.72mmol), and 2-iodopropane (4.77g, 28.06mmol), and allowed to stir at room temperature under an atmosphere of nitrogen for 64h. A voluminous precipitate formed. The reaction mixture was concentrated, and the residue was partitioned between ethyl acetate (170ml) and 5% aqueous citric acid (75ml). The layers were separated, the organic layer was washed with water (75ml), saturated aqueous sodium bicarbonate (75ml), and dried (Na₂SO₄). The drying agent was filtered off and the filtrate was concentrated to dryness. The residue was chromatographed on silica gel (30 g) eluting with 15:85 ethyl acetate-heptane to give pure ester (20) (2.18g, 80.7% yield from aldehyde (14)). ¹H NMR spectrum conformed to structure.

### Example 7c: Esterification

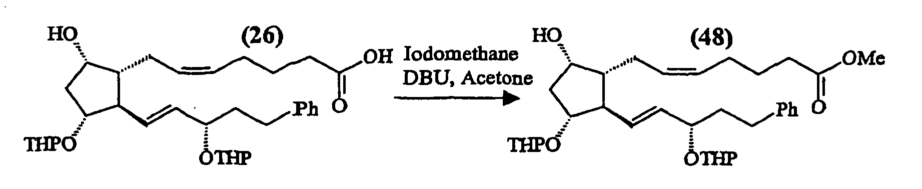

The combined filtrates containing acid (26) from example 6c were transferred into a dry 51 3-necked round bottom flask equipped with an overhead stirrer, a temperature probe, and addition funnel. The contents of the flask were cooled to 5°C in an ice bath, and combined with 1,8-diazabicyclo[5.4.0]undec-7-ene (DBU, 225.0g, 1480.6mmol), keeping the temperature below 10°C. Iodomethane (315.1g, 2221.2mmol) was slowly added at less than 15°C, and the mixture was stirred overnight at room temperature under an atmosphere of nitrogen. A second portion of iodomethane (105.0g, 740.4mmol) was added and the mixture stirred for 5h. The precipitated salts were filtered off, and the filtrate was concentrated to dryness. The residue was partitioned between ethyl acetate (2.51) and 10% aqueous citric acid (0.61). The layers were separated, and the aqueous layer was extracted with ethyl acetate (0.61). The combined organic layers were washed with 20% brine (0.61), saturated aqueous sodium bicarbonate (0.51), and dried (Na₂SO₄). The drying agent was filtered off and the filtrate was concentrated to dryness. The residue was chromatographed on silica gel (2kg) eluting with 15:85 ethyl acetate-heptane followed by 25:75 ethyl acetate-heptane to give pure ester (48) (86.8g) and a slightly contaminated product fraction (58.5g, 69.3% total yield from lactol (23)). ¹H NMR conformed to structure.

### Example 8a: Deprotection

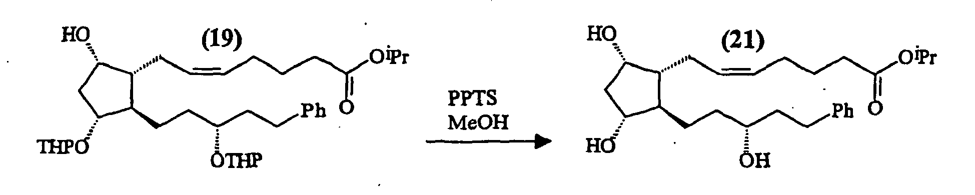

To a solution of ester (19) (1.41g, 2.35mmol) in methanol (20ml) was added pyridinium *p*-toluenesulfonate (PPTS, 30mg, 0.12mmol), and the mixture was heated in an oil bath that was maintained at 52°C for 4h. TLC indicated that all the starting material was consumed. Solid sodium bicarbonate (50mg) was added to the reaction mixture and the solvent was removed under reduced pressure. The residue was chromatographed on silica gel (30g) eluting with 30:70 ethyl acetate-heptane (11), 50:50 ethyl acetate-heptane (11), and 65:35 ethyl acetate-heptane (1.51) to give Latanoprost (21) as a colorless oil (910mg, 90.0% yield). ¹H NMR conformed to structure.

### Example 8b: Deprotection

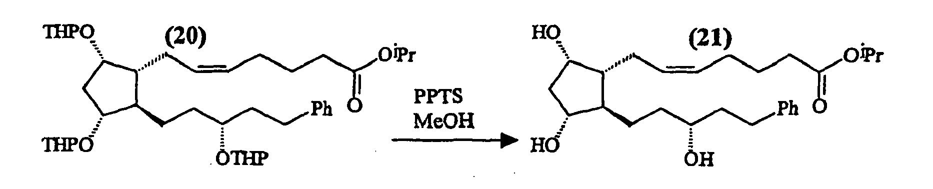

To a solution of ester (20) (2.15g, 3.15mmol) in methanol (40mL) was added pyridinium *p*-toluenesulfonate (PPTS, 10mg, 0.04mmol), and the mixture was heated in an oil bath that was maintained at 45°C for 6h. TLC indicated that all the starting material was consumed. Solid sodium bicarbonate (200mg) was added to the reaction mixture and the solvent was removed under reduced pressure. The residue was partitioned between ethyl acetate (150ml) and water. The layers were separated and the organic phase was washed with saturated sodium bicarbonate (25ml), and water (25ml). The solvent was removed and the residue was chromatographed on silica gel (40g) eluting with 35:65 ethyl acetate-heptane (11), and 50:50 ethyl acetate-heptane (11) to give Latanoprost (21) as a colorless oil (1.14g, 83.7% yield). ¹H NMR spectrum conformed to structure.

### Example 8c: Deprotection

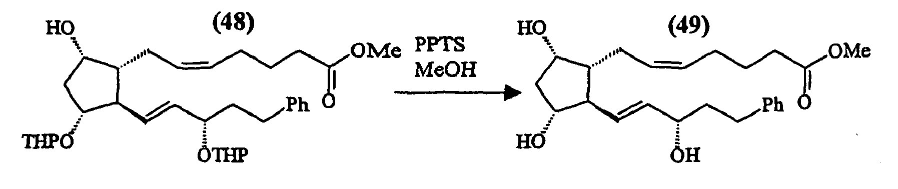

To a solution of ester (48) (86.7g, 152.6mmol) in methanol (600ml) was added pyridinium *p*-toluenesulfonate (PPTS, 1.0g, 4.0mmol), and the mixture was stirred at room temperature overnight, then at 40°C for 4h. TLC indicated that all the starting material was consumed. The solvent was removed under reduced pressure, and the residue was partitioned between ethyl acetate (1.01) and saturated aqueous sodium bicarbonate (300 mL). The layers were separated, and the aqueous layer was extracted with ethyl acetate (0.21). The combined organic layers were concentrated to dryness, the residue dissolved in methanol and concentrated to dryness giving crude ester (49) (62.4g) as an oil. ¹H NMR conformed to structure.

### Example 9: Amidation

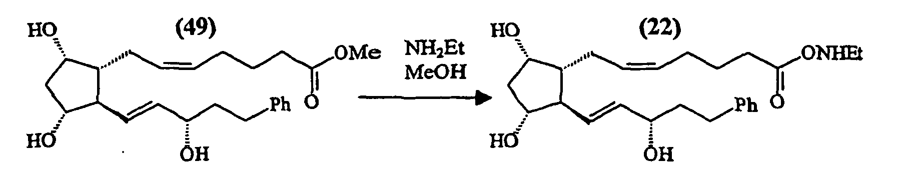

A 21 thick-walled glass vessel equipped with a magnetic stirring bar was charged with a solution of ester (49) (85.2g, 212.7mmol) in methanol (0.41) and 70% aqueous solution of ethylamine (0.41). The vessel was sealed, heated and stirred in an oil bath maintained at 90°C for 45h. TLC analysis showed consumption of ester (49). The volatiles were removed under reduced pressure, and the residue was partitioned between ethyl acetate (1.51) and saturated brine (0.71). The layers were separated, and the aqueous layer was extracted with ethyl acetate (2 x 0.51). The combined organic layers were dried (Na₂SO₄), filtered, and concentrated to dryness. The residue was chromatographed on silica gel (1kg) eluting successively with 80:20 ethyl acetate-heptane (41), ethyl acetate (121), 1:99 methanol-ethyl acetate (161), and 2:99 methanol-ethyl acetate (161). The resulting product (55.6g) was triturated with *tert*-butyl methyl ether to give Bimatoprost (22) as a white solid (47.2g). ¹H and ¹³C NMR conformed to structure.

## Claims

1. A process for the preparation of a compound of Formula (VIII): 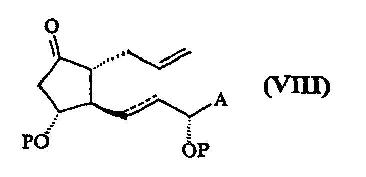 wherein A is (CH₂)₂Ph or CH₂OR"', wherein R"' is phenyl, substituted at the meta position with CF₃; P is a hydroxyl protecting group; and - - - - - represents a double bond or a single bond;
wherein a compound of Formula (IX): 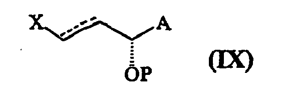 wherein A, P and - - - - - are as defined above and X is a leaving group, is reacted to form a cuprate reagent which undergoes a 1,4 addition reaction with a compound of Formula (X): 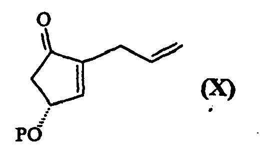 wherein P is as defined above.

2. A process according to claim 1, wherein P is a tetrahydropyranyl (THP) protecting group.

3. A process according to claim 1 or claim 2, wherein X is iodine.

4. A process according to claim 1, wherein A is (CH₂)₂Ph, - - - - - represents a double bond, P is THP and X is 1.

5. A compound of formula (VIII): 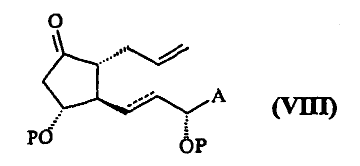 wherein A, P and - - - - - are as defined in claim 1.

6. A compound according to claim 5, wherein A is (CH₂)₂Ph, - - - - - represents a double bond and P is THP.

7. A process for the preparation of a compound of formula (VIIa): 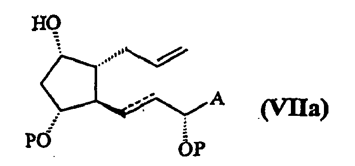 wherein A is (CH₂)₂Ph or CH₂OR"', wherein R"' is phenyl, substituted at the meta position with CF₃; P is a hydroxyl protecting group and - - - - - represents a double bond or a single bond;
wherein a compound of Formula (VIII): 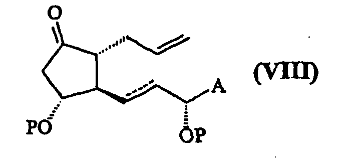 wherein A, P and - - - - - are as defined above, undergoes selective reduction.

8. A process according to claim 7, wherein P is a tetrahydropyranyl (THP) protecting group.

9. A process according to claim 7, wherein A is (CH₂)₂Ph, - - - - - represents a double bond and P is THP.

10. A compound of formula (VIIa): 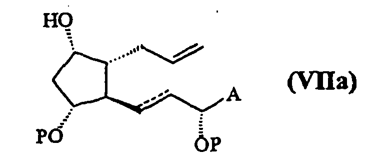 wherein A, P and - - - - - are as defined in claim 7.

11. A compound according to claim 10, wherein A is (CH₂)₂Ph, - - - - - represents a double bond and P is THP.

12. A process for the preparation of a compound of formula (VIIb): 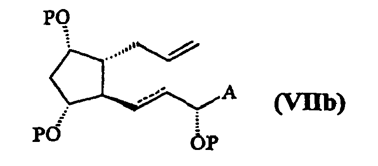 wherein A is (CH₂)₂Ph or CH₂OR"', wherein R"' is phenyl, substituted at the meta position with CF₃; P is a hydroxyl protecting group and - - - - - represents a double bond or a single bond;
wherein a compound of Formula (VIIa): 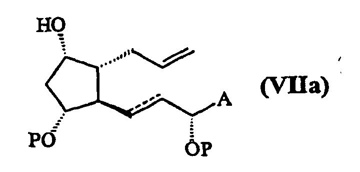 wherein A, P and - - - - - are as defined above, is protected with a hydroxyl protecting group.

13. A process according to claim 12, wherein P is a tetrahydropyranyl (THP) protecting group.

14. A process according to claim 12, wherein A is (CH₂)₂Ph, - - - - - represents a double bond and P is THP.

15. A compound of formula (VIIb): 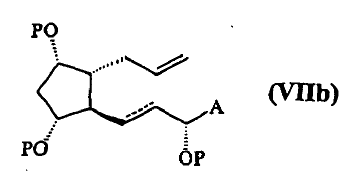 wherein A, P and - - - - - are as defined in claim 12.

16. A compound according to claim 15, wherein A is (CH₂)₂Ph, - - - - - represents a double bond and P is THP.

17. A process for the preparation of a compound of formula (VIIc): 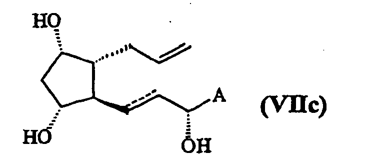 wherein A is (CH₂)₂Ph or CH₂OR"', wherein R"' is phenyl, substituted at the meta position with CF₃ and - - - - - represents a double bond or a single bond;
wherein a compound of Formula (VIIa): 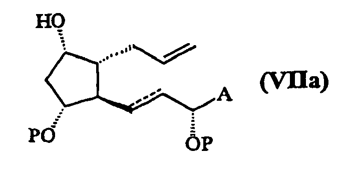 wherein A and - - - - - are as defined above and P is a protecting group, is deprotected.

18. A process according to claim 17, wherein P is a tetrahydropyranyl (THP) protecting group.

19. A process according to claim 17 or claim 18, wherein A is (CH₂)₂Ph and - - - - - represents a double bond:

20. A compound of formula (VIIc): 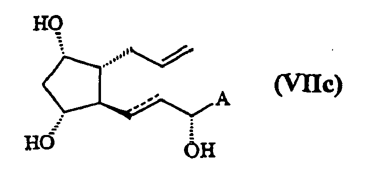 wherein A and - - - - - are as defined in claim 17.

21. A compound according to claim 20, wherein A is (CH₂)₂Ph and - - - - - represents a double bond.

22. A process for the preparation of a compound of formula (VIa), (VIb), (VIc), (Va), (Vb) or (Vc): 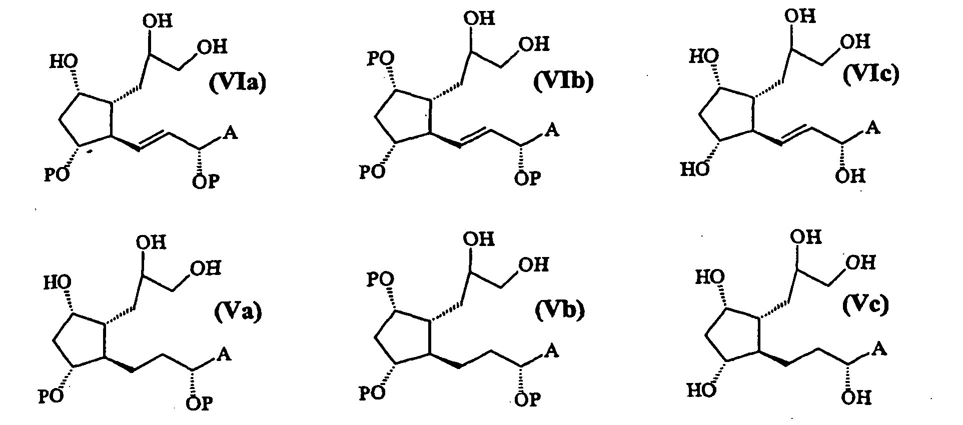 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; and P is a hydroxyl protecting group;
wherein a compound of Formula (VIIa), a compound of Formula (VIIb) or a compound of Formula (VIIc): 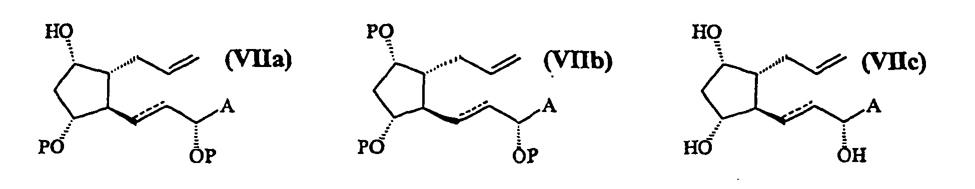 wherein A and P are as defined above and - - - - - is a double or single bond, undergoes dihydroxylation.

23. A process according to claim 22, wherein P is a tetrahydropyranyl (THP) protecting group.

24. A process according to claim 22, wherein A is (CH₂)₂Ph, P is THP, - - - - - represents a double bond, and compound (VIIa) is reacted to give compound (VIa).

25. A process according to claim 22, wherein A is (CH₂)₂Ph, P is THF, - - - - - represents a double bond, and compound (VIIb) is reacted to give compound (VIb).

26. A process according to claim 22, wherein A is (CH₂)₂Ph, - - - - - represents a double bond, and compound (VIIc) is reacted to give compound (VIc).

27. A compound of formula (VIa), (VIb) (VIc), (Va), (Vb) or (Vc): 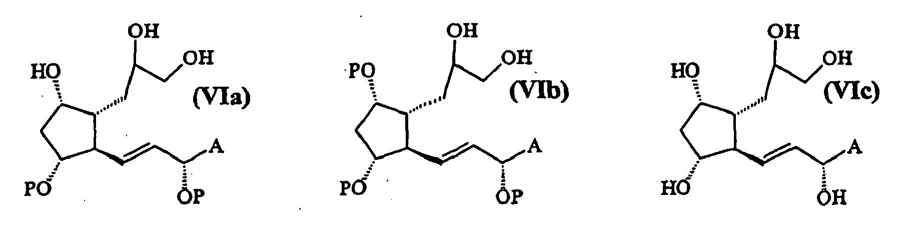 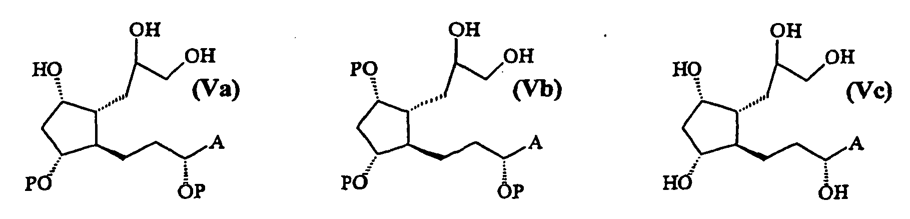 wherein A and P are as defined in claim 22.

28. A compound according to claim 27, wherein A is (CH₂)₂Ph and P is THP.

29. A process for the preparation of a compound of formula (Va), (Vb) or (Vc): 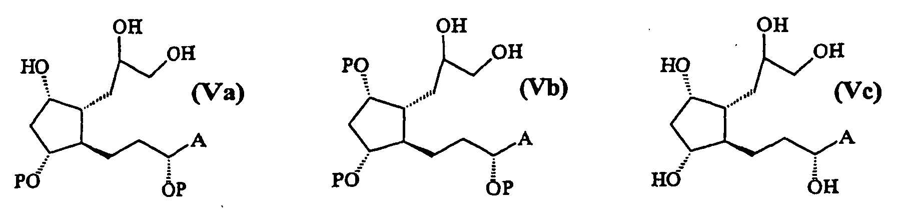 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; and P is a hydroxyl protecting group;
wherein a compound of Formula (VIa), a compound of Formula (VIb) or a compound of Formula (VIc): 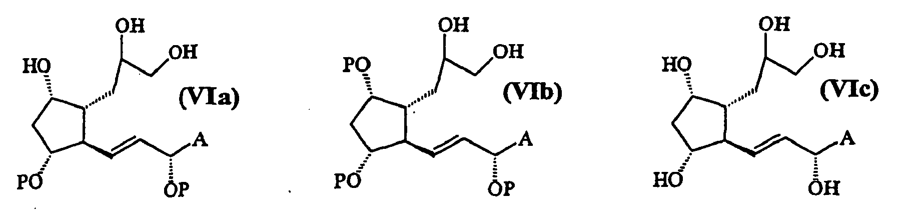 wherein A and P are as defined above, undergoes reduction of the double bond.

30. A process according to claim 29, wherein P is a tetrahydropyranyl (THP) protecting group.

31. A process according to claim 29, wherein A is (CH₂)₂Ph, P is THP and compound (VIa) is reacted to give compound (Va).

32. A process according to claim 29, wherein A is (CH₂)₂Ph, P is THP and compound (VIb) is reacted to give compound (Vb).

33. A process according to claim 29, wherein A is (CH₂)₂Ph and compound (VIc) is reacted to give compound (Vc).

34. A process for the preparation of a compound of formula (IVa), (IVb) or (IVc): 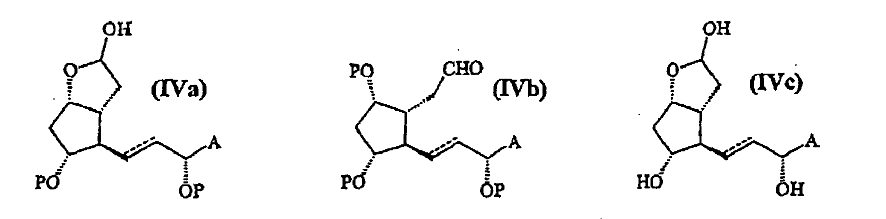 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; P is a hydroxyl protecting group and - - - - - represents a double bond or a single bond;
wherein a compound of Formula (VIa), (Va), (VIb), (Vb), (VIc) or (Vc): 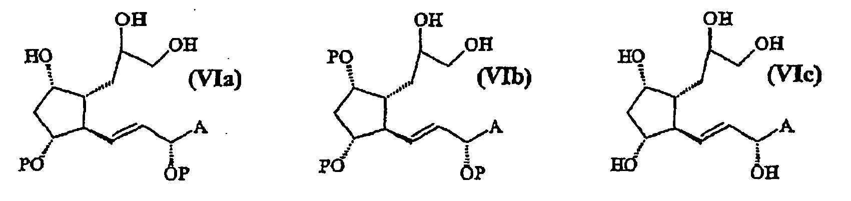 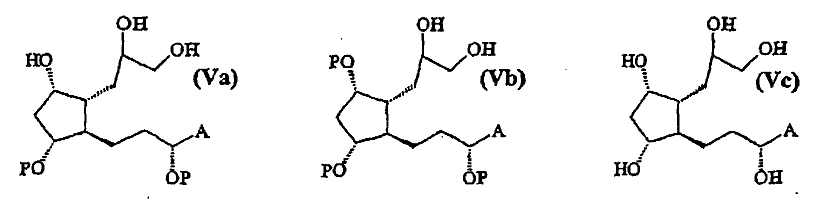 wherein A and P are as defined above, undergoes diol cleavage.

35. A process according to claim 34, wherein P is a tetrahydropyranyl (THP) protecting group.

36. A process according to claim 34, wherein A is (CH₂)₂Ph, P is THP, - - - - - represents a single bond, and compound (Va) is reacted to give compound (IVa).

37. A process according to claim 34, wherein A is (CH₂)₂Ph, P is THP, - - - - - represents a single bond, and compound (Vb) is reacted to give compound (IVb).

38. A process according to claim 34, wherein A is (CH₂)₂Ph, - - - - - represents a single bond, and compound (Vc) is reacted to give compound (IVc).

39. A compound of formula (IVb) wherein A is (CH₂)₂Ph, P is THP and - - - - - is a single bond:

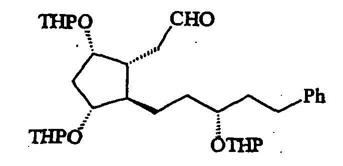

40. A process for the preparation of a prostaglandin compound having the formula (I): 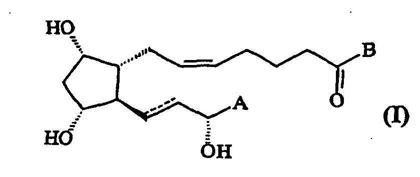 wherein A is selected from the group consisting of C₁-C₆ alkyl; C₇-C₁₆ aralkyl wherein the aryl group is optionally substituted with one to three substituents selected from the group consisting of C₁-C₆ alkyl, halo and CF₃; and (CH₂)ₙOR' wherein n is from 1 to 3 and R' represents a C₆-C₁₀ aryl group which is optionally substituted with one to three substituents selected from the group consisting of C₁-C₆ alkyl, halo and CF₃; B is selected from OR" and NHR" wherein R" is C₁-C₆ alkyl; and - - - - - represents a double bond or a single bond;
comprising a process according to any one of claims 1-4, 7-9, 12-14, 17-19, 22-26 and 29-38.

41. A process for synthesising Latanoprost comprising the steps of:
a) a 1,4 addition reaction according to claim 4,
b) a selective reduction according to claim 9,
c) a dihydroxylation according to claim 24,
d) a reduction according to claim 31,
e) a diol cleavage according to claim 36,
f) a Wittig reaction wherein a compound of formula (IVa) wherein A is (CH₂)₂Ph, P is THP and - - - - - represents a single bond, is reacted to give a compound of formula (IIIa) wherein A is (CH₂)₂Ph, P is THP and - - - - - represents a single bond: 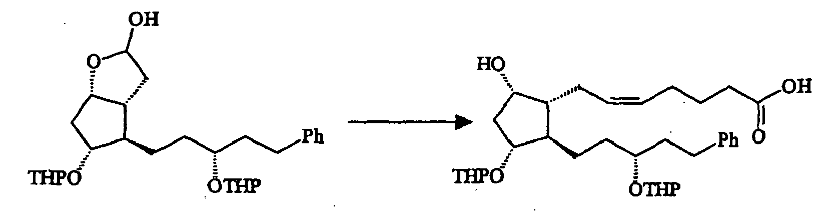
g) an esterification wherein a compound of formula (IIIa) wherein A is (CH₂)₂Ph, P is THP and - - - - - represents a single bond, is reacted to give a compound of formula (IIa) wherein A is (CH₂)₂Ph, P is THP, - - - - - represents a single bond and B is OⁱPr: 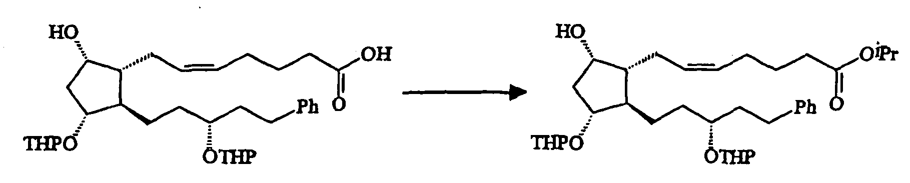 and
h) a deprotection wherein a compound of formula (IIa) wherein A is (CH₂)₂Ph, P is THP, - - - - - represents a single bond and B is OⁱPr, is reacted to give Latanoprost:

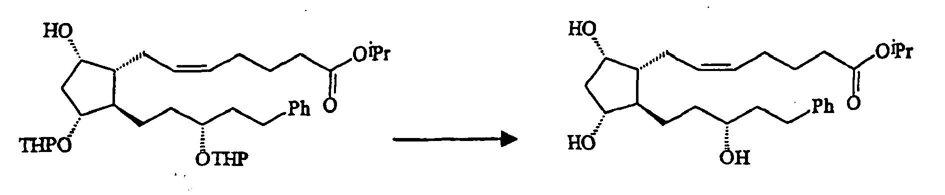

42. A process for synthesising Latanoprost comprising the steps of:
a) a 1,4 addition reaction according to claim 4,
b) a selective reduction according to claim 9,
c) a protection step according to claim 14,
d) a dihydroxylation according to claim 25,
e) a reduction according to claim 32,
f) a diol cleavage according to claim 37,
g) a Wittig reaction wherein a compound of formula (IVb) wherein A is (CH₂)₂Ph, P is THP and - - - - - represents a single bond, is reacted to give a compound of formula (IIIb) wherein A is (CH₂)₂Ph, P is THP and - - - - - represents a single bond: 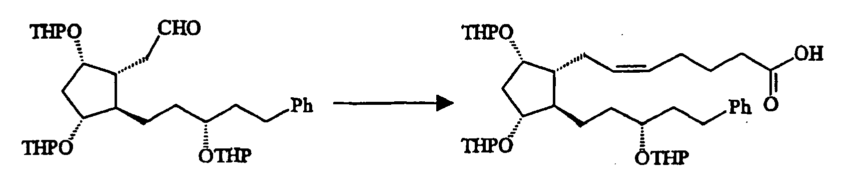
h) an esterification wherein a compound of formula (IIIb) wherein A is (CH₂)₂Ph, P is THP and - - - - - represents a single bond, is reacted to give a compound of formula (IIb) wherein A is (CH₂)₂Ph, P is THP, - - - - - represents a single bond and B is OⁱPr: 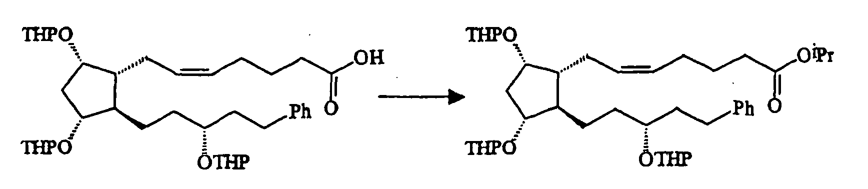 and
i) a deprotection wherein a compound of formula (IIb) wherein A is (CH₂)₂Ph, P is THP, - - - - - represents a single bond and B is OⁱPr, is reacted to give Latanoprost:

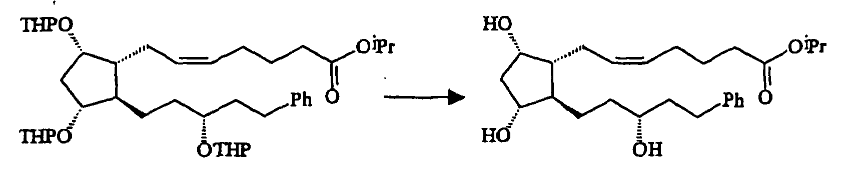

43. A process for synthesising Latanoprost comprising the steps of:
a) a 1,4 addition reaction according to claim 4,
b) a selective reduction according to claim 9,
c) a deprotection step according to claim 19,
d) a dihydroxylation according to claim 26,
e) a reduction according to claim 33,
f) a diol cleavage according to claim 38,
g) a Wittig reaction wherein a compound of formula (IVc) wherein A is (CH₂)₂Ph and - - - - - represents a single bond, is reacted to give a compound of formula (IIIc) wherein A is (CH₂)₂Ph and - - - - - represents a single bond: 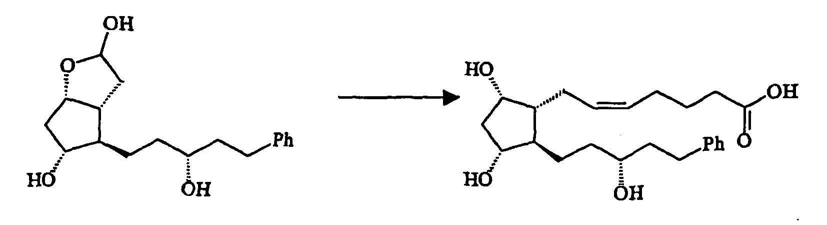 and
h) an esterification wherein a compound of formula (IIIc) wherein A is (CH₂)₂Ph and - - - - - represents a single bond, is reacted to give a compound of formula (I) wherein A is (CH₂)₂Ph, P is THP, - - - - - represents a single bond and B is OⁱPr:

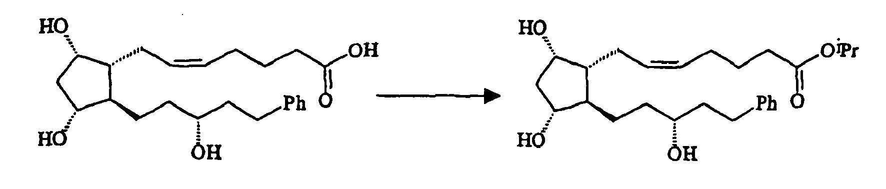

44. A process for synthesising Bimatoprost comprising the steps of:
a) a 1,4 addition reaction according to claim 4,
b) a selective reduction according to claim 9,
c) a dihydroxylation according to claim 24,
d) a diol cleavage according to claim 34, wherein compound (VIa) wherein A is (CH₂)₂Ph and P is THP, is reacted to give compound (IVa) wherein A is (CH₂)₂Ph, P is THP and - - - - - represents a double bond,
e) a Wittig reaction wherein a compound of formula (IVa) wherein A is (CH₂)₂Ph, P is THP and - - - - - represents a double bond, is reacted to give a compound of formula (IIIa) wherein A is (CH₂)₂Ph, P is THP and - - - - - represents a double bond: 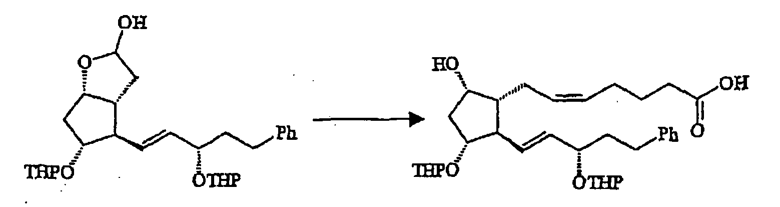
f) an amidation wherein a compound of formula (IIIa) wherein A is (CH₂)₂Ph, P is THP and - - - - - represents a double bond, is reacted to give a compound of formula (IIa) wherein A is (CH₂)₂Ph, P is THP, - - - - - represents a double bond and B is NHEt: 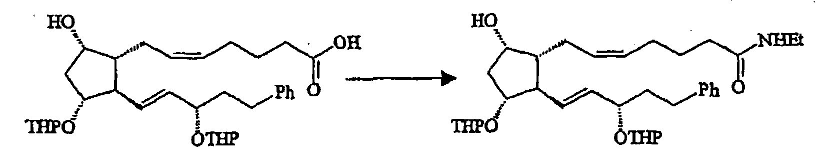 and
g) a deprotection wherein a compound of formula (IIa) wherein A is (CH₂)₂Ph, P is THP, - - - - - represents a double bond and B is NHEt, is reacted to give Bimatoprost:

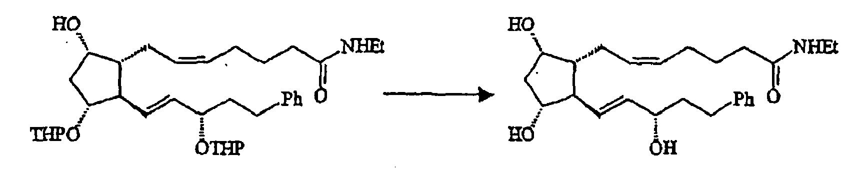

45. A process for synthesising Bimatoprost comprising the steps of:
a) a 1,4 addition reaction according to claim 4,
b) a selective reduction according to claim 9,
c) a protection step according to claim 14,
d) a dihydroxylation according to claim 25,
e) a diol cleavage according to claim 34, wherein compound (VIb) wherein A is (CH₂)₂Ph and P is THP, is reacted to give compound (IVb) wherein A is (CH₂)₂Ph, P is THP and - - - - - represents a double bond,
f) a Wittig reaction wherein a compound of formula (IVb) wherein A is (CH₂)₂Pb, P is THP and - - - - - represents a double bond, is reacted to give a compound of formula (IIIb) wherein A is (CH₂)₂Ph, P is THP and - - - - - represents a double bond: 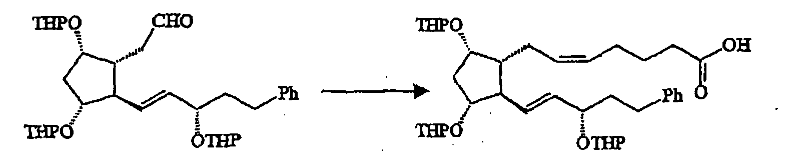
g) an amidation wherein a compound of formula (IIIb) wherein A is (CH₂)₂Ph, P is THP and - - - - - represents a double bond, is reacted to give a compound of formula (IIb) wherein A is (CH₂)₂Ph, P is THP, - - - - - represents a single bond and B is NHEt: 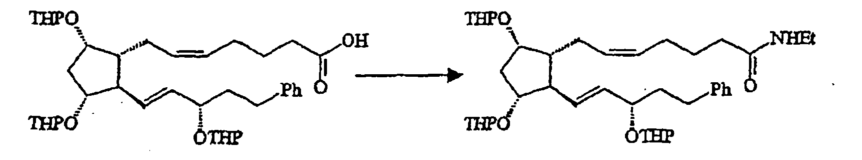 and
h) a deprotection wherein a compound of formula (IIb) wherein A is (CH₂)₂Ph, P is THP, - - - - - represents a double bond and B is NHEt, is reacted to give Bimatoprost:

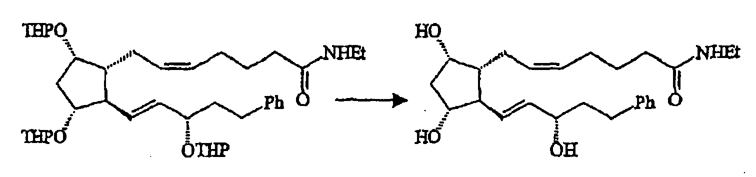

46. A process for synthesising Bimatoprost comprising the steps of:
a) a 1,4 addition reaction according to claim 4,
b) a selective reduction according to claim 9,
c) a deprotection step according to claim 19,
d) a dihydroxylation according to claim 26,
e) a diol cleavage according to claim 34, wherein compound (VIc) wherein A is (CH₂)₂Ph, is reacted to give compound (IVc) wherein A is (CH₂)₂Ph and - - - - - represents a double bond,
f) a Wittig reaction wherein a compound of formula (IVc) wherein A is (CH₂)₂Ph and - - - - - represents a double bond, is reacted to give a compound of formula (IIIc) wherein A is (CH₂)₂Ph and - - - - - represents a double bond: 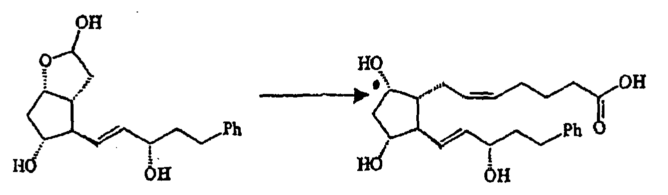 and
g) an amidation wherein a compound of formula (IIIc) wherein A is (CH₂)₂Ph and - - - - - represents a double bond, is reacted to give Bimatoprost:

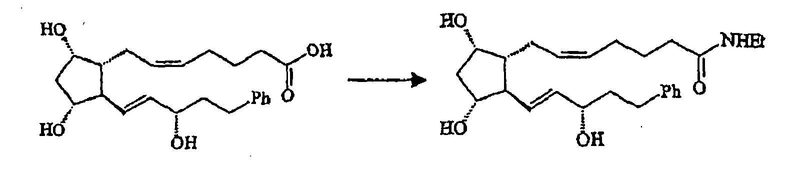

47. A process for synthesising Travoprost comprising a process according to any one of claims 1-3, 7-8, 12-13, 17-18, 22-23, 29-30 and 34-35.

## Patentansprüche

1. Verfahren zur Herstellung einer Verbindung der Formel (VIII): 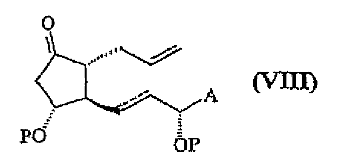 wobei A (CH₂)₂Ph oder CH₂OR"' ist, wobei R"' Phenyl ist, das an der *meta-*Stellung mit CF₃ substituiert ist; P eine Hydroxyl-Schutzgruppe ist; und - - - - - eine Doppelbindung oder eine Einfachbindung repräsentiert;
wobei eine Verbindung der Formel (IX): 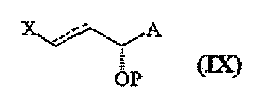 wobei A, P und - - - - - wie oben definiert sind und X eine Abgangsgruppe ist, zur Reaktion gebracht wird, um ein Cuprat-Reagans zu bilden, das einer 1,4-Additionsreaktion mit einer Verbindung der Formel (X): 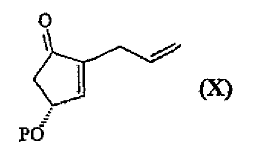 wobei P wie oben definiert ist, unterzogen wird.

2. Verfahren nach Anspruch 1, wobei P eine Tetrahydropyranyl-Schutzgruppe (THP-Schutzgruppe) ist.

3. Verfahren nach Anspruch 1 oder Anspruch 2, wobei X Iod ist.

4. Verfahren nach Anspruch 1, wobei A (CH₂)₂Ph ist, - - - - - eine Doppelbindung repräsentiert, P THP ist und X I ist.

5. Verbindung der Formel (VIII): 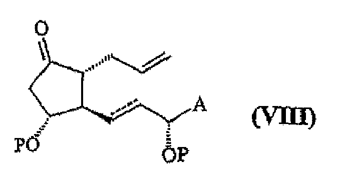 wobei A, P und - - - - - wie in Anspruch 1 definiert sind.

6. Verbindung nach Anspruch 5, wobei A (CH₂)₂Ph ist, - - - - - eine Doppelbindung repräsentiert und P THP ist.

7. Verfahren zur Herstellung einer Verbindung der Formel (Vlla): 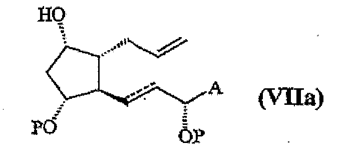 wobei A (CH₂)₂Ph oder CH₂OR"' ist, wobei R"' Phenyl ist, das an der *meta-*Stellung mit CF₃ substituiert ist; P eine Hydroxyl-Schutzgruppe ist und - - - - - eine Doppelbindung oder eine Einfachbindung repräsentiert;
wobei eine Verbindung der Formel (VIII): 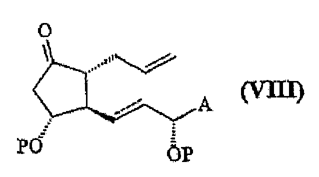 wobei A, P und - - - - - wie oben definiert sind, einer selektiven Reduktion unterzogen wird.

8. Verfahren nach Anspruch 7, wobei P eine Tetrahydropyranyl-Schutzgruppe (THP-Schutzgruppe) ist.

9. Verfahren nach Anspruch 7, wobei A (CH₂)₂Ph ist, - - - - - eine Doppelbindung repräsentiert und P THP ist.

10. Verbindung der Formel (VIIa): 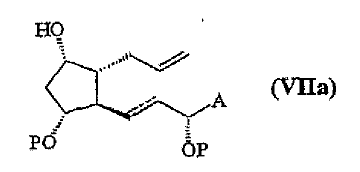 wobei A, P und - - - - - wie in Anspruch 7 definiert sind.

11. Verbindung nach Anspruch 10, wobei A (CH₂)₂Ph ist, - - - - - eine Doppelbindung repräsentiert und P THP ist.

12. Verfahren zur Herstellung einer Verbindung der Formel (VIIb): 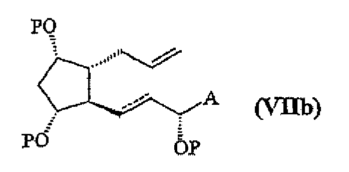 wobei A (CH₂)₂Ph oder CH₂OR"' ist, wobei R"' Phenyl ist, das an der *meta-*Stellung mit CF₃ substituiert ist; P eine Hydroxyl-Schutzgruppe ist und - - - - - eine Doppelbindung oder eine Einfachbindung repräsentiert;
wobei eine Verbindung der Formel (VIIa): 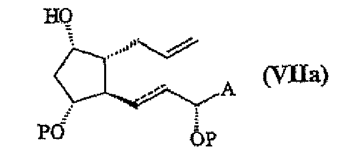 wobei A, P und - - - - - wie oben definiert sind, mit einer Hydroxyl-Schutzgruppe geschützt wird.

13. Verfahren nach Anspruch 12, wobei P eine Tetrahydropyranyl-Schutzgruppe (THP-Schutzgruppe) ist.

14. Verfahren nach Anspruch 12, wobei A (CH₂)₂Ph ist, - - - - - eine Doppelbindung repräsentiert und P THP ist:

15. Verbindung der Formel (VIIb): 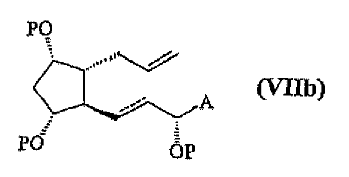 wobei A, P und - - - - - wie in Anspruch 12 definiert sind.

16. Verbindung nach Anspruch 15, wobei A (CH₂)₂Ph ist, - - - - - eine Doppelbindung repräsentiert und P THP ist.

17. Verfahren zur Herstellung einer Verbindung der Formel (Vllc): 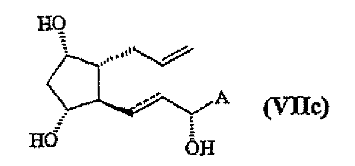 wobei A (CH₂)₂Ph oder CH₂OR"' ist, wobei R"' Phenyl ist, das an der *meta-*Stellung mit CF₃ substituiert ist und - - - - - eine Doppelbindung oder eine Einfachbindung repräsentiert;
wobei eine Verbindung der Formel (Vlla): 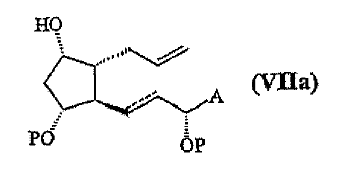 wobei A und - - - - - wie oben definiert sind und P eine Schutzgruppe ist, entschützt wird.

18. Verfahren nach Anspruch 17, wobei P eine Tetrahydropyranyl-Schutzgruppe (THP-Schutzgruppe) ist.

19. Verfahren nach Anspruch 17 oder Anspruch 18, wobei A (CH₂)₂Ph ist und - - - - - eine Doppelbindung repräsentiert.

20. Verbindung der Formel (VIIc): 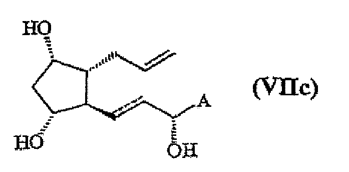 wobei A und - - - - - wie in Anspruch 17 definiert sind.

21. Verbindung nach Anspruch 20, wobei A (CH₂)₂Ph ist und - - - - - eine Doppelbindung repräsentiert.

22. Verfahren zur Herstellung einer Verbindung der Formel (VIa), (Vlb), (Vlc), (Va), (Vb) oder (Vc): 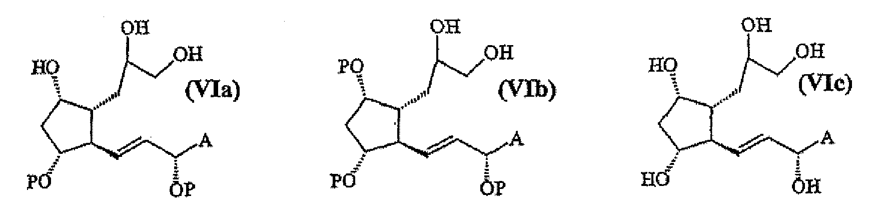 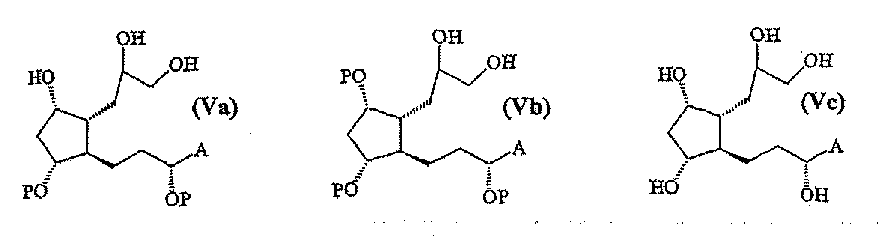 wobei A ausgewählt wird aus der Gruppe bestehend aus C₁-C₆-Alkyl; C₇-C₁₆-Aralkyl, wobei die Arylgruppe gegebenenfalls mit einem bis drei Substituenten substituiert ist, die aus der Gruppe bestehend aus C₁-C₆-Alkyl, Halogen und CF₃ ausgewählt werden; und (CH₂)ₙOR', wobei n 1 bis 3 ist und R' eine C₆-C₁₀-Arylgruppe repräsentiert, die gegebenenfalls mit einem bis drei Substituenten substituiert ist, die aus der Gruppe bestehend aus C₁-C₆-Alkyl, Halogen und CF₃ ausgewählt werden; und P eine Hydroxyl-Schutzgruppe ist;
wobei eine Verbindung der Formel (VIIa), eine Verbindung der Formel (Vllb) oder eine Verbindung der Formel (Vllc): 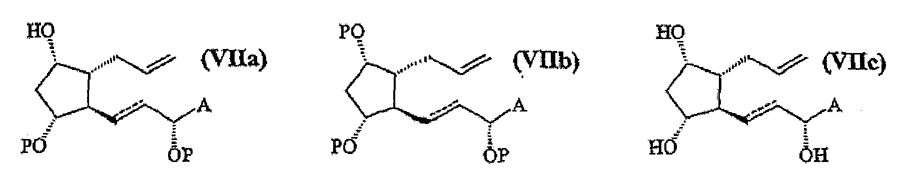 wobei A und P wie oben definiert sind und - - - - - eine Doppel- oder Einfachbindung ist, einer Dillydrooylierung unterzogen wird.

23. Verfahren nach Anspruch 22, wobei P eine Tetrahydropyranyl-Schutzgruppe (THP-Schutzgruppe) ist.

24. Verfahren nach Anspruch 22, wobei A (CH₂)₂Ph ist, P THP ist, - - - - - eine Doppelbindung repräsentiert und die Verbindung (VIIa) zur Reaktion gebracht wird, um die Verbindung (VIa) zu ergeben.

25. Verfahren nach Anspruch 22, wobei A (CH₂)₂Ph ist, P THP ist, - - - - - eine Doppelbindung repräsentiert und die Verbindung (Vllb) zur Reaktion gebracht wird, um die Verbindung (Vlb) zu ergeben.

26. Verfahren nach Anspruch 22, wobei A (CH₂)₂Ph ist, - - - - - eine Doppelbindung repräsentiert und die Verbindung (Vllc) zur Reaktion gebracht wird, um die Verbindung (VIc) zu ergeben.

27. Verbindung der Formel (VIa), (Vlb), (VIc), (Va), (Vb) oder (Vc): 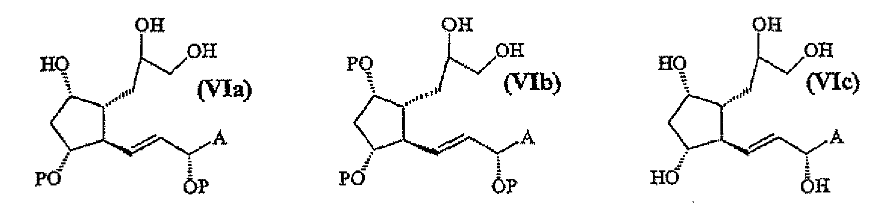 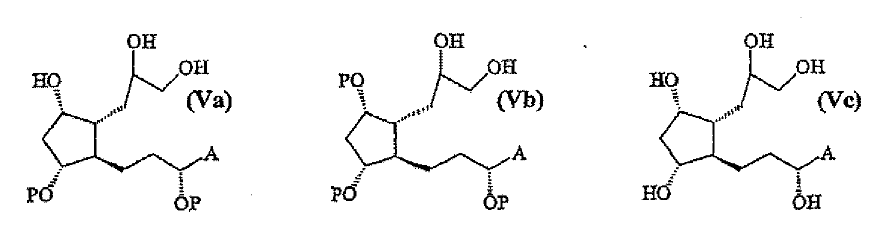 wobei A und P wie in Anspruch 22 definiert sind.

28. Verbindung nach Anspruch 27, wobei A (CH₂)₂Ph ist und P THP ist.

29. Verfahren zur Herstellung einer Verbindung der Formel (Va), (Vb) oder (Vc): 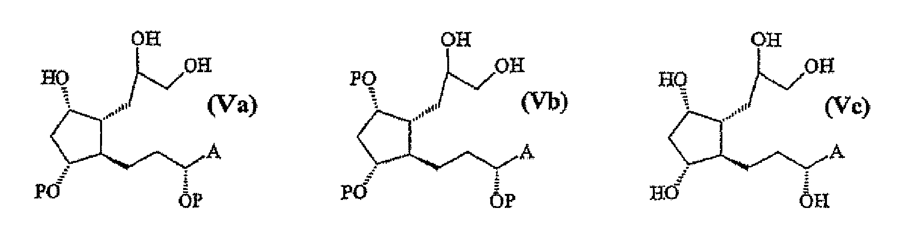 wobei A ausgewählt wird aus der Gruppe bestehend aus C₁-C₆-Alkyl; C₇-C₁₆-Aralkyl, wobei die Arylgruppe gegebenenfalls mit einem bis drei Substituenten substituiert ist, die aus der Gruppe bestehend aus C₁-C₆-Alkyl, Halogen und CF₃ ausgewählt werden; und (CH₂)ₙOR', wobei n 1 bis 3 ist und R' eine C₆-C₁₀-Arylgruppe repräsentiert, die gegebenenfalls mit einem bis drei Substituenten substituiert ist, die aus der Gruppe bestehend aus C₁-C₆-Alkyl, Halogen und CF₃ ausgewählt werden; und P eine Hydroxyl-Schutzgruppe ist;
wobei eine Verbindung der Formel (VIa), eine Verbindung der Formel (VIb) oder eine Verbindung der Formel (VIc): 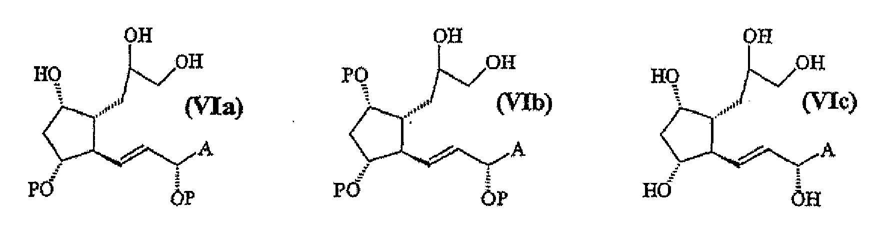 wobei A und P wie oben definiert sind, einer Reduktion der Doppelbindung unterzogen wird.

30. Verfahren nach Anspruch 29, wobei P eine Tetrahydropyranyl-Schutzgruppe (THP-Schutzgruppe) ist.

31. Verfahren nach Anspruch 29, wobei A (CH₂)₂Ph ist, P THP ist und die Verbindung (VIa) zur Reaktion gebracht wird, um die Verbindung (Va) zu ergeben.

32. Verfahren nach Anspruch 29, wobei A (CH₂)₂Ph ist, P THP ist und die Verbindung (VIb) zur Reaktion gebracht wird, um die Verbindung (Vb) zu ergeben.

33. Verfahren nach Anspruch 29, wobei A (CH₂)₂Ph ist und die Verbindung (Vlc) zur Reaktion gebracht wird, um die Verbindung (Vc) zu ergeben.

34. Verfahren zur Herstellung einer Verbindung der Formel (IVa), (IVb) oder (IVc): 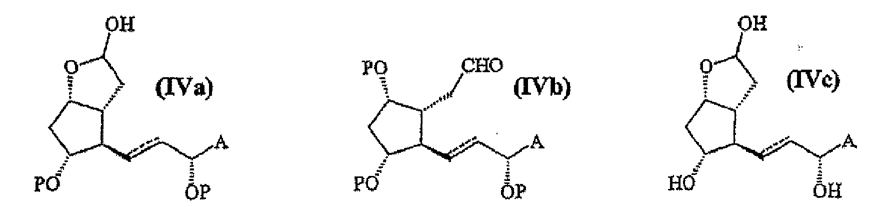 wobei A ausgewählt wird aus der Gruppe bestehend aus C₁-C₆-Alkyl; C₇-C₁₆-Aralkyl, wobei die Arylgruppe gegebenenfalls mit einem bis drei Substituenten substituiert ist, die aus der Gruppe bestehend aus C₁-C₆-Alkyl, Halogen und CF₃ ausgewählt werden; und (CH₂)ₙOR', wobei n 1 bis 3 ist und R' eine C₆-C₁₀-Arylgruppe repräsentiert, die gegebenenfalls mit einem bis drei Substituenten substituiert ist, die aus der Gruppe bestehend aus C₁-C₆-Alkyl, Halogen und CF₃ ausgewählt werden; P eine Hydroxyl-Schutzgruppe ist und - - - - - eine Doppelbindung oder eine Einfachbindung repräsentiert;
wobei eine Verbindung der Formel (VIa), (Va), (Vlb), (Vb), (Vlc) oder (Vc): 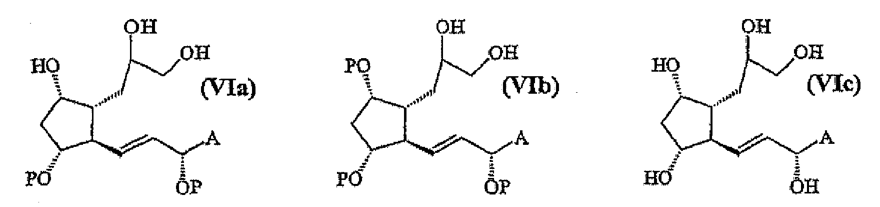 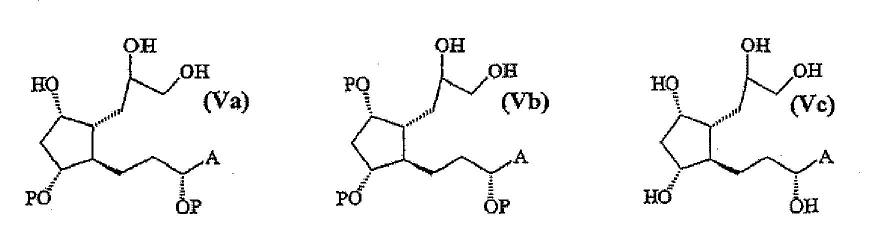 wobei A und P wie oben definiert sind, einer Diolspaltung unterzogen wird.

35. Verfahren nach Anspruch 34, wobei P eine Tetrahydropyranyl-Schutzgruppe (THP-Schutzgruppe) ist.

36. Verfahren nach Anspruch 34, wobei A (CH₂)₂Ph ist, P THP ist, - - - - - eine Einfachbindung repräsentiert und die Verbindung (Va) zur Reaktion gebracht wird, um die Verbindung (Iva) zu ergeben.

37. Verfahren nach Anspruch 34, wobei A (CH₂)₂Ph ist, P THP ist, - - - - - eine Einfachbindung repräsentiert und die Verbindung (Vb) zur Reaktion gebracht wird, um die Verbindung (Ivb) zu ergeben,

38. Verfahren nach Anspruch 34, wobei A (CH₂)₂Ph ist, - - - - - eine Einfachbindung repräsentiert und die Verbindung (Vc) zur Reaktion gebracht wird, um die Verbindung (Ivc) zu ergeben.

39. Verbindung der Formel (Ivb), wobei A (CH₂)₂Ph ist, P THP ist und - - - - - eine Einfachbindung ist:

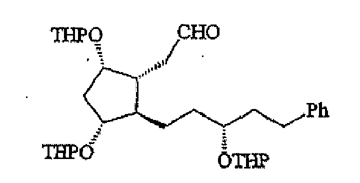

40. Verfahren zur Herstellung einer Prostaglandinverbindung mit der Formel (I): 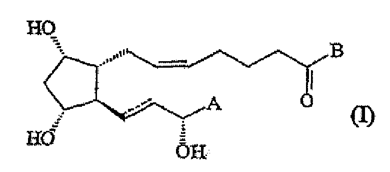 wobei A ausgewählt wird aus der Gruppe bestehend aus C₁-C₆-Alkyl; C₇-C₁₆-Aralkyl, wobei die Arylgruppe gegebenenfalls mit einem bis drei Substituenten substituiert ist, die aus der Gruppe bestehend aus C₁-C₆-Alkyl, Halogen und CF₃ ausgewählt werden; und (CH₂)ₙOR', wobei n 1 bis 3 ist und R' eine C₆-C₁₀-Arylgruppe repräsentiert, die gegebenenfalls mit einem bis drei Substituenten substituiert ist, die aus der Gruppe bestehend aus C₁-C₆-Alkyl, Halogen und CF₃ ausgewählt werden; B aus OR" und NHR" ausgewählt wird, wobei R" C₁-C₆-Alkyl ist; und - - - - - eine Doppelbindung oder eine Einfachbindung repräsentiert;
umfassend ein Verfahren nach irgendeinem der Ansprüche 1-4, 7-9, 12-14, 17-19, 22-26 und 29-38.

41. Verfahren zur Synthetisierung von Latanoprost, umfassend folgende Schritte:
a) eine 1,4-Additionsreaktion nach Anspruch 4,
b) eine selektive Reduktion nach Anspruch 9,
c) eine Dihydroxylierung nach Anspruch 24,
d) eine Reduktion nach Anspruch 31,
e) eine Diolspaltung nach Anspruch 36,
f) eine Wittig-Reaktion, wobei eine Verbindung der Formel (Iva), wobei A (CH₂)₂Ph ist, P THP ist und - - - - - eine Einfachbindung repräsentiert, zur Reaktion gebracht wird, um eine Verbindung der Formel (IIIa) zu ergeben, wobei A (CH₂)₂Ph ist, P THP ist und - - - - - eine Einfachbindung repräsentiert: 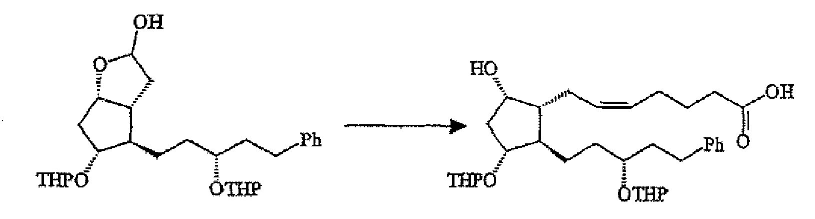
g) eine Veresterung, wobei eine Verbindung der Formel (IIIa), wobei A (CH₂)₂Ph ist, P THP ist und - - - - - eine Einfachbindung repräsentiert, zur Reaktion gebracht wird, um eine Verbindung der Formel (Iia) zu ergeben, wobei A (CH₂)₂Ph ist, P THP ist, - - - - - eine Einfachbindung repräsentiert und B OⁱPr ist: 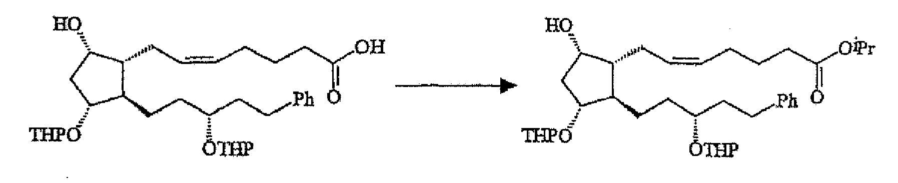 und
h) eine Entschützung, wobei eine Verbindung der Formel (lia), wobei A (CH₂)₂Ph ist, P THP ist, - - - - - eine Einfachbindung repräsentiert und B OⁱPr ist, zur Reaktion gebracht wird, um Latanoprost zu ergeben:

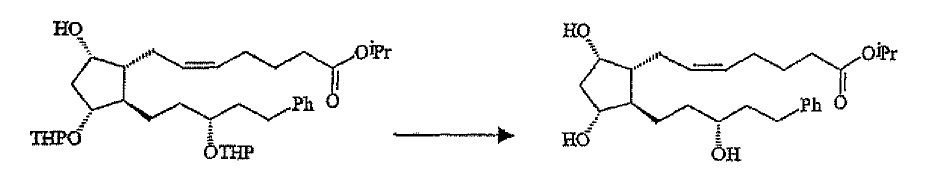

42. Verfahren zur Synthetisierung von Latanoprost, umfassend folgende Schritte:
a) eine 1,4-Additionsreaktion nach Anspruch 4,
b) eine selektive Reduktion nach Anspruch 9,
c) einen Schützungsschritt nach Anspruch 14,
d) eine Dihydroxylierung nach Anspruch 25,
e) eine Reduktion nach Anspruch 32,
f) eine Diolspaltung nach Anspruch 37,
g) eine Wittig-Reaktion, wobei eine Verbindung der Formel (Ivb), wobei A (CH₂)₂Ph ist, P THP ist und - - - - - eine Einfachbindung repräsentiert, zur Reaktion gebracht wird, um eine Verbindung der Formel (IIIb) zu ergeben, wobei A (CH₂)₂Ph ist, P THP ist und - - - - - eine Einfachbindung repräsentiert: 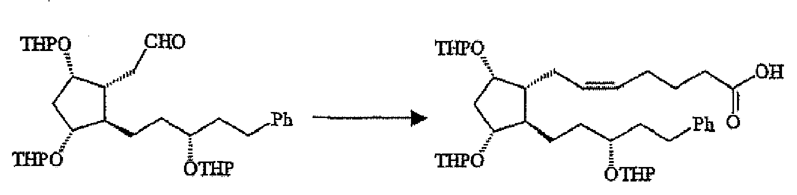
h) eine Veresterung, wobei eine Verbindung der Formel (IIIb), wobei A (CH₂)₂Ph ist, P THP ist und - - - - - eine Einfachbindung repräsentiert, zur Reaktion gebracht wird, um eine Verbindung der Formel (Iib) zu ergeben, wobei A (CH₂)₂Ph ist, P THP ist, - - - - - eine Einfachbindung repräsentiert und B OⁱPr ist: 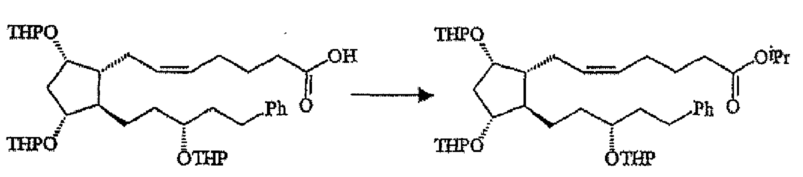 und
i) eine Entschützung, wobei eine Verbindung der Formel (Iib), wobei A (CH₂)₂Ph ist, P THP ist, - - - - - eine Einfachbindung repräsentiert und B OⁱPr ist, zur Reaktion gebracht wird, um Latanoprost zu ergeben:

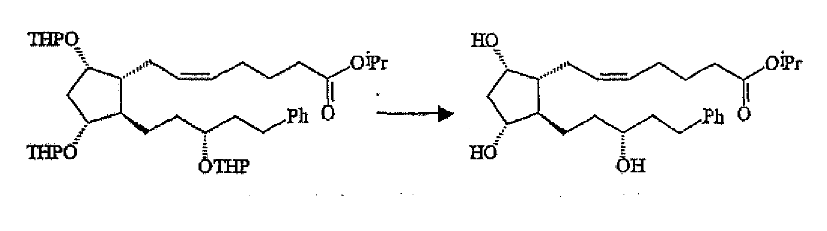

43. Verfahren zur Synthetisierung von Latanoprost, umfassend folgende Schritte:
a) eine 1,4-Additionsreaktion nach Anspruch 4,
b) eine selektive Reduktion nach Anspruch 9,
c) einen Entschützungsschritt nach Anspruch 19,
d) eine Dihydroxylierung nach Anspruch 26,
e) eine Reduktion nach Anspruch 33,
f) eine Diolspaltung nach Anspruch 38,
g) eine Wittig-Reaktion, wobei eine Verbindung der Formel (Ivc), wobei A (CH₂)₂Ph ist und - - - - - eine Einfachbindung repräsentiert, zur Reaktion gebracht wird, um eine Verbindung der Formel (IIIc) zu ergeben, wobei A (CH₂)₂Ph ist und - - - - - eine Einfachbindung repräsentiert: 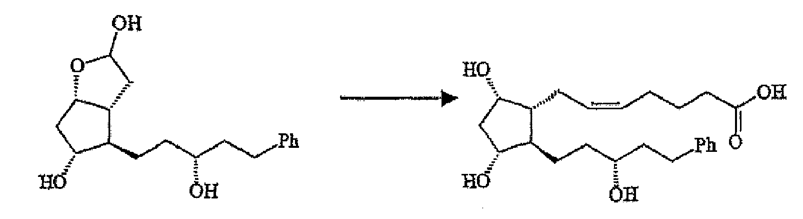 und
h) eine Veresterung, wobei eine Verbindung der Formel (IIIc), wobei A (CH₂)₂Ph ist und - - - - - eine Einfachbindung repräsentiert, zur Reaktion gebracht wird, um eine Verbindung der Formel (I) zu ergeben, wobei A (CH₂)₂Ph ist, P THP ist, - - - - - eine Einfachbindung repräsentiert und B OⁱPr ist:

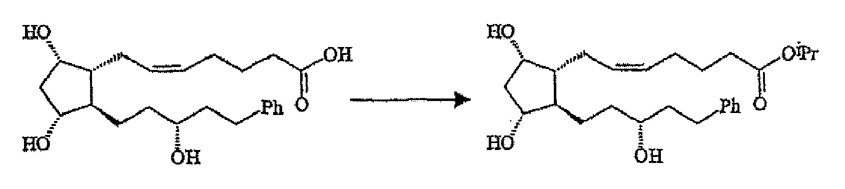

44. Verfahren zur Synthetisierung von Bimatoprost, umfassend folgeende Schritte:
a) eine 1,4-Additionsreaktion nach Anspruch 4,
b) eine selektive Reduktion nach Anspruch 9,
c) eine Dihydroxylierung nach Anspruch 24,
d) eine Diolspaltung nach Anspruch 34, wobei die Verbindung (Via), wobei A (CH₂)₂Ph ist und P THP ist, zur Reaktion gebracht wird, um die Verbindung (Iva) zu ergeben, wobei A (CH₂)₂Ph ist, P THP ist und - - - - - eine Doppelbindung repräsentiert,
e) eine Wittig-Reaktion, wobei eine Verbindung der Formel (IVa), wobei A (CH₂)₂Ph ist, P THP ist und - - - - - eine Doppelbindung repräsentiert, zur Reaktion gebracht wird, um eine Verbindung der Formel (IIa) zu ergeben, wobei A (CH₂)₂Ph ist, P THP ist und - - - - - eine Doppelbindung repräsentiert: 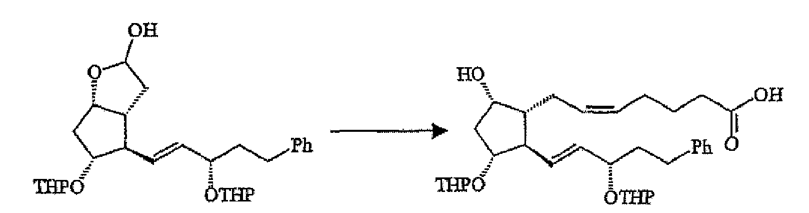
f) eine Amidierung, wobei eine Verbindung der Formel (IIIa), wobei A (CH₂)₂Ph ist, P THP ist und - - - - - eine Doppelbindung repräsentiert, zur Reaktion gebracht wird, um eine Verbindung der Formel (IIa) zu ergeben, wobei A (CH₂)₂Ph ist, P THP ist, - - - - - eine Doppelbindung repräsentiert und B NHEt ist: 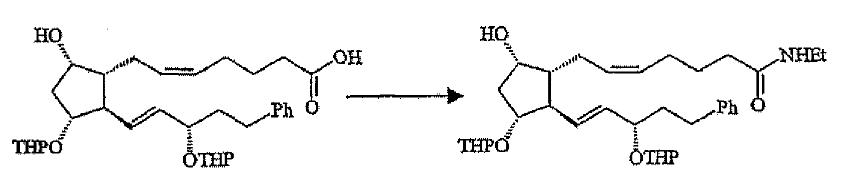 und
g) eine Entschützung, wobei eine Verbindung der Formel (IIa), wobei A (CH₂)₂Ph ist, P THP ist, - - - - - eine Doppelbindung repräsentiert und B NHEt ist, zur Reaktion gebracht wird, um Bimatoprost zu ergeben:

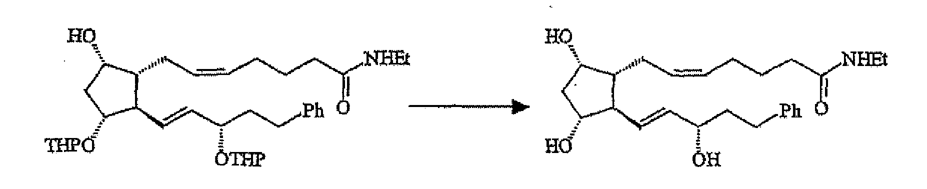

45. Verfahren zur Synthetisierung von Bimatoprost, umfassend folgende Schritte:
a) eine 1,4-Additionsreaktion nach Anspruch 4,
b) eine selektive Reduktion nach Anspruch 9,
c) einen Schützungsschritt nach Anspruch 14,
d) eine Dihydroxylierung nach Anspruch 25,
e) eine Diolspaltung nach Anspruch 34, wobei eine Verbindung (VIb), wobei A (CH₂)₂Ph ist und P THP ist, zur Reaktion gebracht wird, um die Verbindung (IVb) zu ergeben, wobei A (CH₂)₂Ph ist, P THP ist und - - - - - eine Doppelbindung repräsentiert,
f) eine Wittig-Reaktion, wobei eine Verbindung der Formel (IVb), wobei A (CH₂)₂Ph ist, P THP ist und - - - - - eine Doppelbindung repräsentiert, zur Reaktion gebracht wird, um eine Verbindung der Formel (IIIb) zu ergeben, wobei A (CH₂)₂Ph ist, P THP ist und - - - - - eine Doppelbindung repräsentiert: 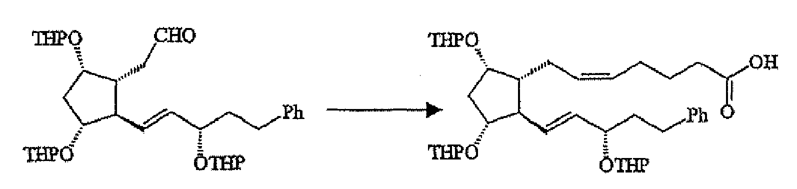
g) eine Amidierung, wobei eine Verbindung der Formel (IIIb), wobei A (CH₂)₂Ph ist, P THP ist und - - - - - eine Doppelbindung repräsentiert, zur Reaktion gebracht wird, um eine Verbindung der Formel (IIb) zu ergeben, wobei A (CH₂)₂Ph ist, P THP ist, - - - - - eine Einfachbindung repräsentiert und B NHEt ist: 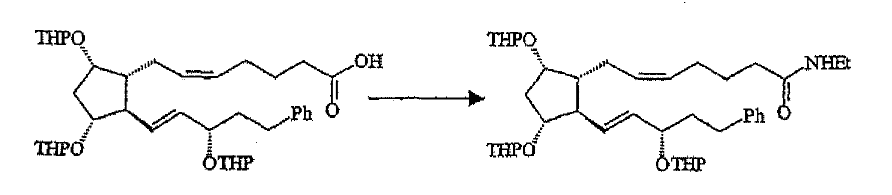 und
h) eine Entschützung, wobei eine Verbindung der Formel (IIb), wobei A (CH₂)₂Ph ist, P THP ist, - - - - - eine Doppelbindung repräsentiert und B NHEt ist, zur Reaktion gebracht wird, um Bimatoprost zu ergeben:

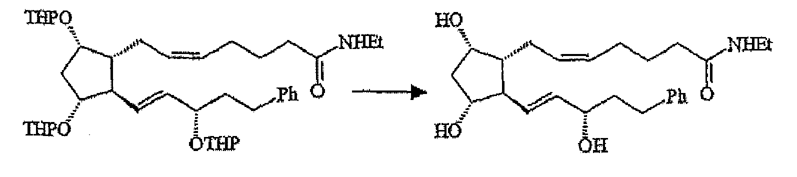

46. Verfahren zur Synthetisierung von Bimatoprost, umfassend folgende Schritte:
a) eine 1,4-Additionsreaktion nach Anspruch 4,
b) eine selektive Reduktion nach Anspruch 9,
c) einen Entschützungsschritt nach Anspruch 19,
d) eine Dihydroxylierung nach Anspruch 26,
e) eine Diolspaltung nach Anspruch 34, wobei die Verbindung (VIc), wobei A (CH₂)₂Ph ist, zur Reaktion gebracht wird, um die Verbindung (IVc) zu ergeben, wobei A (CH₂)₂Ph ist und - - - - - eine Doppelbindung repräsentiert,
f) eine Wittig-Reaktion, wobei eine Verbindung der Formel (IVc), wobei A (CH₂)₂Ph ist und - - - - - eine Doppelbindung repräsentiert, zur Reaktion gebracht wird, um eine Verbindung der Formel (IIIc) zu ergeben, wobei A (CH₂)₂Ph ist und - - - - - eine Doppelbindung repräsentiert: 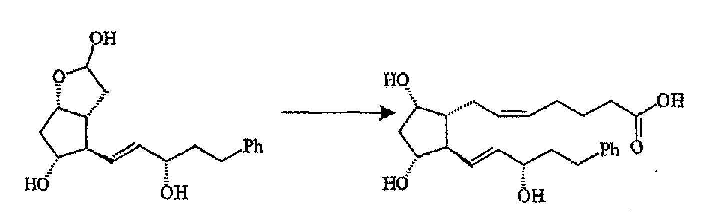 und
g) eine Amidierung, wobei eine Verbindung der Formel (IIIc), wobei A (CH₂)₂Ph ist und - - - - - eine Doppelbindung repräsentiert, zur Reaktion gebracht wird, um Bimatoprost zu ergeben:

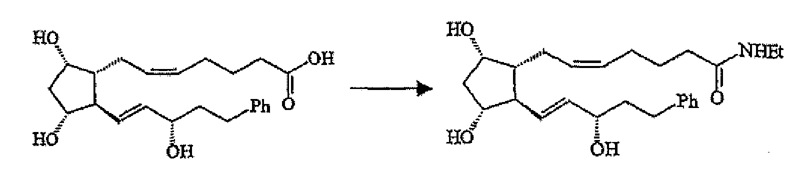

47. Verfahren zur Synthetisierung von Travoprost, umfassend ein Verfahren nach irgendeinem der Ansprüche 1-3, 7-8, 12-13, 17-18, 22-23, 29-30 und 34-35.

## Revendications

1. Procédé pour la préparation d'un composé de Formule (VIII) : 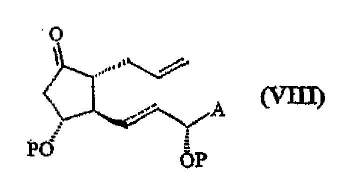 dans laquelle A est (CH₂)₂Ph ou CH₂OR"', dans laquelle R"' est un phényle, substitué à la position méta avec CF₃ ; P est un groupe hydroxy-protecteur ; et - - - - - représente une liaison double ou une liaison simple ;
dans lequel un composé de Formule (IX) : 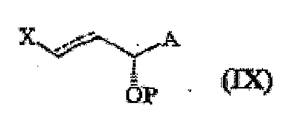 dans laquelle A, P et - - - - - sont tels que définis ci-dessus et X est un groupe sortant, est fait réagir pour former un réactif au cuprate qui subit une réaction d'addition 1,4 avec un composé de Formule (X) : 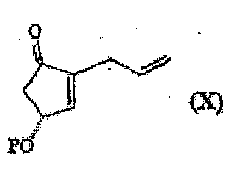 dans laquelle P est tel que défini ci-dessus.

2. Procédé selon la revendication 1, dans lequel P est un groupe protecteur tétrahydropyranyle (THP).

3. Procédé selon la revendication 1 ou la revendication 2, dans lequel X est l'iode.

4. Procédé selon la revendication 1, dans lequel A est (CH₂)₂Ph, - - - - - représente une liaison double, P est le THP et X est I.

5. Composé de Formule (VIII) : 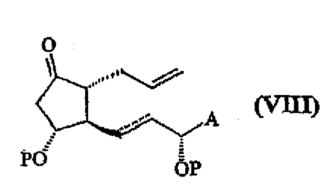 dans laquelle A, P et - - - - - sont tels que définis dans la revendication 1.

6. Composé selon la revendication 5, dans lequel A est (CH₂)₂Ph, - - - - - représente une liaison double et P est le THP.

7. Procédé pour la préparation d'un composé de formule (VIIa) : 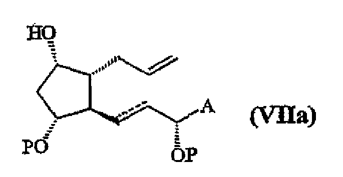 dans laquelle A est (CH₂)₂Ph ou CH₂OR"', dans laquelle R"' est un phényle, substitué à la position méta avec CF₃; P est un groupe hydroxy-protecteur ; et - - - - - représente une liaison double ou une liaison simple ;
dans lequel un composé de Formule (VIII) : 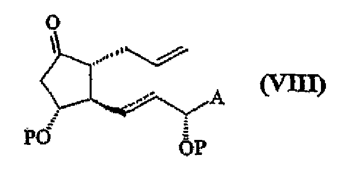 dans laquelle A, P et - - - - - sont tels que définis ci-dessus, subit une réduction sélective.

8. Procédé selon la revendication 7, dans lequel P est un groupe protecteur tétrahydropyranyle (THP).

9. Procédé selon la revendication 7, dans lequel A est (CH₂)₂Ph, - - - - - représente une liaison double et P est le THP.

10. Composé de formule (VIIa) : 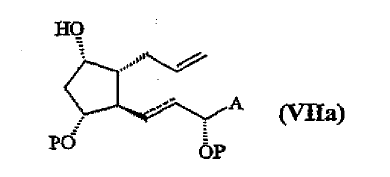 dans laquelle A, P et - - - - - sont tels que définis dans la revendication 7.

11. Composé selon la revendication 10, dans lequel A est (CH₂)₂Ph, - - - - - représente une liaison double et P est le THP.

12. Procédé pour la préparation d'un composé de formule (VIIb) : 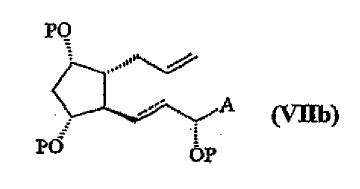 dans laquelle A est (CH₂)₂Ph ou CH₂OR"', dans laquelle R"' est un phényle, substitué à la position méta avec CF₃ ; P est un groupe hydroxy-protecteur et - - - - - représente une liaison double ou une liaison simple ;
dans lequel un composé de Formule (VIIa) : 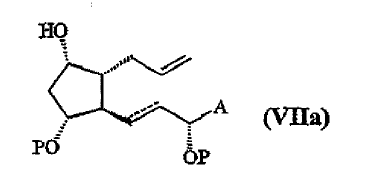 dans laquelle A, P et - - - - - sont tels que définis ci-dessus, est protégé avec un groupe hydroxy-protecteur.

13. Procédé selon la revendication 12, dans lequel P est un groupe protecteur tétrahydropyranyle (THP).

14. Procédé selon la revendication 12, dans lequel A est (CH₂)₂Ph, - - - - - représente une liaison double et P est le THP.

15. Composé de formule (VIIb) : 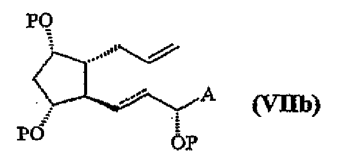 dans laquelle A, P et - - - - - sont tels que définis dans la revendication 12:

16. Composé selon la revendication 15, dans lequel A est (CH₂)₂Ph, - - - - - représente une liaison double et P est le THP.

17. Procédé pour la préparation d'un composé de formule (VIIc) : 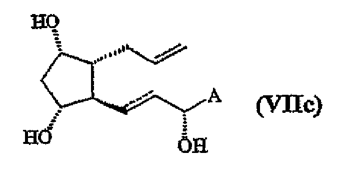 dans laquelle A est (CH₂)₂Ph ou CH₂OR"', dans laquelle R"' est un phényle, substitué à la position méta avec CF₃ et - - - - - représente une liaison double ou une liaison simple ;
dans lequel un composé de Formule (VIIa) : 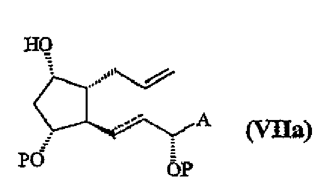 dans laquelle A et - - - - - sont tels que définis ci-dessus et P est un groupe protecteur, est déprotégé.

18. Procédé selon la revendication 17, dans lequel P est un groupe protecteur tétrahydropyranyle (THP).

19. Procédé selon la revendication 17 ou la revendication 18, dans lequel A est (CH₂)₂Ph et - - - - - représente une liaison double.

20. Composé de formule (VIIc) : 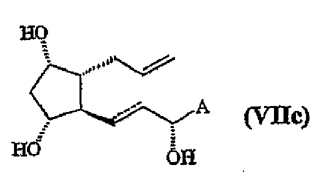 dans laquelle A et - - - - - sont tels que définis dans la revendication 17.

21. Composé selon la revendication 20, dans lequel A est (CH₂)₂Ph et - - - - - représente une liaison double.

22. Procédé pour la préparation d'un composé de formule (VIa), (Vlb), (VIc), (Va), (Vb) ou (Vc) : 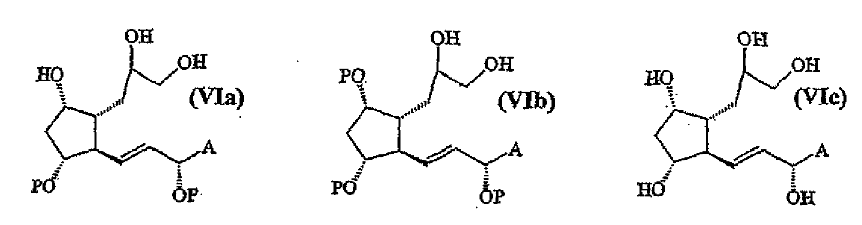 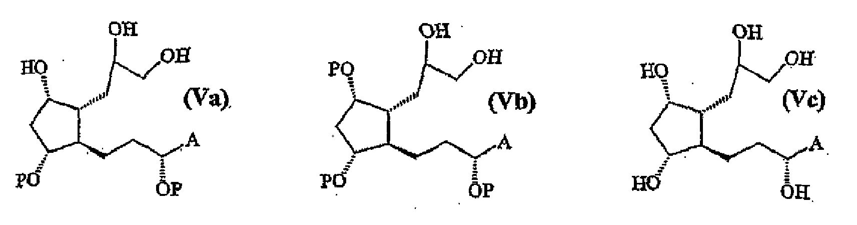 dans laquelle A est choisi parmi le groupe consistant en un alkyle en C₁-C₆ ; un aralkyle en C₇-C₁₆ dans lequel le groupe aryle est facultativement substitué avec un à trois substituant(s) choisi(s) parmi le groupe consistant en un alkyle en C₁-C₆, un groupe halogéno et CF₃; et (CH₂)ₙOR' dans lequel n est de 1 à 3 et R' représente un groupe aryle en C₆-C₁₀ qui est facultativement substitué avec un à trois substituant(s) choisi(s) parmi le groupe consistant en un alkyle en C₁-C₆, un groupe halogéno et CF₃ ; et P est un groupe hydroxy-protecteur ;
dans lequel un composé de Formule (VIIa), un composé de Formule (VIIb) ou composé de Formule (VIIc) : 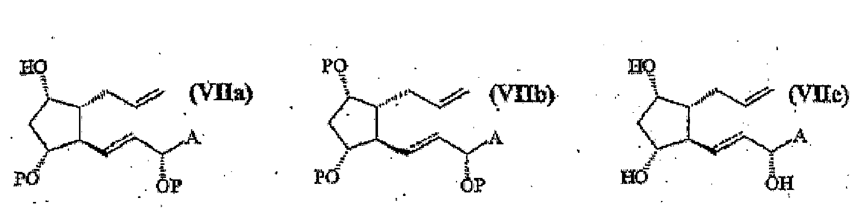 dans laquelle A et P sont tels que définis ci-dessus et - - - - - est une liaison double ou simple, subit une dihydroxylation.

23. Procédé selon la revendication 22, dans lequel P est un groupe protecteur tétrahydropyranyle (THP).

24. Procédé selon la revendication 22, dans lequel A est (CH₂)₂Ph, P est le THP, - - - - - représente une liaison double, et le composé (VIIa) est fait réagir pour donner le composé (VIa).

25. Procédé selon la revendication 22, dans lequel A est (CH₂)₂Ph, P est le THP, - - - - - représente une liaison double, et le composé (Vllb) est fait réagir pour donner le composé (VIb).

26. Procédé selon la revendication 22, dans lequel A est (CH₂)₂Ph, - - - - - représente une liaison double, et le composé (VIIc) est fait réagir pour donner le composé (VIc).

27. Composé de formule (VIa), (VIb), (VIc), (Va), (Vb) ou (Vc) : 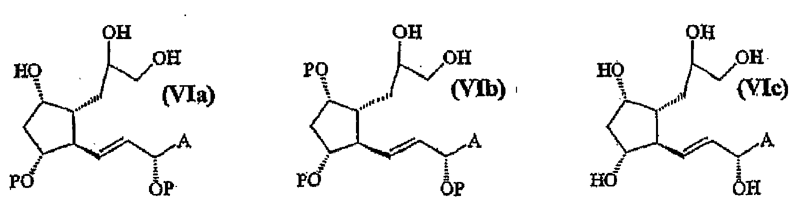 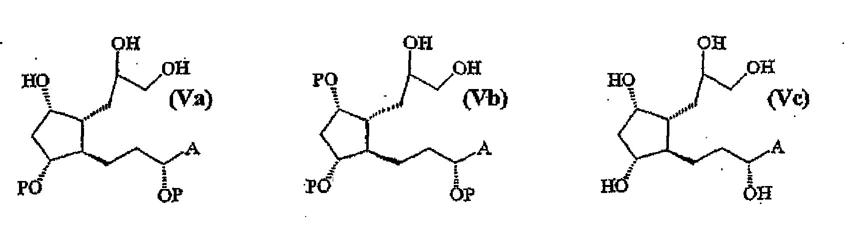 dans laquelle A et P sont tels que définis dans la revendication 22.

28. Composé selon la revendication 27, dans lequel A est (CH₂)₂Ph et P est le THP.

29. Procédé pour la préparation d'un composé de formule (Va), (Vb) ou (Vc) : 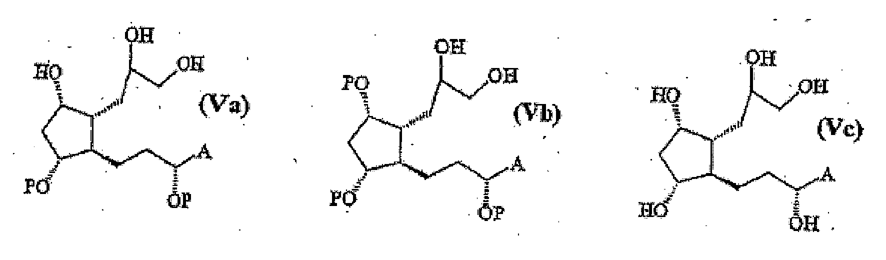 dans laquelle A est choisi parmi le groupe consistant en un alkyle en C₁-C₆; un aralkyle en C₇-C₁₆ dans lequel le groupe aryle est facultativement substitué avec un à trois substituant(s) choisi(s) parmi le groupe consistant en un alkyle en C₁-C₆, un groupe halogéno et CF₃ ; et (CH₂)ₙOR' dans lequel n est de 1 à 3 et R' représente un groupe aryle en C₆-C₁₀ qui est facultativement substitué avec un à trois substituant(s) choisi(s) parmi le groupe consistant en un alkyle en C₁-C₆, un groupe halogéno et CF₃ ; et P est un groupe hydroxy-protecteur ;
dans lequel un composé de Formule (VIa), un composé de Formule (VIb) ou composé de Formule (VIc) : 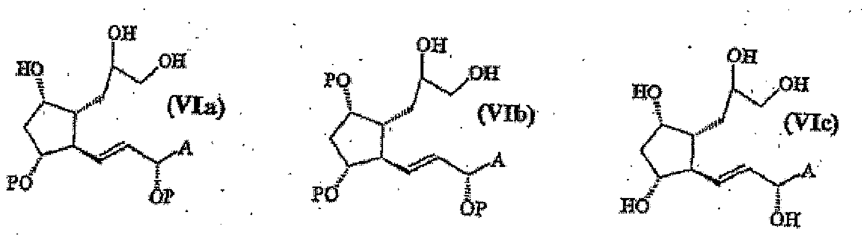 dans laquelle A et P sont tels que définis ci-dessus, subit une réduction de la liaison double.

30. Procédé selon la revendication 29, dans lequel P est un groupe protecteur tétrahydropyranyle (THP).

31. Procédé selon la revendication 29, dans lequel A est (CH₂)₂Ph, P est le THP et le composé (VIa) est fait réagir pour donner le composé (Va).

32. Procédé selon la revendication 29, dans lequel A est (CH₂)₂Ph, P est le THP et le composé (Vlb) est fait réagir pour donner le composé (Vb).

33. Procédé selon la revendication 29, dans lequel A est (CH₂)₂Ph et le composé (VIc) est fait réagir pour donner le composé (Vc).

34. Procédé pour la préparation d'un composé de formule (IVa), (IVb) ou (IVc) : 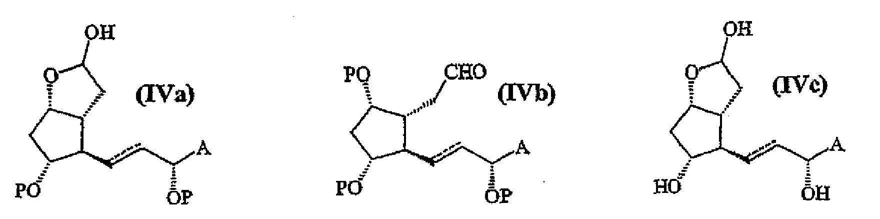 dans laquelle A est choisi parmi le groupe consistant en un alkyle en C₁-C₆ ; un aralkyle en C₇-C₁₆ dans lequel le groupe aryle est facultativement substitué avec un à trois substituant(s) choisi(s) parmi le groupe consistant en un alkyle en C₁-C₆, un groupe halogéno et CF₃ ; et (CH₂)ₙOR' dans lequel n est de 1 à 3 et R' représente un groupe aryle en C₆-C₁₀ qui est facultativement substitué avec un à trois substituant(s) choisi(s) parmi le groupe consistant en un alkyle en C₁-C₆, un groupe halogéno et CF₃ ; et P est un groupe hydroxy-protecteur et - - - - - représente une liaison double ou une liaison simple ;
dans lequel un composé de Formule (VIa), (Va), (VIb), (Vb), (VIc) ou (Vc) : 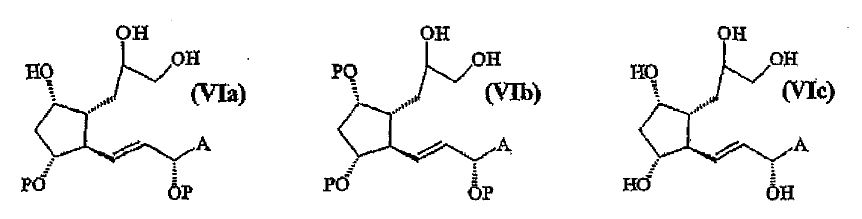 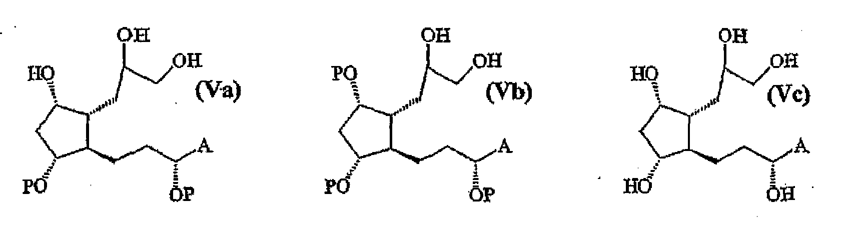 dans laquelle A et P sont tels que définis ci-dessus, subit un clivage du diol.

35. Procédé selon la revendication 34, dans lequel P est un groupe protecteur tétrahydropyranyle (THP).

36. Procédé selon la revendication 34, dans lequel A est (CH₂)₂Ph, P est le THP, - - - - - représente une liaison simple, et le composé (Va) est fait réagir pour donner le composé (IVa).

37. Procédé selon la revendication 34, dans lequel A est (CH₂)₂Ph, P est le THP, - - - - - représente une liaison simple, et le composé (Vb) est fait réagir pour donner le composé (IVb).

38. Procédé selon la revendication 34, dans lequel A est (CH₂)₂Ph, - - - - - représente une liaison simple, et le composé (Vc) est fait réagir pour donner le composé (IVc).

39. Composé de formule (IVb) dans lequel A est (CH₂)₂Ph, P est le THP et - - - - - est une liaison simple :

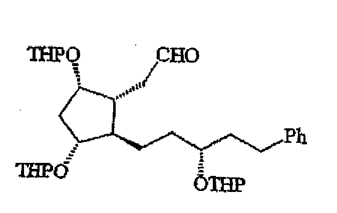

40. Procédé pour la préparation d'un composé de prostaglandine ayant la formule (I) : 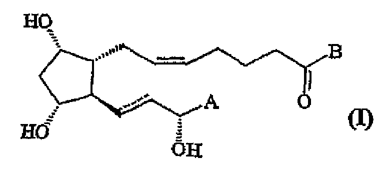 dans laquelle A est choisi parmi le groupe consistant en un alkyle en C₁-C₆; un aralkyle en C₇-C₁₆ dans lequel le groupe aryle est facultativement substitué avec un à trois substituant(s) choisi(s) parmi le groupe consistant en un alkyle en C₁-C₆, un groupe halogéno et CF₃ ; et (CH₂)ₙOR' dans lequel n est de 1 à 3 et R' représente un groupe aryle en C₆-C₁₀ qui est facultativement substitué avec un à trois substituant(s) choisi(s) parmi le groupe consistant en un alkyle en C₁-C₆, un groupe halogéno et CF₃ ; B est choisi parmi OR" et NHR" où R" est un alkyle en C₁-C₆ ; et - - - - - représente une liaison double ou une liaison simple ;
comprenant un procédé selon l'une quelconque des revendications 1-4, 7-9, 12-14, 17-19, 22-26 et 29-38.

41. Procédé pour synthétiser du Latanoprost comprenant les étapes de :
a) réaction d'addition 1,4 selon la revendication 4,
b) réduction sélective selon la revendication 9,
c) dihydroxylation selon la revendication 24,
d) réduction selon la revendication 31,
e) clivage du diol selon la revendication 36,
f) réaction de Wittig dans laquelle un composé de formule (IVa) dans lequel A est (CH₂)₂Ph, P est le THP et - - - - - représente une liaison simple, est fait réagir pour donner un composé de formule (IIIa) dans lequel A est (CH₂)₂Ph, P est le THP et - - - - - représente une liaison simple : 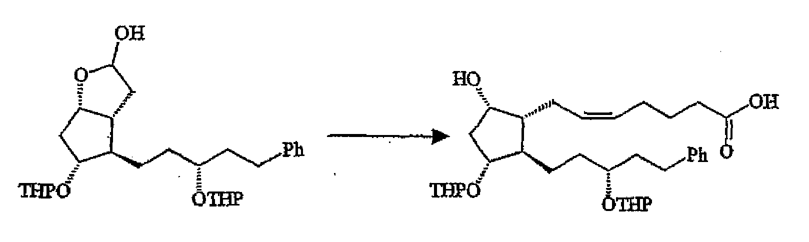
g) estérification dans laquelle un composé de formule (IIIa) dans lequel A est (CH₂)₂Ph, P est le THP et - - - - - représente une liaison simple, est fait réagir pour donner un composé de formule (IIa) dans lequel A est (CH₂)₂Ph, P est le THP, - - - - - représente une liaison simple et B est OiPr : 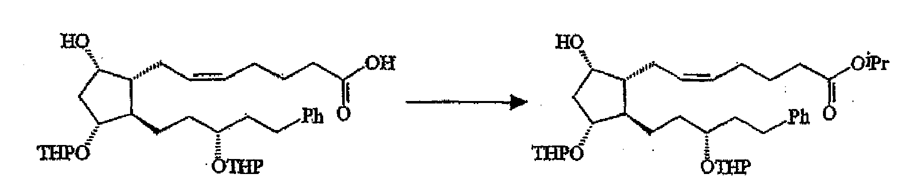 et
h) déprotection dans laquelle un composé de formule (IIa) dans lequel A est (CH₂)₂Ph, P est le THP, - - - - - représente une liaison simple et B est OiPr, est fait réagir pour donner du Latanoprost :

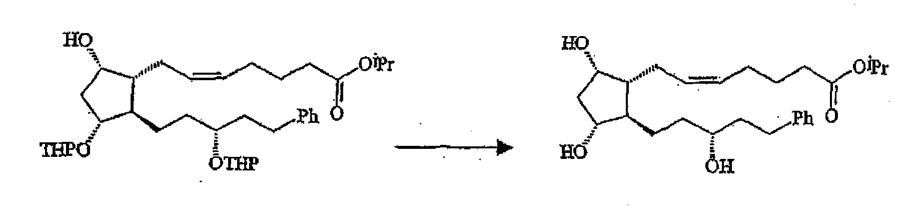

42. Procédé pour synthétiser du Latanoprost comprenant les étapes de :
a) réaction d'addition 1,4 selon la revendication 4,
b) réduction sélective selon la revendication 9,
c) étape de protection selon la revendication 14,
d) dihydroxylation selon la revendication 25,
e) réduction selon la revendication 32,
f) clivage du diol selon la revendication 37,
g) réaction de Wittig dans laquelle un composé de formule (IVb) dans lequel A est (CH₂)₂Ph, P est le THP et - - - - - représente une liaison simple, est fait réagir pour donner un composé de formule (IIIb) dans lequel A est (CH₂)₂Ph, P est le THP et - - - - - représente une liaison simple : 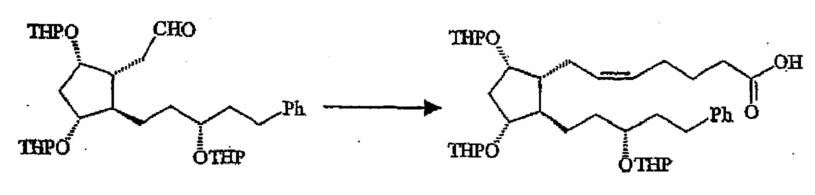
h) estérification dans laquelle un composé de formule (IIIb) dans lequel A est (CH₂)₂Ph, P est le THP et - - - - - représente une liaison simple, est fait réagir pour donner un composé de formule (IIb) dans lequel A est (CH₂)₂Ph, P est le THP, - - - - - représente une liaison simple et B est OⁱPr : 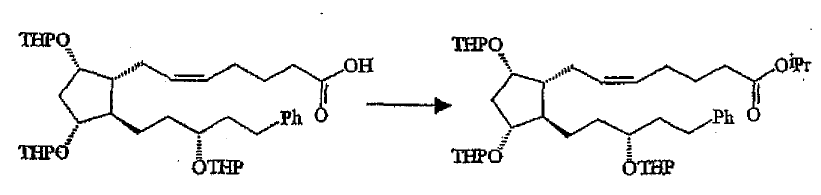 et
i) déprotection dans laquelle un composé de formule (IIb) dans lequel A est (CH₂)₂Ph, P est le THP, - - - - - représente une liaison simple et B est OⁱPr, est fait réagir pour donner du Latanoprost :

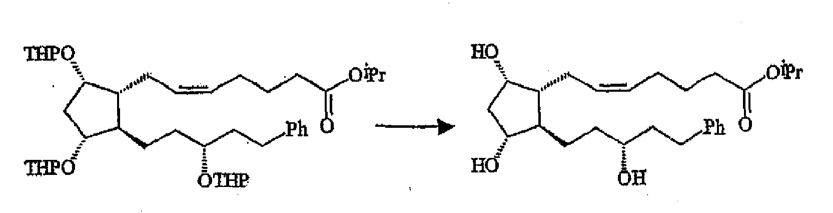

43. Procédé pour synthétiser du Latanoprost comprenant les étapes de :
a) réaction d'addition 1,4 selon la revendication 4,
b) réduction sélective selon la revendication 9,
c) étape de déprotection selon la revendication 19,
d) dihydroxylation selon la revendication 26,
e) réduction selon la revendication 33,
f) clivage du diol selon la revendication 38,
g) réaction de Wittig dans laquelle un composé de formule (IVc) dans lequel A est (CH₂)₂Ph et - - - - - représente une liaison simple, est fait réagir pour donner un composé de formule (IIIc) dans lequel A est (CH₂)₂Ph et - - - - - représente une liaison simple : 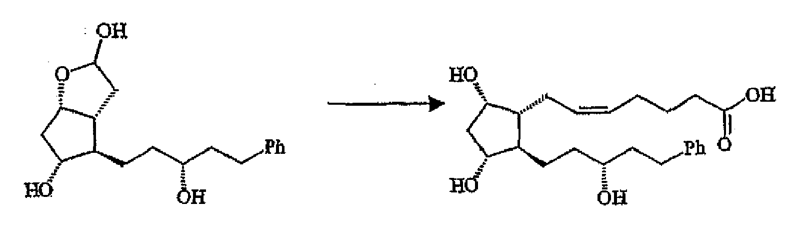 et
h) estérification dans laquelle un composé de formule (IIIc) dans lequel A est (CH₂)₂Ph et - - - - - représente une liaison simple, est fait réagir pour donner un composé de formule (I) dans lequel A est (CH₂)₂Ph, P est le THP, - - - - - représente une liaison simple et B est OⁱPr :

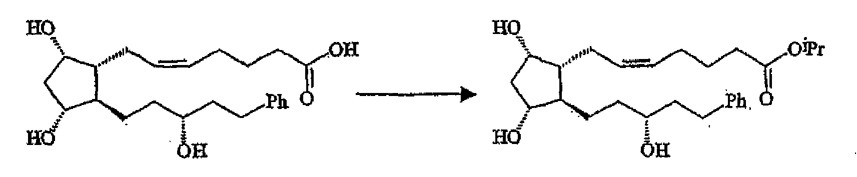

44. Procédé pour synthétiser du Bimatoprost comprenant les étapes de :
a) réaction d'addition 1,4 selon la revendication 4,
b) réduction sélective selon la revendication 9,
c) dihydroxylation selon la revendication 24,
d) clivage du diol selon la revendication 34, dans laquelle un composé (VIa) dans lequel A est (CH₂)₂Ph et P est le THP, est fait réagir pour donner un composé (IVa) dans lequel A est (CH₂)₂Ph, P est le THP et - - - - - représente une liaison double,
e) réaction de Wittig dans laquelle un composé de formule (IVa) dans lequel A est (CH₂)₂Ph, P est le THP et - - - - - représente une liaison double, est fait réagir pour donner un composé de formule (IIIa) dans lequel A est (CH₂)₂Ph, P est le THP et - - - - - représente une liaison double : 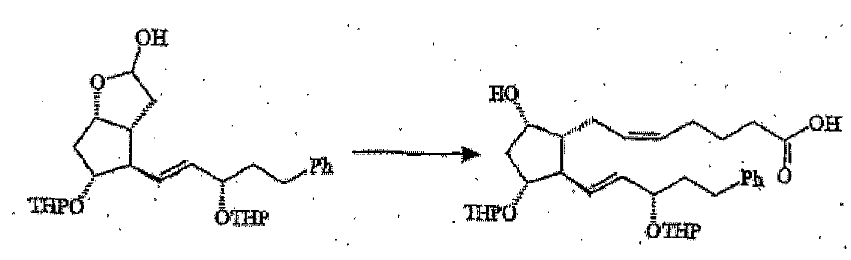
f) amidation dans laquelle un composé de formule (IIIa) dans lequel A est (CH₂)₂Ph, P est le THP et - - - - - représente une liaison double, est fait réagir pour donner un composé de formule (IIa) dans lequel A est (CH₂)₂Ph, P est le THP, - - - - - représente une liaison double et B est NHEt : 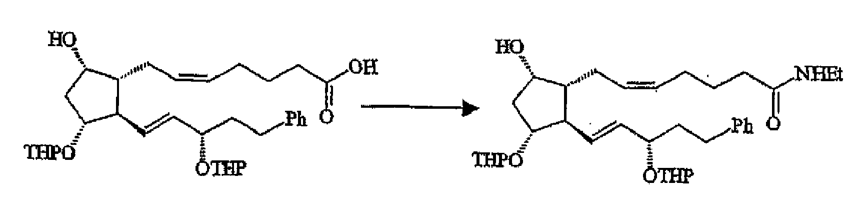 et
g) déprotection dans laquelle un composé de formule (IIa) dans lequel A est (CH₂)₂Ph, P est le THP, - - - - - représente une liaison double et B est NHEt, est fait réagir pour donner du Bimatoprost :

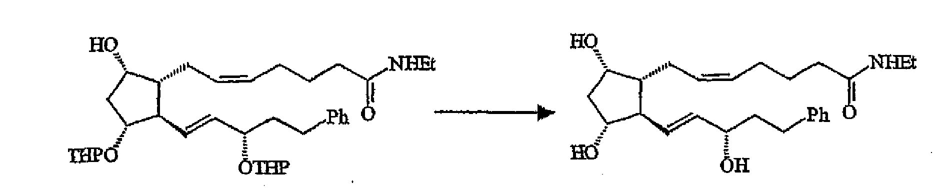

45. Procédé pour synthétiser du Bimatoprost comprenant les étapes de :
a) réaction d'addition 1,4 selon la revendication 4,
b) réduction sélective selon la revendication 9,
c) étape de protection selon la revendication 14,
d) dihydroxylation selon la revendication 25,
e) clivage du diol selon la revendication 34, dans laquelle un composé (Vlb) dans lequel A est (CH₂)₂Ph et P est le THP, est fait réagir pour donner un composé (IVb) dans lequel A est (CH₂)₂Ph, P est le THP et - - - - - représente une liaison double,
f) réaction de Wittig dans laquelle un composé de formule (IVb) dans lequel A est (CH₂)₂Ph, P est le THP et - - - - - représente une liaison double, est fait réagir pour donner un composé de formule (IIIb) dans lequel A est (CH₂)₂Ph, P est le THP et - - - - - représente une liaison double : 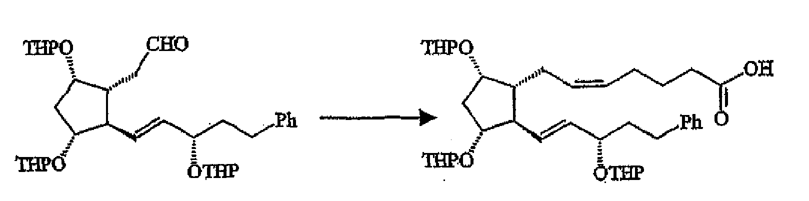
g) amidation dans laquelle un composé de formule (IIIb) dans lequel A est (CH₂)₂Ph, P est le THP et - - - - - représente une liaison double, est fait réagir pour donner un composé de formule (IIb) dans lequel A est (CH₂)₂Ph, P est le THP, - - - - - représente une liaison simple et B est NHEt : 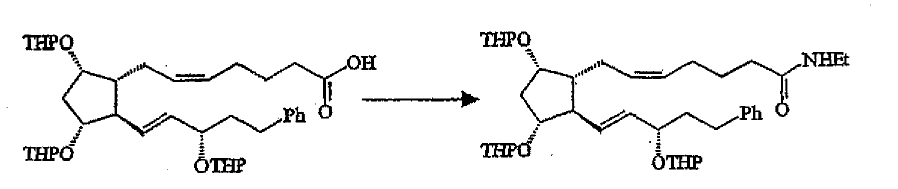 et
h) déprotection dans laquelle un composé de formule (IIb) dans lequel A est (CH₂)₂Ph, P est le THP, - - - - - représente une liaison double et B est NHEt, est fait réagir pour donner du Bimatoprost :

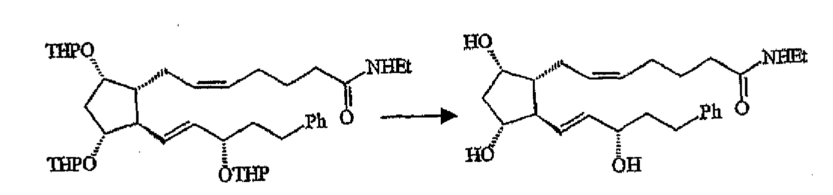

46. Procédé pour synthétiser du Bimatoprost comprenant les étapes de :
a) réaction d'addition 1,4 selon la revendication 4,
b) réduction sélective selon la revendication 9,
c) étape de déprotection selon la revendication 19,
d) dihydroxylation selon la revendication 26,
e) clivage du diol selon la revendication 34, dans laquelle un composé (VIc) dans lequel A est (CH₂)₂Ph, est fait réagir pour donner un composé (IVc) dans lequel A est (CH₂)₂Ph et - - - - - représente une liaison double,
f) une réaction de Wittig dans laquelle un composé de formule (IVc) dans lequel A est (CH₂)₂Ph et - - - - - représente une liaison double, est fait réagir pour donner un composé de formule (IIIc) dans lequel A est (CH₂)₂Ph et - - - - - représente une liaison double : 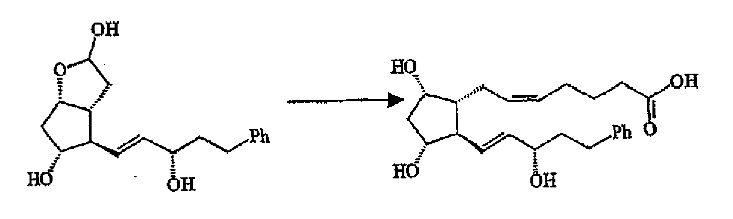 et
g) amidation dans laquelle un composé de formule (IIIc) dans lequel A est (CH₂)₂Ph et - - - - - représente une liaison double, est fait réagir pour donner du Bimatoprost :

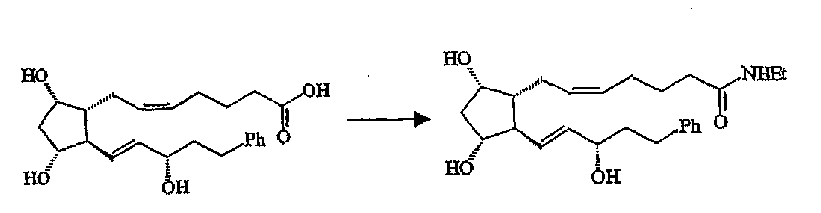

47. Procédé pour synthétiser du Travoprost comprenant un procédé selon l'une quelconque des revendications 1-3, 7-8, 12-13, 17-18, 22-23, 29-30 et 34-35.
